(19) Europäisches Patentamt / European Patent Office / Office européen des brevets

(11) EP 4 685 146 A1

# (12) EUROPEAN PATENT APPLICATION

published in accordance with Art. 153(4) EPC

(43) Date of publication:
**28.01.2026 Bulletin 2026/05**

(21) Application number: **24797279.7**

(22) Date of filing: **19.03.2024**

(51) International Patent Classification (IPC):
*C07D 495/14* (2006.01) *C07D 495/04* (2006.01)
*C07D 493/04* (2006.01) *C07F 7/08* (2006.01)
*C07D 493/14* (2006.01) *C07D 517/14* (2006.01)
*C07D 517/04* (2006.01) *C07D 333/78* (2006.01)
*C07D 307/94* (2006.01) *C07D 331/00* (2006.01)

(52) Cooperative Patent Classification (CPC):
Y02E 10/549

(86) International application number:
**PCT/KR2024/003448**

(87) International publication number:
**WO 2024/225617 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.04.2023 KR 20230053947**

(71) Applicant: **Duk San Neolux Co., Ltd.**
**Chungcheongnam-do 31027 (KR)**

(72) Inventors:
- **HAHN, Seung Hoon**
  **Cheonan-si, Chungcheongnam-do 31027 (KR)**
- **LEE, Hyung Dong**
  **Cheonan-si, Chungcheongnam-do 31027 (KR)**
- **YOON, Ki Hwan**
  **Cheonan-si, Chungcheongnam-do 31027 (KR)**

(74) Representative: **Prüfer & Partner mbB**
**Patentanwälte · Rechtsanwälte**
**Sohnckestraße 12**
**81479 München (DE)**

## (54) COMPOUND FOR ORGANIC PHOTOVOLTAIC DEVICES, AND ORGANIC PHOTOVOLTAIC DEVICE, IMAGE SENSOR, AND ELECTRONIC DEVICE EACH CONTAINING SAME

(57) The present invention discloses a compound for an organic photoelectric element represented by Formula 1, organic photoelectric element, image sensor and electronic device comprising the same. The compound represented by Formula 1 is a material with high sensitivity and high heat resistance, which can significantly improve the efficiency and stability of the element when employed to an organic photoelectric element.

FIG. 1

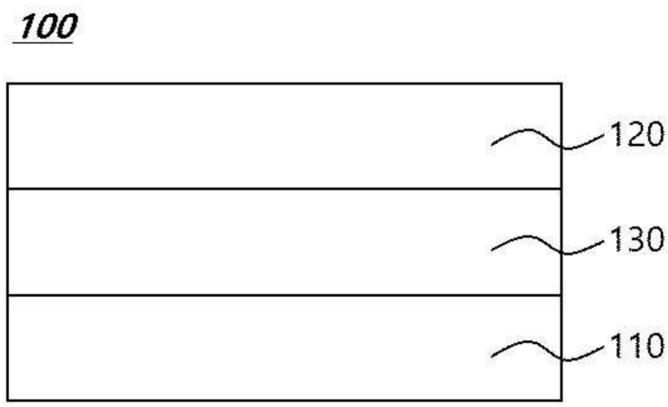




Processed by Luminess, 75001 PARIS (FR)

## Description

### Technical Field

The present invention relates to a compound for an organic photoelectric element, an organic photoelectric element, an image sensor and an electronic device comprising the same.

### Background Art

A photoelectric element is an element that converts light into electrical signals using the photoelectric effect, and it can be applied to image sensor. Therefore, development of photoelectric elements has been continuously carried out to develop image sensor.

The resolution of image sensor is increasing as technology develops, and accordingly, the size of pixels is also decreasing. However, as the pixel size gets smaller, the absorption area decreases, which can cause a drop in sensitivity. To overcome this, active research on photoelectric elements is being conducted.

Recently, organic materials deposited by vacuum evaporation are increasingly replacing silicon materials or polymer materials using inkjet, which have been mainly used as photoelectric elements. Organic materials have high absorption coefficients and can be designed to selectively absorb light in specific wavelength regions depending on their molecular structure. That is, organic materials can selectively detect light by absorbing light in the desired color range, thereby replacing both conventional photoelectric elements and color filters. Therefore, organic materials should have high sensitivity, and organic photoelectric elements that show high efficiency even in high-temperature processes such as vacuum evaporation are needed. For this purpose, it is necessary to develop organic materials with excellent heat resistance.

Therefore, research is needed to develop materials with high sensitivity and high heat resistance in order to realize excellent organic photoelectric elements.

## Detailed Description of the Invention

### Technical Challenge

To solve the problems aforementioned in background Art, the present invention has discovered a compound with a novel structure, and has also found that applying this compound to an organic photoelectric element can significantly improve the efficiency and stability of the elements. Accordingly, the purpose of the present invention is to provide a novel compound for an organic photoelectric element, an organic photoelectric element, an image sensor and an electronic device comprising the same.

### Means of Solving Problems

In one aspect, the present invention provides a compound represented by the following Formula.

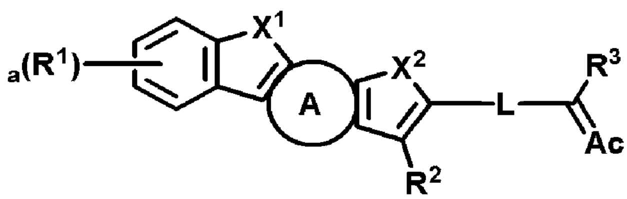

In another aspect, the present invention provides an organic photoelectric element, an image sensor, and an electronic device comprising a compound represented by the above Formula.

### Effect of Invention

By employing a compound according to the present invention, it is possible to provide a material having high sensitivity and high heat resistance, thereby greatly improving the efficiency and stability of an organic photoelectric element.

## BRIEF DESCRIPTION OF THE DRAWINGS

Figures 1 and 2 show the laminated structure of an organic photoelectric element according to an embodiment of the present invention.

[Description of Reference Numerals]

| | | | |
|---|---|---|---|
| 110: | first electrode | 120: | second electrode |
| 130: | active layer | 210: | hole transport region |
| 220: | electron transport region | | |

## DETAILED DESCRIPTION

Unless otherwise stated, the term "aryl group" or "arylene group" as used herein refers to a group having 6 to 60 carbon atoms, but is not limited thereto. The aryl group or arylene group in the present invention comprise a monocyclic ring, polycyclic and condensed ring, and the like.

As used herein, the term "fluorenyl group" refers to a substituted or unsubstituted fluorenyl group, and the term "fluorenylene group" refers to a substituted or unsubstituted fluorenylene group. The fluorenyl group or fluorenylene group used in the present invention includes spiro compounds formed by the bonding of R and R' in the following structure, and also includes compounds in which adjacent R" groups are bonded to form a ring. The terms "substituted fluorenyl group" and "substituted fluorenylene group" mean that at least one of R, R', or R" in the following structure is a substituent other than hydrogen. In the following structure, the number of R" groups may range from 1 to 8. Throughout this specification, the fluorenyl group and fluorenylene group may collectively be referred to as a "fluorene group" or "fluorene," regardless of their valence.

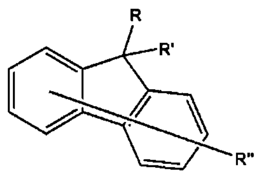

As used herein, the term "spiro compound" refers to a compound having a spiro linkage, which is a structure in which two rings are connected through a single common atom. The atom shared by the two rings is referred to as a "spiro atom," and the compound may be classified as a monospiro, dispiro, or trispiro compound depending on the number of spiro atoms present in the molecule.

As used herein, the term "heterocyclic group" comprises both aromatic rings, such as a "heteroaryl group" or a "heteroarylene group," and non-aromatic rings. Unless otherwise specified, the "heterocyclic group" refers to a ring structure containing one or more heteroatoms and having from 2 to 60 carbon atoms, but is not limited thereto. The term "heteroatom," as used herein, refers to atoms such as nitrogen (N), oxygen (O), sulfur (S), phosphorus (P), or silicon (Si), and may also include heteroatomic groups such as $SO_2$, P=O, and the like, which can replace a carbon atom in the ring structure as shown in the following compound.

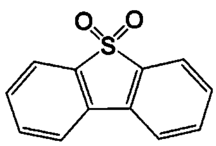

In addition, the heterocyclic group includes monocyclic, polycyclic, or condensed(fused) rings containing a heteroatom. In the case of condensed rings, if at least one of the rings in the condensed system contains a heteroatom, it is defined as a heterocycle. For example, the condensed ring systems where a heterocycle such as furan, dihydrofuran, thiophene, pyrrole, or pyridine is fused with an aromatic ring such as benzene, naphthalene, or phenanthrene, or with an alicyclic ring such as cyclopentane or cyclohexane fall under the category of heterocycles. Likewise, a spiro compound in which at least one ring contains a heteroatom is also considered heterocycle.

The term "aliphatic ring group" as used in this specification refers to a cyclic hydrocarbon excluding aromatic

hydrocarbons. It includes a monocyclic, a polycyclic, a fused ring, and a spiro compound. Unless otherwise specified, it generally refers to rings containing 3 to 60 carbon atoms, but is not limited thereto. In particular, an aliphatic ring (group) in this specification is defined as a hydrocarbon ring that does not contain any aromatic ring. Therefore, not only a saturated hydrocarbon ring such as cycloalkyl group, but also a ring containing one or more double bonds is considered aliphatic ring as long as it is not an aromatic hydrocarbon.

The term "a fused ring (group)" or "a condensed ring (group)" as used in the present specification refers, unless otherwise specified, to a ring formed by the fusion of an aliphatic ring and an aromatic hydrocarbon ring (aromatic ring or aryl ring), and, unless otherwise stated, it means a ring condensed of an aliphatic ring having 3 to 60 carbon atoms and an aromatic hydrocarbon having 6 to 60 carbon atoms.

In this specification, the 'group name' corresponding to an aryl group, an arylene group, a heterocyclic group, and the like, exemplified for each symbol and its substituent, may be expressed either as 'a functional group name reflecting the valence' or as 'the name of a parent compound'. For example, in the case of 'phenanthrene,' which is a type of aryl group, it may be described as 'phenanthryl' when referring to a monovalent group, and as 'phenanthrylene' when referring to a divalent group. Alternatively, it may also be described by its parent compound name 'phenanthrene,' regardless of valence. Similarly, in the case of pyrimidine, it may be referred to as 'pyrimidine' regardless of its valence. Alternatively, it may be described by the name of the corresponding functional group, such as 'pyrimidinyl' for a monovalent group and 'pyrimidinylene' for a divalent group.

In addition, in the present specification, numerical and alphabetical indicators of positions may be omitted when describing the name of a compound or a substituent. For example, compounds such as pyrido[4,3-d]pyrimidine, benzofuro[2,3-d]pyrimidine, and 9,9-dimethyl-9H-fluorene may be described in a simplified manner as pyridopyrimidine, benzofurropyrimidine, and dimethylfluorene, respectively. Accordingly, both benzo[g]quinoxaline and benzo[f]quinoxaline may be generally referred to as benzoquinoxaline.

In addition, unless otherwise specified, the definitions of substituents in the Formula used in the present invention may be applied in accordance with the definitions of the index in the following Formula.

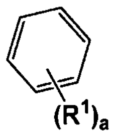

Here, when "a" is an integer of 0, it means that the substituent $R^1$ is absent. In other words, when a is 0, all carbon atoms forming the benzene ring are bonded to hydrogen atoms, and in this case, the hydrogen atoms bonded to the carbon atoms may be omitted in the depiction of the formula or compound. In addition, when "a" is an integer of 1, one substituent $R^1$ is bonded to any one of the carbon atoms forming the benzene ring. When "a" is an integer of 2 or 3, the substituents may be bonded as shown below, and when "a" is an integer from 4 to 6, the substituents are also bonded to the carbon atoms of the benzene ring in a similar manner. When "a" is an integer of 2 or more, the $R^1$ substituents may be the same or different from each other.

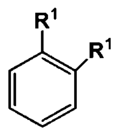

(a=2)

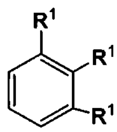

(a=3)

In addition, unless otherwise specified, the term "ring" as used in this specification refers to an aryl ring, a heteroaryl ring, a fluorene ring, an aliphatic ring, a fused ring, and the like. The expression "number-ring" denotes a fused(condensed) ring system, whereas "number-membered ring" may refer to the shape of the ring. For example, naphthalene corresponds to a fused ring consisting of two rings, anthracene corresponds to a fused ring consisting of three rings, thiophene and furan correspond to five-membered heterocycles, and benzene and pyridine corresponds to six-membered aromatic rings.

In addition, unless otherwise specified in the present specification, when adjacent groups are linked to each other to form a ring, the ring may be selected from the group consisting of a $C_6$-$C_{60}$ aromatic ring group, a fluorenyl group, a $C_2$-$C_{60}$ heterocyclic group containing at least one heteroatom selected from O, N, S, Si, and P, a $C_3$-$C_{60}$ aliphatic ring group, and a fused ring of a $C_3$-$C_{60}$ aliphatic ring and a $C_6$-$C_{60}$ aromatic ring. Here, the aromatic ring group may be an aryl ring, and the heterocyclic group may comprise a heteroaryl ring.

Unless otherwise specified, the term 'adjacent groups,' as used herein, comprises not only the relationships such as $R_1$ and $R_2$, $R_2$ and $R_3$, $R_3$ and $R_4$, and $R_5$ and $R_6$ but also $R_7$ and $R_8$ sharing a common carbon atom. It may further

comprise cases substituents attached to different ring-forming atoms (e.g., carbon or nitrogen), such as $R_1$ and $R_7$, $R_1$ and $R_8$, or $R_4$ and $R_5$. That is, even when substituents are not directly adjacent on the same atom, one substituent may be considered adjacent to another substituent attached to a neighboring ring-forming atom. Additionally, substituents bonded to the same carbon atom forming the ring may also be regarded as adjacent groups. In the following Formula, when substituents such as $R_7$ and $R_8$, which are bonded to the same carbon atom, are connected to form a ring, a compound containing a spiro moiety may be formed.

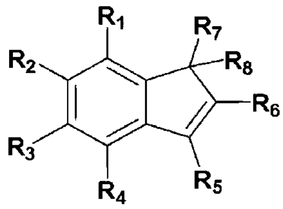
,
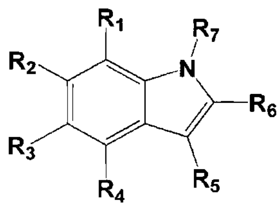

In addition, in the present specification, the expression 'adjacent groups may be linked to each other to form a ring' is used in the same sense as 'adjacent groups are selectively linked to each other to form a ring,' and refers to a case where at least one pair of adjacent groups may be bonded to form a ring.

In addition, unless otherwise specified in the present specification, substituents such as an aryl group, an arylene group, a fluorenyl group, a fluorenylene group, a heterocyclic group, an aliphatic ring group, an alkyl group, an alkenyl group, an alkynyl group, an alkoxyl group, an aryloxyl group, alkylthio group, arylthio group, etc., and a ring formed by adjacent groups may be each optionally substituted with one or more substituents selected from the group consisting of deuterium, halogen, a cyano group, a nitro group, siloxane group, a $C_6$-$C_{30}$ aryl group, a fluorenyl group, a $C_2$-$C_{30}$ heterocyclic group containing at least one heteroatom of O, N, S, Si and P, a $C_3$-$C_{30}$ aliphatic ring group, a $C_1$-$C_{20}$ alkyl group, a $C_2$-$C_{20}$ alkenyl group, a $C_2$-$C_{20}$ alkynyl group, a $C_1$-$C_{20}$ alkoxyl group, a $C_6$-$C_{20}$ aryloxy group, a $C_1$-$C_{20}$ alkylthio group, a $C_6$-$C_{20}$ arylthio group, a silane group unsubstituted or substituted with a $C_1$-$C_{20}$ alkyl group or a $C_6$-$C_{20}$ aryl group, and a phosphine oxide group unsubstituted or substituted with a $C_1$-$C_{20}$ alkyl group or a $C_6$-$C_{20}$ aryl group.

Hereinafter, a compound according to one aspect of the present invention will be described.

A compound according to one aspect of the present invention is represented by Formula 1 below.

<Formula 1>

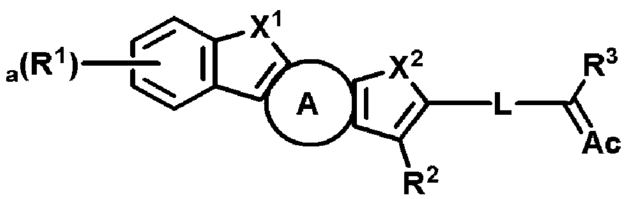

In Formula 1, the symbols are defined as set forth below.

A ring of A is selected from the group consisting of the following Formula A-1 to A-4,

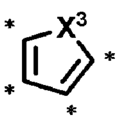
A-1

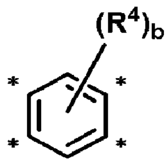
A-2

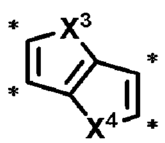
A-3

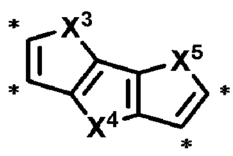
A-4

$X^1$ to $X^5$ are each independently O, S, Se, Te, $C(R^a)(R^b)$ or $Si(R^c)(R^d)$.

$R^1$ to $R^4$, $R^a$ to $R^d$ are each independently selected from the group consisting of hydrogen, deuterium, halogen, a $C_6$-$C_{60}$ aryl group, a fluorenyl group, a $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{30}$ alkyl group, a $C_2$-$C_{30}$ alkenyl group, a $C_2$-$C_{30}$ alkynyl group, a $C_1$-$C_{30}$ alkoxyl group, a $C_1$-$C_{30}$ alkylthio group, and a $C_6$-$C_{30}$ aryloxy group, and adjacent $R^1$ groups may be bonded to each other to form a ring, $R^a$ and $R^b$ may be bonded to each other to form

a ring, $R^c$ and $R^d$ may be bonded to each other to form a ring.

When adjacent $R^1$ groups are bonded to each other to form a ring, the ring may be selected from the group consisting of a $C_6$-$C_{60}$ aromatic ring group; a fluorenyl group; a $C_3$-$C_{60}$ heterocyclic group containing at least one heteroatom of O, N, S, Si and P; a $C_3$-$C_{60}$ aliphatic ring; and a fused ring of a $C_3$-$C_{60}$ aliphatic ring and a $C_6$-$C_{60}$ aromatic ring.

a is an integer from 0 to 4, b is an integer from 0 to 2, when these are integers of 2 or greater, each of the plurality of $R^1$, and each of the plurality of $R^4$ may be the same as or different from each other.

When $R^a$ and $R^b$ are bonded to each other to form a ring, a spiro ring having C as the spiro atom may be formed, and the ring may be selected from the group consisting of a $C_6$-$C_{30}$ aromatic ring group; a fluorenyl group; a $C_3$-$C_{30}$ heterocyclic group containing at least one heteroatom of O, N, S, Si, P and Se; a $C_3$-$C_{30}$ aliphatic ring; and a fused ring of a $C_3$-$C_{30}$ aliphatic ring and a $C_6$-$C_{30}$ aromatic ring.

For example, when $R^a$ and $R^b$ are bonded to each other to form an aliphatic ring group, the aliphatic ring group, may be, for example, a $C_3$-$C_{30}$, a $C_3$-$C_{29}$, a $C_3$-$C_{28}$, a $C_3$-$C_{27}$, a $C_3$-$C_{26}$, a $C_3$-$C_{25}$, a $C_3$-$C_{24}$, a $C_3$-$C_{23}$, a $C_3$-$C_{22}$, a $C_3$-$C_{21}$, a $C_3$-$C_{20}$, a $C_3$-$C_{19}$, a $C_3$-$C_{18}$, a $C_3$-$C_{17}$, a $C_3$-$C_{16}$, a $C_3$-$C_{15}$, a $C_3$-$C_{14}$, a $C_3$-$C_{13}$, a $C_3$-$C_{12}$, a $C_3$-$C_{11}$, a $C_3$-$C_{10}$, a $C_3$-$C_8$, a $C_3$-$C_6$, a $C_5$-$C_6$, a $C_6$, a $C_{10}$, a $C_{11}$, a $C_{12}$, a $C_{13}$, a $C_{14}$, a $C_{15}$, a $C_{16}$, a $C_{17}$ or a $C_{18}$ aliphatic ring group, specifically, a cyclopentanyl group, a cyclohexanyl group, an indenyl group, a tetralin group, a norbornyl group, an adamantyl group, etc.

When $R^c$ and $R^d$ are bonded to each other to form a ring, a $C_3$-$C_{60}$ heterocyclic group having Si as the spiro atom may be formed.

L is selected from the group consisting of a single bond, a $C_6$-$C_{60}$ arylene group, a fluorenylene group, a $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{30}$ alkylene group, a $C_2$-$C_{30}$ alkenylene group, a $C_2$-$C_{30}$ alkynylene group, and a $C_1$-$C_{30}$ akoxylene group.

Ac is selected from the group consisting of a $C_2$-$C_{60}$ heterocyclic group containing at least one heteroatom of O, N, S, Si and P, a $C_3$-$C_{60}$ aliphatic ring group, and a fused ring of a $C_3$-$C_{60}$ aliphatic ring and a $C_6$-$C_{60}$ aromatic ring, the Ac comprises one or more functional groups selected from the group consisting of C=O, C=S, C=Se, CN and $CF_3$.

L and $R^3$ may be bonded to each other to form a ring, and $R^3$ and Ac may be bonded to each other to form a ring.

When L and $R^3$ or $R^3$ and Ac are bonded to each other to form a ring, the ring may be selected from the group consisting of a $C_6$-$C_{60}$ aromatic ring group; a fluorenyl group; a $C_2$-$C_{60}$ heterocyclic group containing at least one heteroatom of O, **N,** S, Si and P; a $C_3$-$C_{60}$ aliphatic ring; and a fused ring of a $C_3$-$C_{60}$ aliphatic ring and a $C_6$-$C_{60}$ aromatic ring.

For example, when L and $R^3$ or $R^3$ and Ac are bonded to each other to form an aliphatic ring group, the aliphatic ring group may be a $C_3$-$C_{30}$, a $C_3$-$C_{29}$, a $C_3$-$C_{28}$, a $C_3$-$C_{27}$, a $C_3$-$C_{26}$, a $C_3$-$C_{25}$, a $C_3$-$C_{24}$, a $C_3$-$C_{23}$, a $C_3$-$C_{22}$, a $C_3$-$C_{21}$, a $C_3$-$C_{20}$, a $C_3$-$C_{19}$, a $C_3$-$C_{18}$, a $C_3$-$C_{17}$, a $C_3$-$C_{16}$, a $C_3$-$C_{15}$, a $C_3$-$C_{14}$, a $C_3$-$C_{13}$, a $C_3$-$C_{12}$, a $C_3$-$C_{11}$, a $C_3$-$C_{10}$, a $C_3$-$C_8$, a $C_3$-$C_6$, a $C_5$-$C_6$, a $C_6$, a $C_{10}$, a $C_{11}$, a $C_{12}$, a $C_{13}$, a $C_{14}$, a $C_{15}$, a $C_{16}$, a $C_{17}$ or a $C_{18}$ aliphatic ring group, and may contain one or more double bonds within the ring, specifically, may be a cyclopentenyl group, a cyclohexenyl group, a cyclopentanyl group, a cyclohexanyl group, an indenyl group, a tetralin group, a norbornyl group, an adamantyl group, etc.

In addition, when L and $R^3$ or $R^3$ and Ac are bonded to each other to form a heterocycle, the heterocycle may be a $C_2$-$C_{24}$, a $C_2$-$C_{23}$, a $C_2$-$C_{22}$, a $C_2$-$C_{21}$, a $C_2$-C20, a $C_2$-$C_{19}$, a $C_2$-$C_{18}$, a $C_2$-a $C_{17}$, a $C_2$-$C_{16}$, a $C_2$-$C_{15}$, a $C_2$-$C_{14}$, a $C_2$-$C_{13}$, a $C_2$-$C_{12}$, a $C_2$-$C_{11}$, a $C_2$-$C_{10}$, a $C_2$-$C_9$, a $C_2$-$C_8$, a $C_2$-$C_7$, a $C_2$-$C_6$, a $C_2$-$C_5$, a $C_2$-$C_4$, a $C_2$-$C_3$, a $C_3$-C8, a $C_4$-$C_8$, a $C_2$, a $C_3$, a $C_4$, a $C_5$, a $C_6$, a $C_7$, a $C_8$, a $C_9$, a $C_{10}$, a $C_{11}$, a $C_{12}$, a $C_{13}$, a $C_{14}$, a $C_{15}$, a $C_{16}$, a $C_{17}$, a $C_{18}$, a $C_{19}$, a $C_{20}$, a $C_{21}$, a $C_{22}$, a $C_{23}$, or a $C_{24}$ heterocycle. The heterocyclic group may be a monocyclic ring containing a heteroatom, a fused ring in which a heterocyclic ring is fused with another heterocyclic ring, or a fused ring in which a heterocyclic ring is fused with a hydrocarbon ring such as an alicyclic or aromatic ring. For example, the heterocyclic group may be 4H-pyran, furan, thiophene, benzothiophene, benzofuran, dibenzothiophene, and dibenzofuran.

Ac contains one or more functional groups selected from the group consisting of C=O, C=S, C=Se, CN, and $CF_3$. In the case where Ac contains a C=O, C=S, or C=Se functional group, it may be, for example, in the form in which O, S, or Se is double-bonded to a carbon forming a ring, and in the case where Ac contains CN or $CF_3$ as a functional group, it may be, for example, in the form in which these substituents are directly or indirectly attached to the carbon forming a ring. An indirectly substituted CN or $CF_3$ refers, for example, to a case where an alkenyl group is substituted on the Ac ring, and CN is further substituted on this alkenyl group. That is, a CN- or $CF_3$-substituted alkenyl group may, for example, be in the form in which $=CH_2$ is directly substituted on the Ac ring, and one or two hydrogens are replaced with CN to give a substituted form of =C(CN)(H) or $=C(CN)_2$.

When Ac is a fused ring of a $C_3$-$C_{60}$ aliphatic ring and a $C_6$-$C_{60}$ aromatic ring, the aliphatic ring group, may be, for example, a $C_3$-$C_{30}$, a $C_3$-$C_{29}$, a $C_3$-$C_{28}$, a $C_3$-$C_{27}$, a $C_3$-$C_{26}$, a $C_3$-$C_{25}$, a $C_3$-$C_{24}$, a $C_3$-$C_{23}$, a $C_3$-$C_{22}$, a $C_3$-$C_{21}$, a $C_3$-$C_{20}$, a $C_3$-$C_{19}$, a $C_3$-$C_{18}$, a $C_3$-$C_{17}$, a C3-C16, a C3-C15, a C3-C14, a C3-C13, a C3-C12, a C3-C11, a C3-C10, a C3-C8, a C3-$C_6$, a $C_5$-$C_6$, a $C_6$, a $C_{10}$, a $C_{11}$, a $C_{12}$, a $C_{13}$, a $C_{14}$, a $C_{15}$, a $C_{16}$, a $C_{17}$ or a $C_{18}$ aliphatic ring group, and may contain one or more double bonds within the ring, specifically, may be a cyclopentenyl group, a cyclohexenyl group, a cyclopentanyl group, a cyclohexanyl group, an indenyl group, a tetralin group, a norbornyl group, an adamantyl group, etc, and the aromatic ring

may be a $C_6$-$C_{20}$, a $C_6$-$C_{18}$, a $C_6$-$C_{16}$, a $C_6$-$C_{14}$, a $C_6$-$C_{13}$, a $C_6$-$C_{12}$, a $C_6$-$C_{10}$, a $C_6$, a $C_{10}$, a $C_{12}$, a $C_{14}$, a $C_{15}$, a $C_{16}$, a $C_{18}$ aromatic ring, specifically, may be an aryl ring such as benzene, naphthalene, anthracene, phenanthrene, pyrene, etc.

When Ac is a heterocycle, the heterocycle may be a $C_2$-$C_{24}$, a $C_2$-$C_{23}$, a $C_2$-$C_{22}$, a $C_2$-$C_{21}$, a $C_2$-C20, a $C_2$-$C_{19}$, a $C_2$-$C_{18}$, a $C_2$-a $C_{17}$, a $C_2$-$C_{16}$, a $C_2$-$C_{15}$, a $C_2$-$C_{14}$, a $C_2$-$C_{13}$, a $C_2$-$C_{12}$, a $C_2$-$C_{11}$, a $C_2$-$C_{10}$, a $C_2$-$C_9$, a $C_2$-$C_8$, a $C_2$-$C_7$, a $C_2$-$C_6$, a $C_2$-$C_5$, a $C_2$-$C_4$, a $C_2$-$C_3$, a $C_3$-C8, a $C_4$-$C_8$, a $C_2$, a $C_3$, a $C_4$, a $C_5$, a $C_6$, a $C_7$, a $C_8$, a $C_9$, a $C_{10}$, a $C_{11}$, a $C_{12}$, a $C_{13}$, a $C_{14}$, a $C_{15}$, a $C_{16}$, a $C_{17}$, a $C_{18}$, a $C_{19}$, a $C_{20}$, a $C_{21}$, a $C_{22}$, a $C_{23}$, a $C_{24}$ heterocycle. The heterocyclic group may be a monocyclic ring containing a hetero atom, a fused ring in which a heterocyclic ring is fused to another heterocyclic ring, or a fused ring in which a hydrocarbon ring such as an aliphatic or aromatic ring is fused to a heterocyclic ring. For example, the heterocyclic group may be a monocyclic ring such as furan, tetrahydrofuran, thiophene, pyrazolidine, pyridine, pyrimidine, pyrazine, tetrahydropyridine, hexahydropyrimidine, dioxane, dithiane, or thiazolidine, or a fused ring in which a hydrocarbon ring such as cyclopentane, cyclohexane, benzene, naphthalene, phenanthrene, or triphenylene is fused thereto, or a fused ring in which a heterocyclic ring such as thiophene, benzothiophene, dibenzothiophene, furan, benzofuran, dibenzofuran, thienothiophene, quinoline, or quinazoline is fused. In this case, the monocyclic or fused ring contains one or more functional groups selected from the group consisting of C=O, C=S, C=Se, C=Te, CN, and $CF_3$.

When at least one of $R^1$ to $R^4$, $R^a$ to $R^d$ is an aryl group, the aryl group may be a $C_6$-$C_{20}$, a $C_6$-$C_{18}$, a $C_6$-$C_{16}$, a $C_6$-$C_{14}$, a $C_6$-$C_{13}$, a $C_6$-$C_{12}$, a $C_6$-$C_{10}$, a $C_6$, a $C_{10}$, a $C_{12}$, a $C_{14}$, a $C_{15}$, a $C_{16}$, or a $C_{18}$ aromatic ring, specifically, an aromatic ring such as benzene, naphthalene, anthracene, phenanthrene, pyrene, etc.

When at least one of $R^1$ to $R^4$, $R^a$ to $R^d$ is a heterocycle, the heterocycle may be a $C_2$-$C_{24}$, a $C_2$-$C_{23}$, a $C_2$-$C_{22}$, a $C_2$-$C_{21}$, a $C_2$-C20, a $C_2$-$C_{19}$, a $C_2$-$C_{18}$, a $C_2$-a $C_{17}$, a $C_2$-$C_{16}$, a $C_2$-$C_{15}$, a $C_2$-$C_{14}$, a $C_2$-$C_{13}$, a $C_2$-$C_{12}$, a $C_2$-$C_{11}$, a $C_2$-$C_{10}$, a $C_2$-$C_9$, a $C_2$-$C_8$, a $C_2$-$C_7$, a $C_2$-$C_6$, a $C_2$-$C_5$, a $C_2$-$C_4$, a $C_2$-$C_3$, a $C_3$-C8, a $C_4$-$C_8$, a $C_2$, a $C_3$, a $C_4$, a $C_5$, a $C_6$, a $C_7$, a $C_8$, a $C_9$, a $C_{10}$, a $C_{11}$, a $C_{12}$, a $C_{13}$, a $C_{14}$, a $C_{15}$, a $C_{16}$, a $C_{17}$, a $C_{18}$, a $C_{19}$, a $C_{20}$, a $C_{21}$, a $C_{22}$, a $C_{23}$, or a $C_{24}$ heterocycle, specifically, thiophene, benzothiophene, dibenzothiophene, furan, benzofuran, dibenzofuran, etc.

When at least one of $R^1$ to $R^4$, $R^a$ to $R^d$ is an alkyl group, the alkyl group may be a $C_1$-$C_{20}$, a $C_1$-$C_{10}$, a $C_1$-$C_4$, a $C_1$, a $C_2$, a $C_3$, or a $C_4$ alkyl group, for example, methyl, ethyl, t-butyl, etc.

The aryl group, the arylene group, the fluorenyl group, the fluorenylene group, the heterocyclic group, the aliphatic ring group, the fused ring, the alkyl group, the alkylene group, the alkenyl group, the alkenylene group, the alkynyl group, the alkynylene group, the alkoxy group, the alkoxylene group, the arylthio group, the aryloxyl group, and the ring formed by adjacent groups may be each substituted with one or more substituents selected from the group consisting of deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide substituted or unsubstituted with a $C_1$-$C_{20}$ alkyl group or a $C_6$-$C_{20}$ aryl group, $C_1$-$C_{20}$ alkylthio group, $C_1$-$C_{20}$ alkoxy group, $C_6$-$C_{30}$ aryloxy group, $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{60}$ heterocyclic group comprising at least one heteroatom selected from the group consisting of O, N, S, Si and P, a $C_3$-$C_{30}$ aliphatic ring group, a fused ring of a $C_3$-$C_{30}$ aliphatic ring and a $C_6$-$C_{30}$ aromatic ring, =O, =C(CN)$_2$, and =S.

When at least one of the aryl group, the arylene group, the fluorenyl group, the fluorenylene group, the heterocyclic group, the aliphatic ring group, the fused ring, the alkyl group, the alkylene group, the alkenyl group, the alkenylene group, the alkynyl group, the alkynylene group, the alkoxy group, the alkoxylene group, the arylthio group, the aryloxyl group, and the ring formed by adjacent groups is substituted with an alkyl group, the alkyl group may be, for example, a $C_1$-$C_{20}$, a $C_1$-$C_{10}$, a $C_1$-$C_4$, a $C_1$, a $C_2$, a $C_3$, or a $C_4$ alkyl group, for example, methyl, ethyl, t-butyl, etc.

Formula 1 may be represented by one of Formula 2 to Formula 15.

<Formula 2>

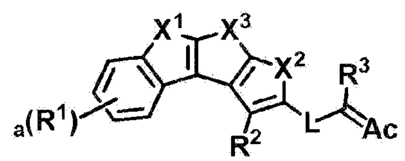

[67]

<Formula 3>

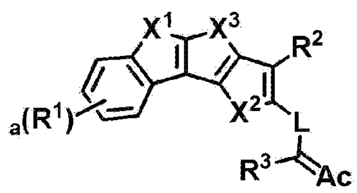

<Formula 4>

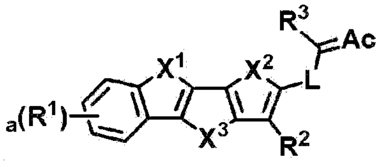

<Formula 5>

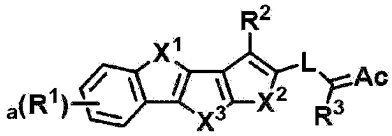

<Formula 6>

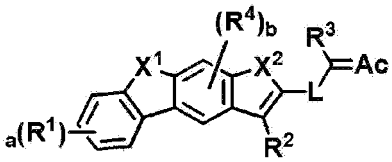

<Formula 7>

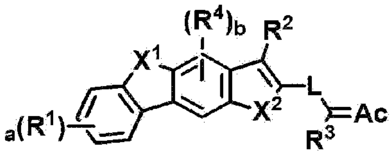

<Formula 8>

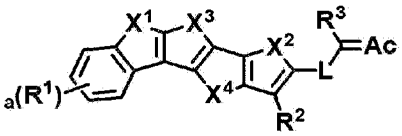

<Formula 9>

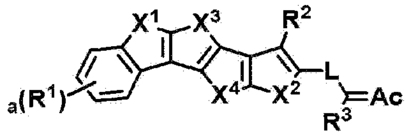

<Formula 10>

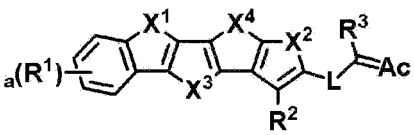

<Formula 11>

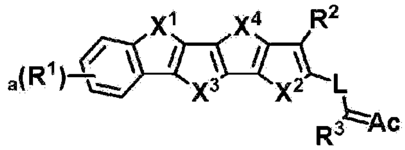

<Formula 12>

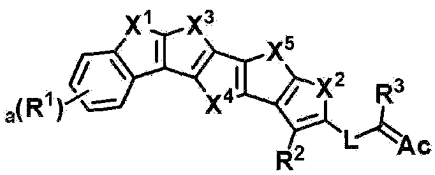

<Formula 13>

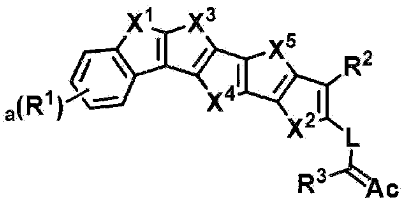

<Formula 14>

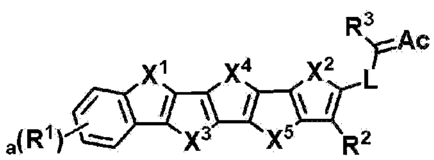

<Formula 15>

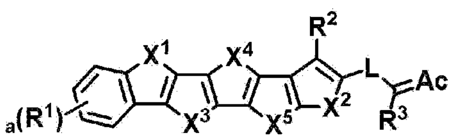

In Formula 2 to Formula 15, $X^1$ to $X^5$, $R^1$ to $R^3$, L, Ac, a are the same as defined for Formula 1.

In addition, Formula 1 may be represented by one of Formulae 1-1 to 1-6 below. Formulae 1-1 and 1-2 correspond to cases where $R^3$ and L are bonded to each other to form an aliphatic ring, Formulae 1-3 and 1-4 correspond to cases where $R^3$ and L are bonded to each other to form a heterocyclic ring, and Formulae 1-5 and 1-6 correspond to cases where $R^3$ and Ac are bonded to each other to form a heterocyclic ring.

<Formula 1-1>

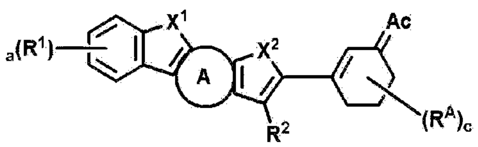

<Formula 1-2>

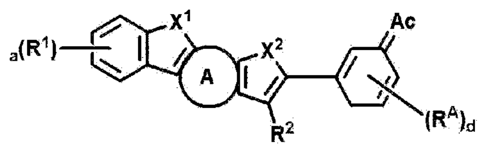

<Formula 1-3>

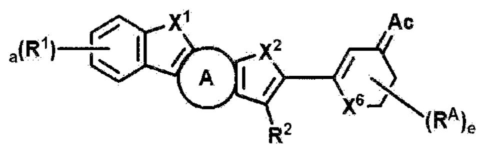

<Formula 1-4>

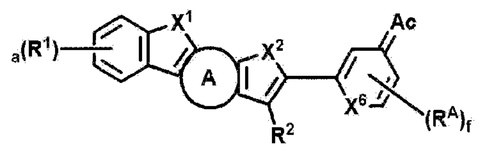

<Formula 1-5>

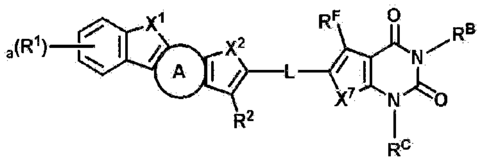

<Formula 1-6>

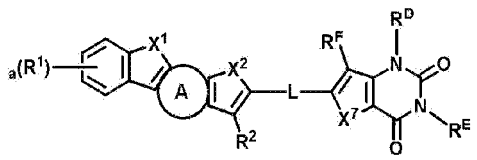

In Formula 1-1 to Formula 1-6, A ring, $X^1$, $X^2$, $R^1$, $R^2$, Ac, L, a are the same as defined for Formula 1, and $X^6$ and $X^7$ are each O or S.

$R^A$ to $R^F$ are each independently selected from the group consisting of hydrogen, deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide substituted or unsubstituted with a $C_1$-$C_{20}$ alkyl group or a $C_6$-$C_{20}$ aryl group, $C_1$-$C_{20}$ alkylthio group, $C_1$-$C_{20}$ alkoxy group, $C_6$-$C_{30}$ aryloxy group, $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{24}$ heterocyclic group comprising at least one heteroatom selected from the group consisting of O, N, S, Si and P, and a $C_3$-$C_{30}$ aliphatic ring group, a fused ring of a $C_3$-$C_{30}$ aliphatic ring and a $C_6$-$C_{30}$ aromatic ring, =O, =C(CN)$_2$, and =S.

c is an integer of 0 to 7, d and e are each an integer of 0 to 5, f is an integer of 0 to 3, when these are integers of 2 or more, a plurality of $R^A$ groups may be the same or different, and adjacent groups may be bonded to each other to form a ring.

In addition, in Formula 1, Ac may be selected from the group consisting of the following Formula 16 to Formula 18.

<Formula 16>

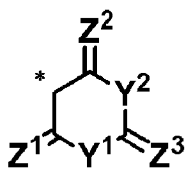

<Formula 17>

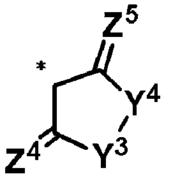

<Formula 18>

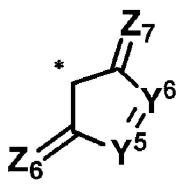

In Formula 16 to Formula 18, the symbols are defined as set forth below.

$Z^1$ to $Z^5$, $Z_6$ and $Z_7$ are each independently O, S, Se, Te or C($R^e$)($R^f$), $Y^1$ to $Y^4$ are each independently O, S, Se, Te, C($R^g$)($R^h$) or N($R^i$), and $Y^5$ and $Y^6$ are each independently N or C($R^i$).

$R^e$ to $R^i$ are each independently selected from the group consisting of hydrogen, deuterium, halogen, a cyano group, a $C_1$-$C_{20}$ alkyl group, and a $C_2$-$C_{20}$ alkenyl group.

$R^j$ is selected from the group consisting of hydrogen, deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide substituted or unsubstituted with a $C_1$-$C_{20}$ alkyl group or a $C_6$-$C_{20}$ aryl group, $C_1$-$C_{20}$ alkylthio group, $C_1$-$C_{20}$ alkoxy group, $C_6$-$C_{30}$ aryloxy group, $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{60}$ heterocyclic group comprising at least one heteroatom selected from the

group consisting of O, N, S, Si and P, a $C_3$-$C_{30}$ aliphatic ring group, a fused ring of a $C_3$-$C_{30}$ aliphatic ring and a $C_6$-$C_{30}$ aromatic ring, =O, =C(CN)$_2$, and =S, and adjacent groups may be bonded to each other to form a ring.

Formula 16 may be represented by the following Formulae, but is not limited thereto.

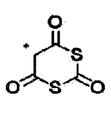 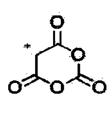 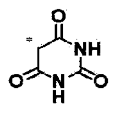 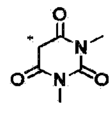 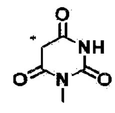 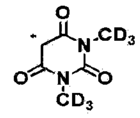

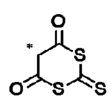 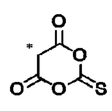 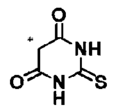 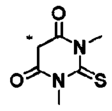 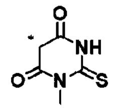 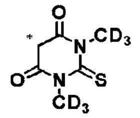

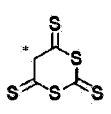 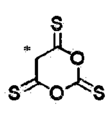 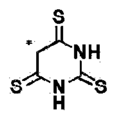 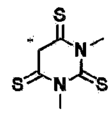 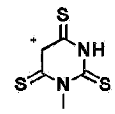 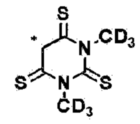

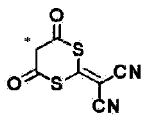 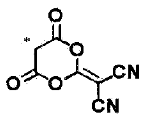 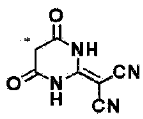 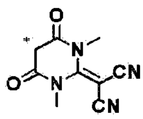 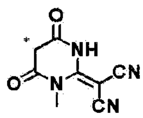 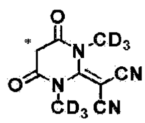

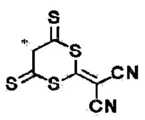 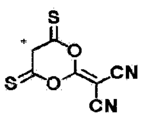 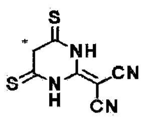 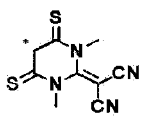 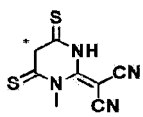 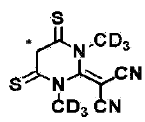

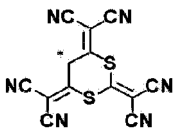 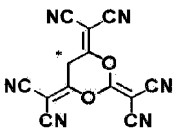 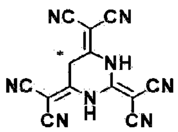 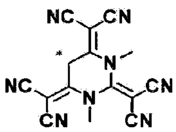 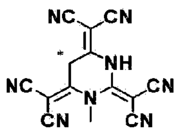 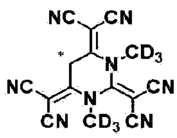

Formula 17 or Formula 18 may be represented by the following Formulae, but is not limited thereto.

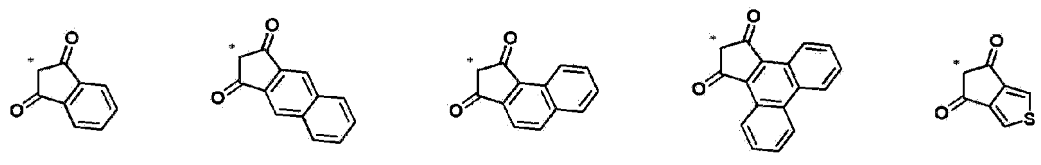

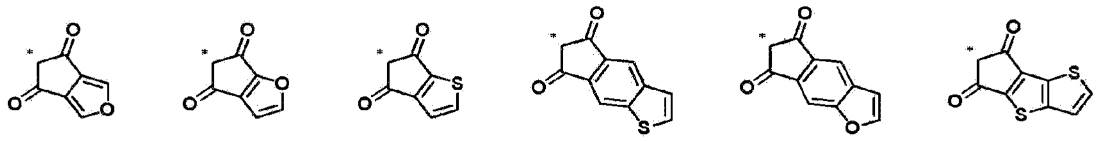

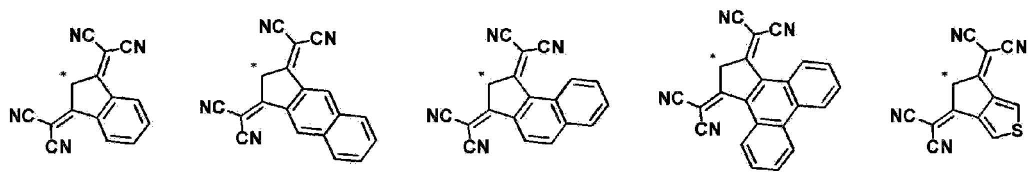

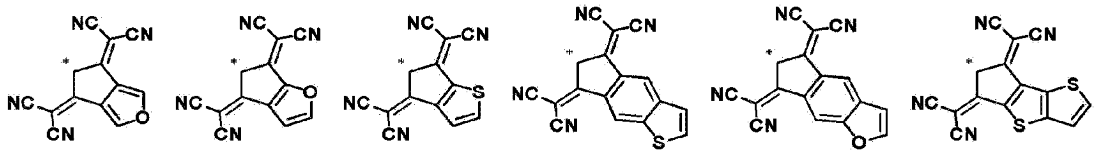

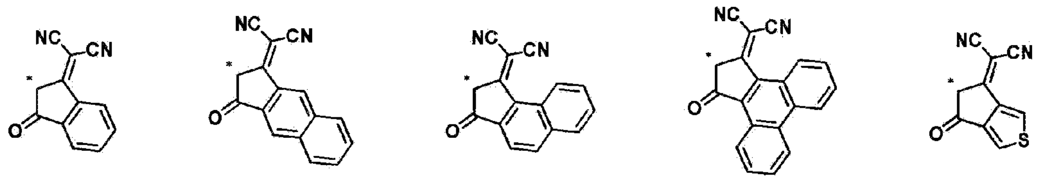

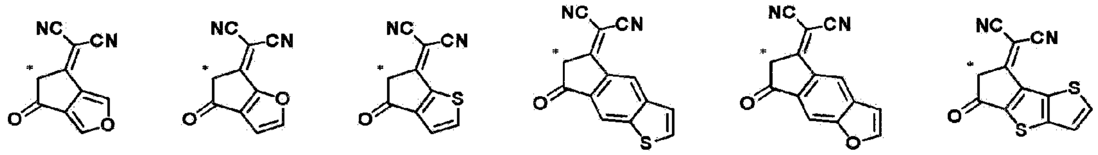

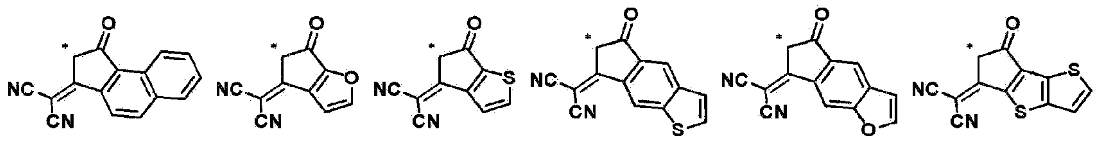

Specifically, Formula 1 may be one of the following compounds, but is not limited thereto.

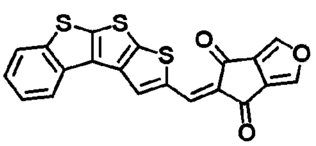

P1

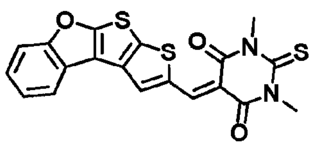

P2

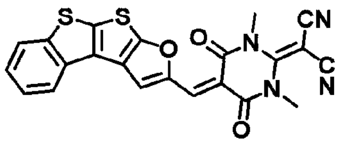

P3

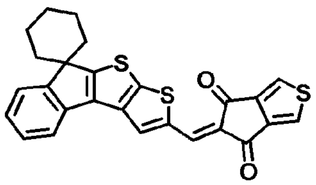

P4

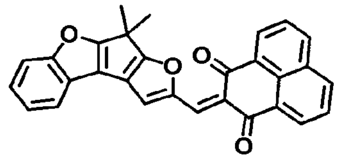

P5

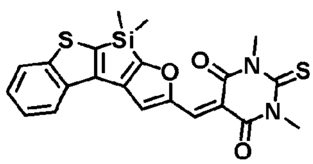

P6

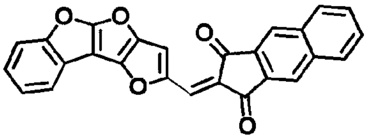

P7

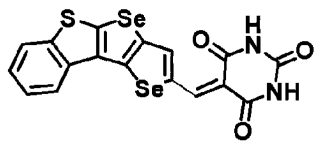

P8

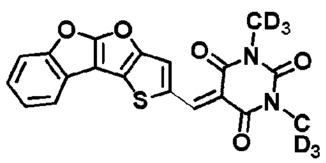

P9

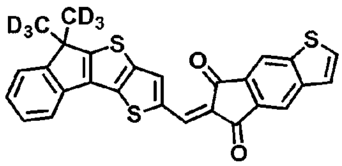

**P10**

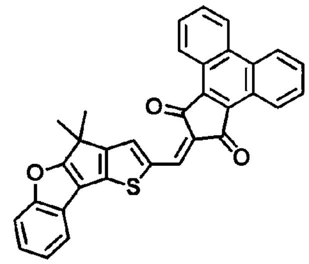

**P11**

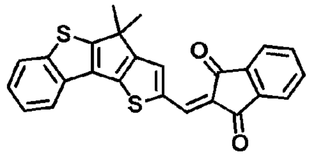

**P12**

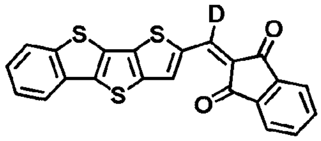

**P13**

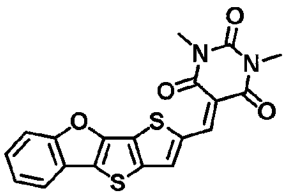

**P14**

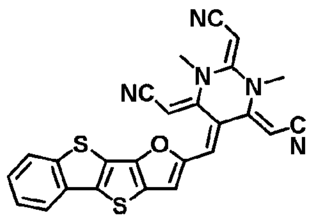

**P15**

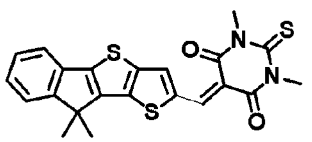

**P16**

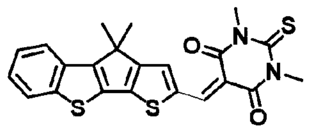

**P17**

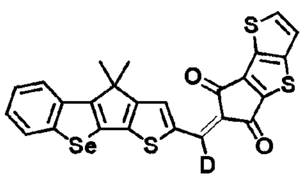

**P18**

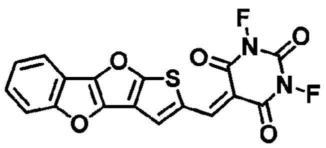

**P19**

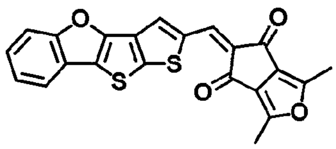

**P20**

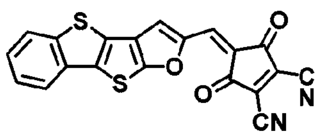

**P21**

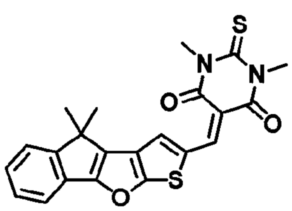

**P22**

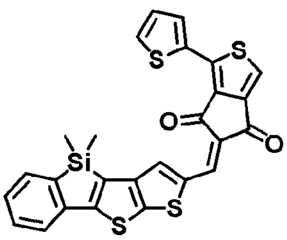

**P23**

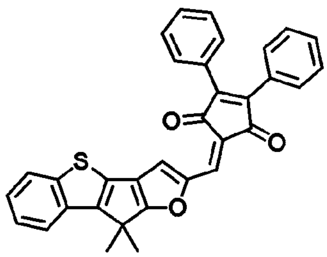

**P24**

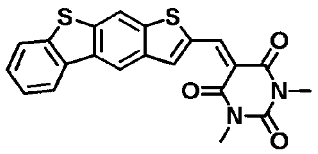

**P25**

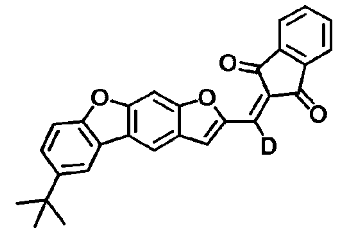

**P26**

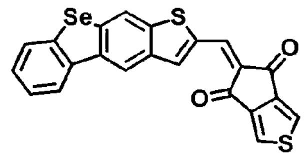

**P27**

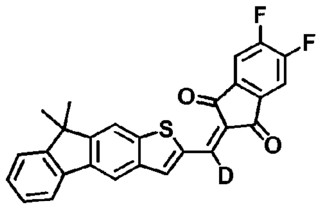

**P28**

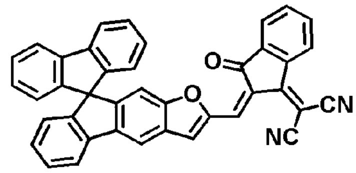

**P29**

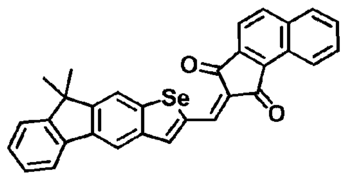

**P30**

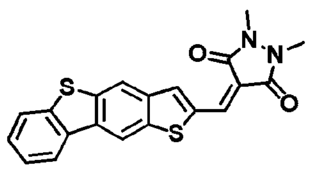

**P31**

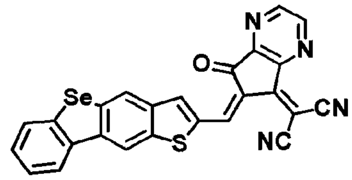

**P32**

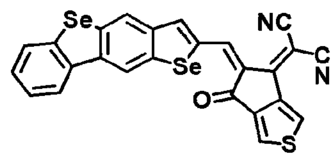

**P33**

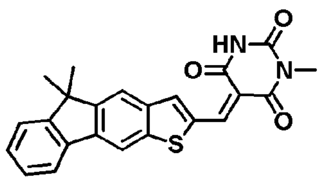

**P34**

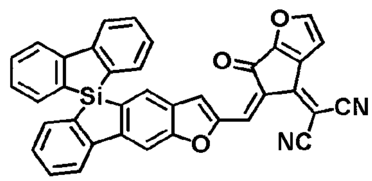

**P35**

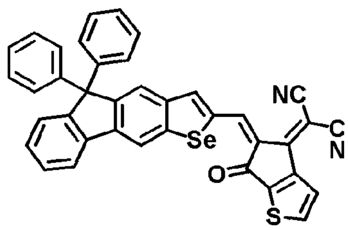

**P36**

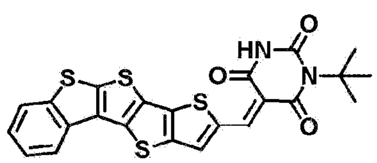

**P37**

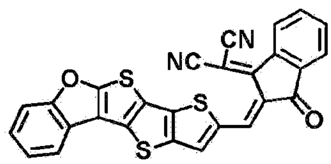

**P38**

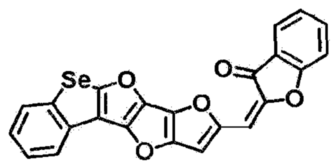

**P39**

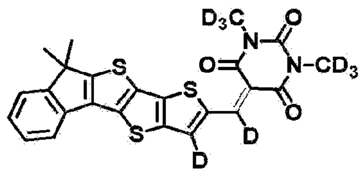

**P40**

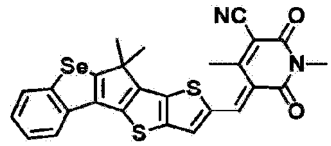

**P41**

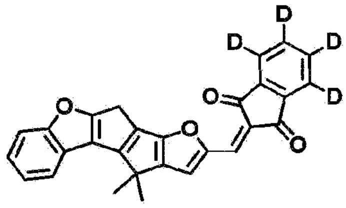

**P42**

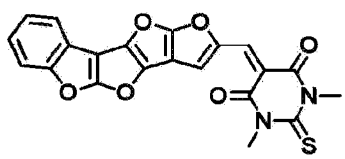

**P43**

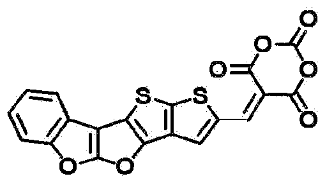

**P44**

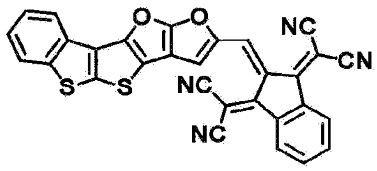

**P45**

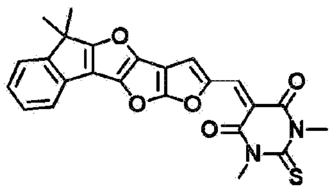

**P46**

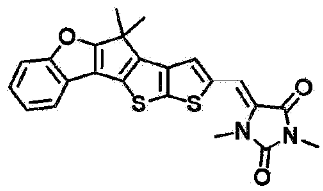

**P47**

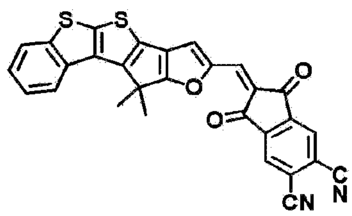

**P48**

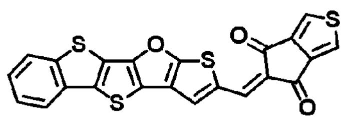

**P49**

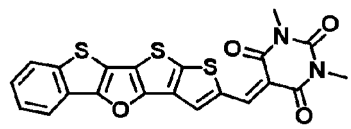

**P50**

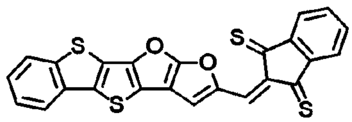

**P51**

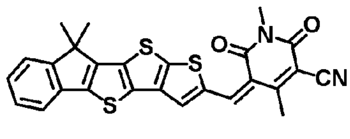

**P52**

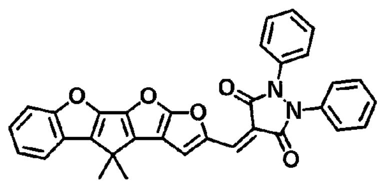

**P53**

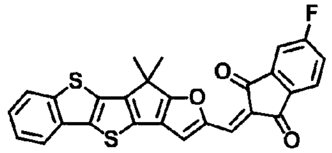

**P54**

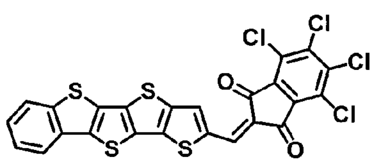

**P55**

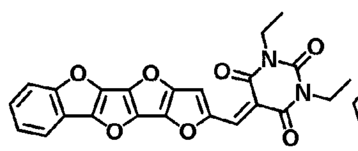

**P56**

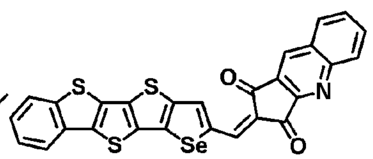

**P57**

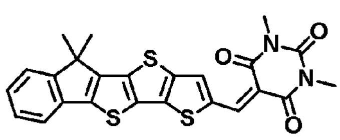

P58

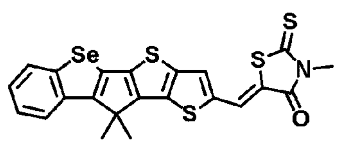

P59

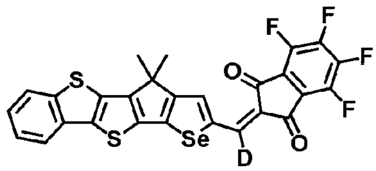

P60

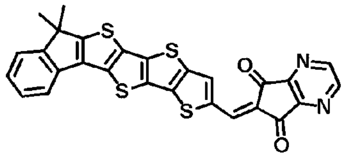

P61

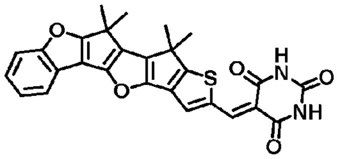

P62

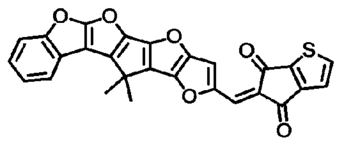

P63

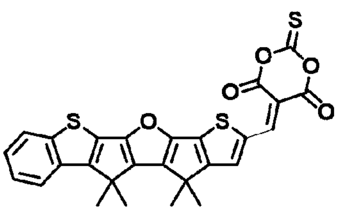

P64

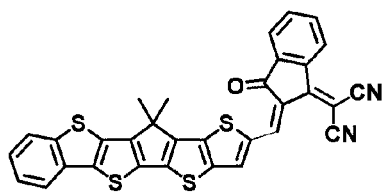

P65

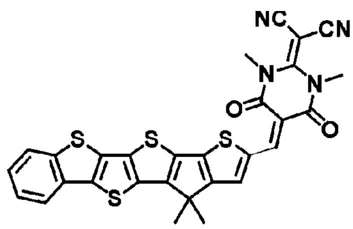

P66

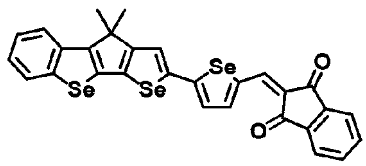

P67

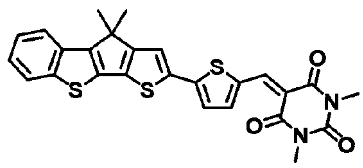

P68

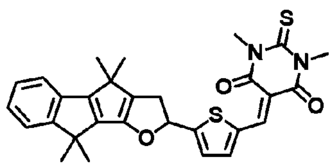

P69

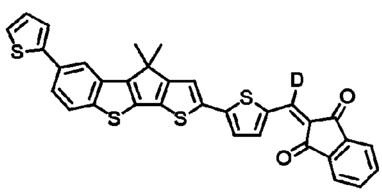

P70

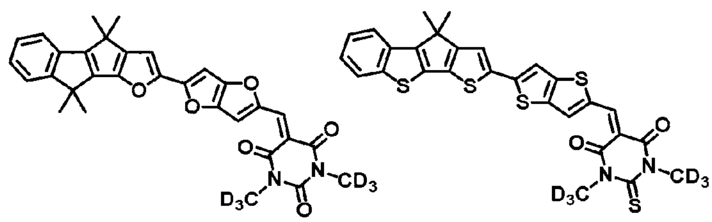

P71

P72

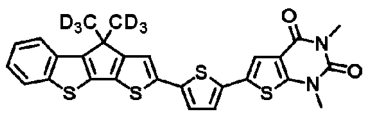

P73

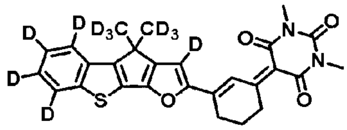

P74

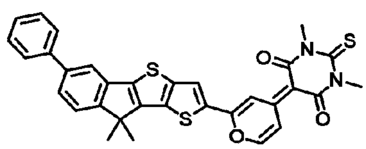

P75

The compound represented by Formula 1 of the present invention is comprised in the active layer of an organic photoelectric element. That is, the compound of Formula 1 according to the present invention can be used as a compound for an organic photoelectric element.

Hereinafter, an organic photoelectric element comprising a compound of Formula 1 according to the present invention will be described with reference to Figures 1 and 2.

Figures 1 and 2 shows the laminated structure of an organic photoelectric element according to an embodiment of the present invention.

In the designation of reference numerals for components in the respective drawings, it should be understood that the same elements are denoted by the same reference numerals, even if they appear in different drawings. Furthermore, in the following description of the present invention, detailed explanations of well-known functions and configurations will be omitted where they may unnecessarily obscure the essence of the invention.

Terms such as "first," "second," "A," "B," "(a)," "(b)," and the like may be used to describe various components of the present invention. These terms are merely intended to distinguish one component from another and do not imply any particular order, importance, or essential characteristics. Furthermore, it should be understood that when a component is described as being "connected," "coupled," or "joined" to another component, this may include both direct connections as well as indirect connections through one or more intervening components.

Additionally, it is to be understood that when an element such as a layer, film, region, or substrate is described as being "on" or "over" another element, it may be positioned directly on the other element or with one or more intervening layers therebetween. In contrast, the expression "directly on" indicates that no intervening elements are present between the two elements.

Referring to Figure 1, an organic photoelectric element according to an embodiment of the present invention comprises a first electrode 110, a second electrode 120, and an active layer 130 disposed between the first electrode 110 and the second electrode 120. Referring to Figure 2, the organic photoelectric element may further comprise a hole transport region 210 between the first electrode 110 and the active layer 130, and an electron transport region 220 between the second electrode 120 and the active layer 130.

One of the first electrode 110 and the second electrode 120 serves as the cathode, and the other serves as the anode. An anode may be a transparent electrode. Typically, materials for forming the transparent electrode comprises transparent conductive oxides (TCOs), such as indium tin oxide (ITO), zinc oxide (ZnO), tin oxide ($SnO_2$), indium zinc oxide (IZO), or combinations thereof, but are not limited thereto. The anode may be formed as a multilayer comprising two or more layers.

A cathode is preferably composed of one or more metal materials, which may be selected from magnesium (Mg), silver (Ag), aluminum (Al), lithium (Li), calcium (Ca), indium (In), and combinations thereof. Preferably, a cathode is composed of a mixture of Mg and Ag, but is not limited thereto. A cathode may also be formed as a multilayer comprising two or more layers.

When light is incident from the outside, the compound in the active layer 130 is excited by the photoelectric effect, resulting in the generation of excitons. The generated excitons are dissociated into holes and electrons, which then migrate to a first electrode and a second electrode 120, respectively, thereby generating an electrical signal. For example, when a first electrode 110 serves as the anode, holes move toward a first electrode 110 while electrons move toward a second electrode 120, generating an electrical signal.

The active layer 130 may be formed as a single layer or multiple layers and may include two or more different compounds. The active layer 130 comprises p-type semiconductor compounds and n-type semiconductor compounds, thereby forming a p-n junction.

The compound represented by Formula 1 of the present invention is comprised in the active layer 130, and preferably serves as a p-type semiconductor compound in the active layer 130. To form a p-n junction in the active layer 130, the active layer 130 may further comprise a n-type semiconductor compound.

The n-type semiconductor compound may be fullerene, a fullerene derivative, a compound represented by at least one of Formulae 30 to 33, or a mixture thereof, but these are merely examples of n-type semiconductor compounds and are not intended to be limiting.

The fullerene may be composed of a $C_{60}$-$C_{540}$ hydrocarbon ring. For example, the fullerene may be $C_{60}$, $C_{70}$, $C_{76}$, $C_{78}$, $C_{80}$, $C_{82}$, $C_{84}$, $C_{90}$, $C_{96}$, $C_{240}$, $C_{540}$, mixtures thereof, fullerene nanotubes, etc.

The fullerene derivative refers to a compound having substituents on the fullerene. The fullerene derivative may be a fullerene substituted with substituents such as a $C_1$-$C_{30}$ alkyl group, a $C_6$-$C_{30}$ aryl group, or a $C_3$-$C_{30}$ heterocyclic group.

The aryl group may be phenyl, naphthyl, biphenyl, terphenyl, anthracenyl, phenanthrenyl, fluorenyl, triphenylenyl, naphthacenyl, etc., the heterocyclic group may be pyrrole, furan, thiophene, imidazole, oxazole, thiazole, pyridine, pyrazine, pyrimidine, pyridazine, indolizine, indole, benzofuran, benzothiophene, isobenzofuran, benzimidazole, imidazopyridine, quinolizine, quinoline, phthalazine, naphthyridine, quinoxaline, quinazoline, isoquinoline, carbazole, phenanthridine, acridine, phenanthroline, thiatrene, chromene, xanthene, phenoxazine, phenoxathiin, phenothiazine, or phenazine ring, but is not limited thereto.

<Formula 30>

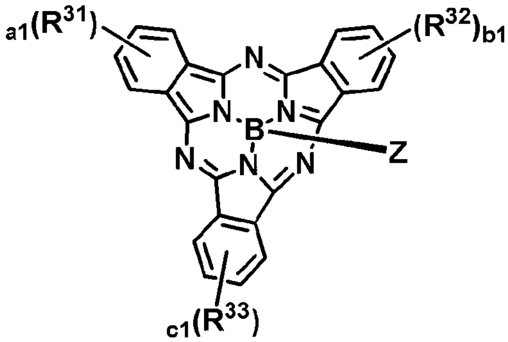

In Formula 30, the symbols are defined as set forth below.

$R^{31}$ to $R^{33}$ are each independently selected from the group consisting of hydrogen, deuterium, halogen, a cyano group, a $C_6$-$C_{60}$ aryl group, a fluorenyl group, a $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{20}$ alkyl group, a $C_2$-$C_{20}$ alkenyl group, a $C_2$-$C_{20}$ alkynyl group, a $C_1$-$C_{20}$ alkoxyl group, and a $C_6$-$C_{60}$ aryloxy group, and adjacent groups may be bonded to each other to form a ring.

a1, b1, and c1 are each integers from 1 to 3, and when these are integers of 2 or greater, each of $R^{31}$, each of $R^{32}$, and each of $R^{33}$ may be the same as or different from each other.

Z is a monovalent substituent and may be a halogen or a halogen-containing group, for example, F, Cl, a fluorine-containing group, or a chlorine-containing group.

<Formula 31>

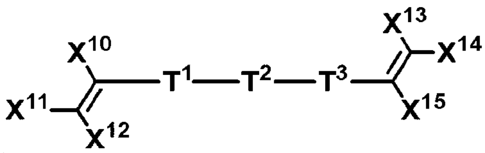

<Formula 32>

EWG$^1$——T$^1$——T$^2$——T$^3$—EWG$^2$

In Formulas 31 and 32, the symbols are defined as set forth below.

$T^1$ to $T^3$ may be heterocyclic groups containing a thiophene ring, and $T^1$ to $T^3$ may be bonded to each other to form a ring.

$X^{10}$ to $X^{15}$ are each independently selected from the group consisting of hydrogen, deuterium, halogen, a cyano group, a $C_6$-$C_{60}$ aryl group, a fluorenyl group, a $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{20}$ alkyl group, a $C_2$-$C_{20}$ alkenyl group, a $C_2$-$C_{20}$ alkynyl group, a $C_1$-$C_{20}$ alkoxyl group, and a $C_6$-$C_{60}$ aryloxy group.

EWG1 and EWG2 represent electron withdrawing groups. For example, at least one of $X^{10}$ to $X^{15}$ in Formula 31 may be an electron withdrawing group, preferably CN or a CN-containing group.

<Formula 33>

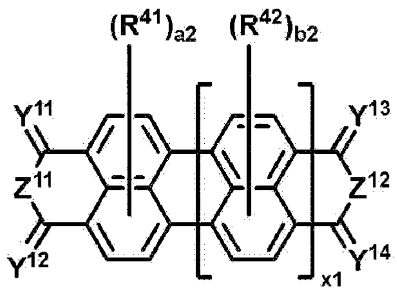

In Formula 33, the symbols are defined as set forth below.

$Z^{11}$ and $Z^{12}$ are each independently $N(R_1)$, O or S, and $Y^{11}$ to $Y^{14}$ are $C(R_2)(R_3)$, O or S.

$R^{41}$, $R^{42}$, $R_1$ to $R_3$ are each independently selected from the group consisting of hydrogen, deuterium, halogen, a cyano group, a $C_6$-$C_{60}$ aryl group, a fluorenyl group, a $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{20}$ alkyl group, a $C_2$-$C_{20}$ alkenyl group, a $C_2$-$C_{20}$ alkynyl group, a $C_1$-$C_{20}$ alkoxyl group, and a $C_6$-$C_{60}$ aryloxy group, and adjacent groups may be bonded to each other to form a ring, and $R_2$ and $R^3$ may be bonded to each other to form a ring.

$R_2$ and $R^3$ are preferably halogen or CN.

a2 and b2 are each integers from 0 to 4, and x1 is an integer from 1 to 3.

In Formula 30 to Formula 33, the aryl group, the arylene group, the fluorenyl group, the fluorenylene group, the heterocyclic group, the aliphatic ring group, the fused ring, the alkyl group, the alkenyl group, the alkynyl group, the alkoxyl group, the aryloxyl group, and the ring formed by adjacent groups may be each substituted with one or more substituents selected from the group consisting of deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide unsubstituted or substituted with a $C_1$-$C_{20}$ alkyl group or a $C_6$-$C_{20}$ aryl group, a $C_1$-$C_{20}$ alkylthio group, a $C_1$-$C_{20}$ alkoxy group, a $C_6$-$C_{30}$ aryloxy group, a $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{24}$ heterocyclic group comprising at least one heteroatom selected from the group consisting of O, N, S, Si and P, a $C_3$-$C_{30}$ aliphatic ring, and a fused ring of a $C_3$-$C_{30}$ aliphatic ring and a $C_6$-$C_{30}$ aromatic ring.

Specifically, the compound represented by Formula 33 may be one of the following compounds, but is not limited thereto.

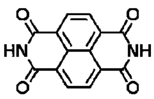
C-1

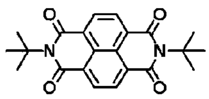
C-2

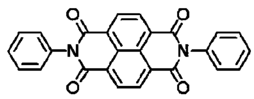
C-3

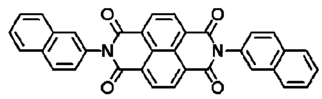
C-4

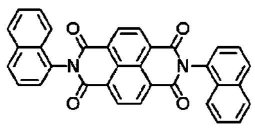
C-5

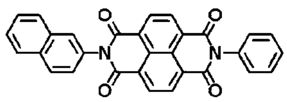
C-6

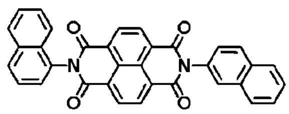
C-7

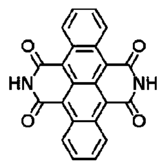
C-8

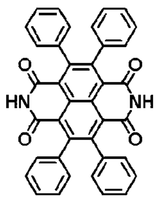
C-9

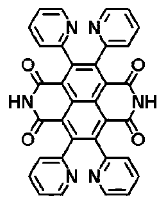
C-10

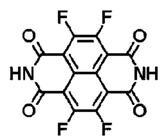
C-11

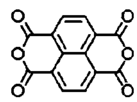
C-12

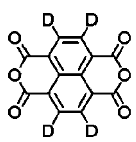
C-13

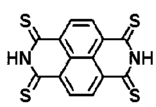
C-14

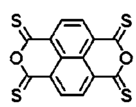
C-15

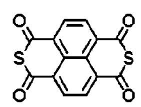
C-16

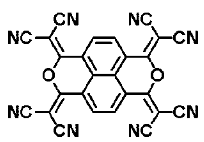
C-17

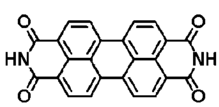
C-18

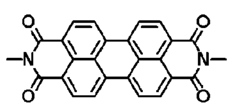
C-19

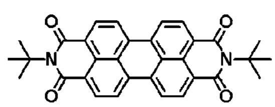
C-20

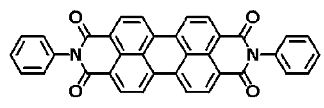 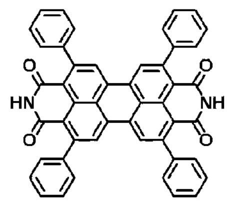 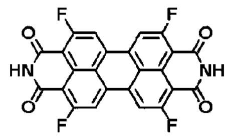 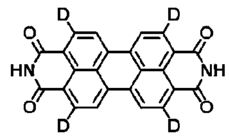

C-21 C-22 C-23 C-24

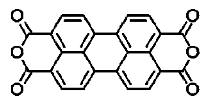 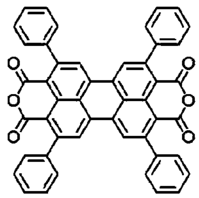 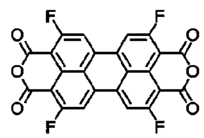 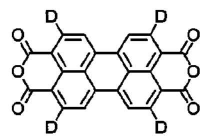

C-25 C-26 C-27 C-28

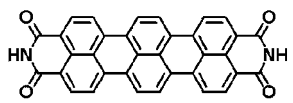 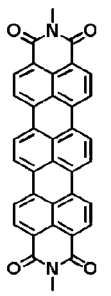 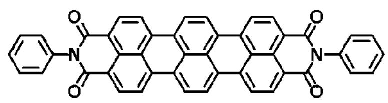 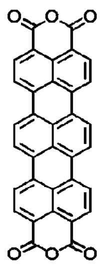

C-29 C-30 C-31 C-32

The compound represented by Formula 1 generally absorbs light in the visible region and preferably has a maximum absorption wavelength ($\lambda_{max}$) of 450 nm or more in a thin film state.

Particularly, it is preferable that the compound has a maximum absorption wavelength of 450 nm to 650 nm in a thin film state. That is, the active layer 130 containing the compound preferably has a maximum absorption wavelength of 450 nm or more as described above, and more preferably has a maximum absorption wavelength of 450 nm to 650 nm.

Therefore, the active layer 130 can selectively absorb and/or detect light in the green wavelength region and may serve as a substitute for the color filter of green pixels.

The compound represented by Formula 1 preferably has a small full width at half maximum (FWHM) in its absorption spectrum. A smaller FWHM indicates that the compound has a high selectivity for the wavelength of light it absorbs. In other words, a compound with a narrow FWHM can be regarded as a material having high selectivity and high sensitivity. Preferably, the active layer 130 containing the compound represented by Formula 1 has an FWHM of 50 nm to 100 nm.

When the compound represented by Formula 1 is applied as a p-type semiconductor compound, it is preferable that its LUMO energy level is higher than that of the n-type semiconductor with which it is mixed. The energy levels described below are expressed in absolute values. For example, when mixed with a n-type material such as fullerene, which has a LUMO energy level of about 4.2 eV, it is preferable that the compound represented by Formula 1 has a LUMO energy level higher than that of fullerene, specifically in the range of 3.7 to 2.7 eV.

Additionally, in order to absorb energy in the green wavelength region, it is preferable that the energy bandgap is 2.0 to 2.5 eV. Therefore, the HOMO energy level of the compound represented by Formula 1 is preferably in the range of 4.7 to 6.2 eV.

When the compound represented by Formula 1, which satisfies the above conditions, is used in the active layer 130, it can effectively absorb light in the green wavelength region and exhibit a high external quantum efficiency (EQE).

Additionally, as described above, by including the compound represented by Formula 1 in the active layer 130, aggregation among the compounds in the thin film state can be prevented, thereby maintaining the absorption characteristics according to wavelength. Accordingly, an organic photoelectric element that selectively absorbs light of the green wavelength can be provided. In particular, unnecessary absorption of light at wavelengths other than green can be reduced, enabling the provision of a highly sensitive organic photoelectric element.

The p-type semiconductor compound represented by Formula 1 and the n-type semiconductor compound may

be present in a volume ratio of about 9:1 to about 1:9, preferably about 1:3 to 1:5.

The ratio of the p-type semiconductor compound to the n-type semiconductor compound significantly affects the performance of the organic photoelectric element. When the ratio of p-type to n-type semiconductor compounds is 1:1, the absorption coefficient can be about $6.0 \times 10^4$ $cm^{-1}$ or higher, and it is preferable to have an absorption coefficient in the range of $6.0 \times 10^4$ $cm^{-1}$ to $1.0 \times 10^5$ $cm^{-1}$.

The active layer 130 containing the p-type or the n-type semiconductor compound semiconductor compound may be a mixture of two or more compounds, but is not limited thereto.

Photo-converted charges may remain unused and accumulate as residual charges. When the residual charge is high, even after continuous light exposure is stopped, the presence of residual charges can cause the sensor to perceive that light exposure is still occurring. Therefore, materials with high residual charge are unsuitable for application in image sensors. In other words, it is preferable to use materials with low residual charge, and using materials with low residual charge is necessary to provide high-sensitivity organic photoelectric elements.

To measure residual charge, light of a specific wavelength range is irradiated at a constant intensity for a certain period, then the light irradiation is stopped, and the current flowing after stopping the light irradiation is measured by integrating it over time.

An organic photoelectric element can be fabricated by forming the active layer 130 through high-temperature processes such as vacuum deposition. Vacuum deposition offers many advantages in the process because it is favorable for forming uniform thin films and has a low possibility of impurity contamination. However, if the decomposition temperature ($T_d$) of the compound is lower than the deposition temperature ($T_s$), the compound may decompose at high temperature and deposit along with decomposition products, which can degrade the overall performance of the device. Therefore, it is preferable that the decomposition temperature of the compound is higher than the deposition temperature.

In other words, when the deposition temperature (Ts) is higher than the decomposition temperature (Td), decomposition occurs before sublimation (deposition), preventing the fabrication of a proper device and causing performance degradation due to deposition of decomposed impurities.

Therefore, to manufacture a stable image sensor, the decomposition temperature ($T_d$) should be higher than the deposition temperature ($T_s$), and it is preferable that $T_d - T_s \geq 10°C$.

In addition, when manufacturing an image sensor, it is necessary to form a microlens array (MLA) after device fabrication for light collection, and the formation of the microlens array requires a high temperature (about 160°C-190°C or higher). Therefore, the organic photoelectric element must not degrade during the MLA heat treatment process.

In other words, degradation occurring during the MLA process does not mean chemical decomposition but refers to changes caused by morphology alteration. Morphology changes generally occur when thermal treatment initiates vibrational motion of the material.

Therefore, by designing the molecular structure robustly, the vibrational motion caused by heat can be reduced, preventing degradation due to thermal treatment. In the case of the compounds represented by Formula 1 of the present invention, having a conjugated structure in the core can inhibit molecular vibration, thereby improving process stability.

It is also preferable that among the compounds represented by Formula 1, those that can be stably deposited are used as materials for the active layer 130. For example, compounds with a molecular weight of about 300 to 1000 g/mol, preferably 300 to 800 g/mol, and more preferably 350 to 700 g/mol can be used in the deposition process.

When the compound represented by Formula 1 that satisfies the above conditions is used in an organic photoelectric element a highly heat-resistant organic photoelectric element suitable for high-temperature processes such as vacuum deposition can be manufactured.

The active layer 130 may have a thickness of 1 to 500 nm, preferably 5 to 300 nm. When formed with such thickness, light can be effectively absorbed, and holes and electrons can be efficiently separated and transported, thereby maximizing efficiency. The thickness of the active layer 130 is determined according to the absorption coefficient of the active layer 130 material, and it is preferable that the thickness allows absorption of at least 70%, preferably over 80%, and more preferably over 90% of the light.

Referring to FIG. 2, the organic photoelectric element may further include a hole transport region 210 between the first electrode 110 and the active layer 130, and an electron transport region 220 between the second electrode 120 and the active layer 130.

By forming the hole transport region 210 and the electron transport region 220, the movement of holes and electrons separated in the active layer 130 can be facilitated and accelerated, thereby improving the efficiency of the organic photoelectric element.

The hole transport region 210 may include a hole injection layer (HIL), a hole transport layer (HTL), an electron blocking layer (EBL), and a hole transport auxiliary layer, and may contain at least one hole transport layer.

The hole transport region 210 may comprise organic materials, inorganic materials, or organic-inorganic materials. The organic materials may be organic compounds having hole transport properties, but are not limited to these.

The hole transport region 210 may comprise polymer compounds such as Poly(3,4-ethylenedioxythiophene):poly(styrenesulfonate) (PEDOT:PSS) or polyarylamine, or organic compounds such as N,N,N',N'-tetrakis(4-methoxyphenyl)-benzidine (TPD), 4,4'-bis[N-(1-naphthyl)-N-phenyl-amino]biphenyl (NPB), m-MTDATA, 4,4',4"-tris(N-carbazolyl)-triphenylamine (TCTA), N-([1,1'-biphenyl]-4-yl)-9,9-dimethyl-N-(4-(9-phenyl-9H-carbazol-3-yl)phenyl)-9H-fluoren-2-amine, but is not limited thereto.

For example, the hole transport region 210 may comprise amine compounds, specifically including compounds represented by Formula 40 or Formula 41.

<Formula 40>

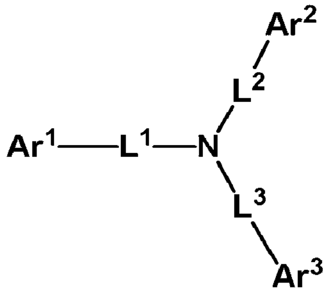

<Formula 41>

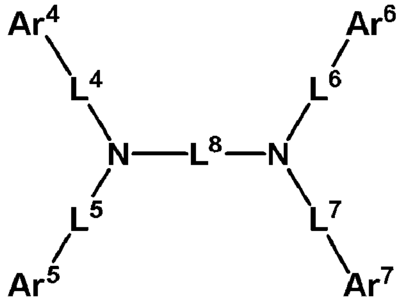

In Formulas 40 and 41, the symbols are defined as set forth below.

$Ar^1$ to $Ar^7$ are each independently selected from the group consisting of a $C_6$-$C_{60}$ aryl group, a fluorenyl group, a $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, and a $C_1$-$C_{20}$ alkyl group.

$L^1$ to $L^8$ are each independently selected from the group consisting of a single bond, a $C_6$-$C_{60}$ arylene group, a fluorenylene group, a $C_3$-$C_{60}$ aliphatic ring group, a fused ring of a $C_3$-$C_{60}$ aliphatic ring and a $C_6$-$C_{60}$ aromatic ring, and a $C_2$-$C_{60}$ heterocyclic group containing at least one heteroatom of O, N, S, Si and P.

The aryl group, the arylene group, the fluorenyl group, the fluorenylene group, the heterocyclic group, the aliphatic ring group, the fused ring, and the alkyl group may be each substituted with one or more substituents selected from the group consisting of deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide unsubstituted or substituted with a $C_1$-$C_{20}$ alkyl group or a $C_6$-$C_{20}$ aryl group, a $C_1$-$C_{20}$ alkylthio group, a $C_1$-$C_{20}$ alkoxy group, a $C_6$-$C_{30}$ aryloxy group, a $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{60}$ heterocyclic group comprising at least one heteroatom selected from the group consisting of O, **N,** S, Si and P, a $C_3$-$C_{30}$ aliphatic ring, and a fused ring of a $C_3$-$C_{30}$ aliphatic ring and a $C_6$-$C_{30}$ aromatic ring.

Formula 40 may be represented by one of Formula 40-1 to Formula 40-3.

<Formula 40-1>

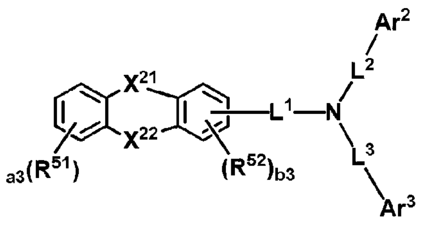

<Formula 40-2>

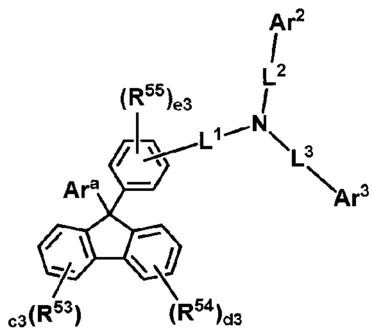

<Formula 40-3>

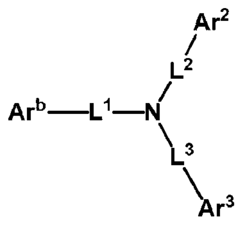

In Formula 40-1 to Formula 40-3, $Ar^2$, $Ar^3$, $L^1$-$L^3$ are the same as defined for Formula 40.

$X^{21}$ and $X^{22}$ are each independently a single bond, O, S, $N(R_{11})$, $C(R_{12})(R_{13})$ or $Si(R_{14})(R_{15})$, and the case where both $X^{21}$ and $X^{22}$ are single bonds is excluded.

$Ar^a$, $R_{11}$ to $R_{15}$, $R^{51}$ to $R^{55}$ are each independently selected from the group consisting of hydrogen, deuterium, halogen, a cyano group, a nitro group, a $C_6$-$C_{60}$ aryl group, a fluorenyl group, $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{20}$ alkyl group, a $C_2$-$C_{20}$ alkenyl group, a $C_2$-$C_{20}$ alkynyl group, a $C_1$-$C_{20}$ alkoxyl group, and a $C_6$-$C_{60}$

aryloxy group, and adjacent groups may be bonded to each other to form a ring.

$Ar^b$ is a $C_3$-$C_{60}$ aliphatic ring group, preferably a $C_3$-$C_{60}$ cycloalkyl group.

a3, c3, d3 and e3 are each integers from 0 to 4, and b3 is an integer from 0 to 3. When these are integers of 2 or greater, each of the plurality of $R^{51}$, each of the plurality of $R^{52}$, each of the plurality of $R^{53}$, each of the plurality of $R^{54}$, each of the plurality of $R^{55}$ may be the same as or different from each other.

Formula 40-1 may be represented by Formula 40-1-1 or Formula 41-1-2.

<Formula 40-1-1>

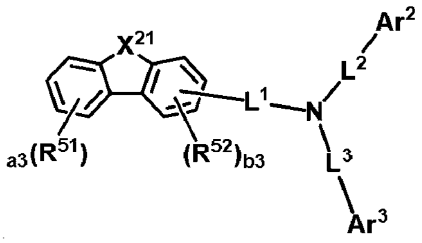

<Formula 40-1-2>

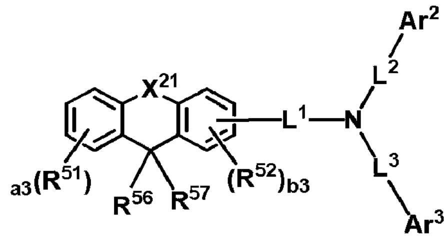

In Formula 40-1-1 and Formula 40-1-2, $Ar^2$, $Ar^3$, $L^1$-$L^3$, $X^{21}$, $R^{51}$, $R^{52}$, a3, b3 are the same as defined for Formula 40-1, $R^{56}$ and $R^{57}$ are defined in the same manner as $R_{12}$ and $R_{13}$.

In Formula 40-3, $Ar^b$ may be selected from the following Formulae, but is not limited thereto.

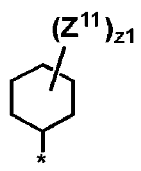
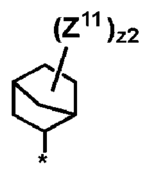
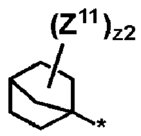
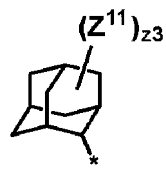
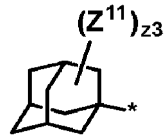

In Formula structures, $Z^{11}$ is selected from the group consisting of hydrogen, deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide unsubstituted or substituted with a $C_1$-$C_{20}$ alkyl group or a $C_6$-$C_{20}$ aryl group, a $C_1$-$C_{20}$ alkylthio group, a $C_1$-$C_{20}$ alkoxy group, a $C_6$-$C_{30}$ aryloxy group, a $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{60}$ heterocyclic group comprising at least one heteroatom selected from the group consisting of O, N, S, Si and P, a $C_3$-$C_{30}$ aliphatic ring, and a fused ring of a $C_3$-$C_{30}$ aliphatic ring and a $C_6$-$C_{30}$ aromatic ring, and adjacent groups may be bonded to each other to form an aliphatic ring. z1 and z2 are each integers from 0 to 11, and z3 is an integer from 0 to 15, and when these are integers of 2 or greater, each of the plurality of $R^{11}$ may be the same as or different from each other.

Formula 41 may be represented by Formula 41-1 or Formula 41-2.

<Formula 41-1>

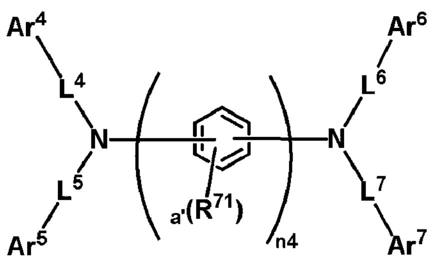

<Formula 41-2>

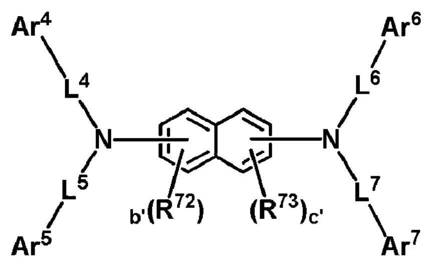

In Formula 41-1 and Formula 41-2, $Ar^4$ to $Ar^7$ and $L^4$ to $L^7$ are the same as defined for Formula 41.

$R^{71}$ to $R^{73}$ are each independently selected from the group consisting of hydrogen, deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide unsubstituted or substituted with a $C_1$-$C_{20}$ alkyl group or a $C_6$-$C_{20}$ aryl group, a $C_1$-$C_{20}$ alkylthio group, a $C_1$-$C_{20}$ alkoxy group, a $C_6$-$C_{30}$ aryloxy group, a $C_6$-$C_{30}$ arylthio group, a $C_1$-$C_{20}$ alkyl group, a

$C_2$-$C_{20}$ alkenyl group, a $C_2$-$C_{20}$ alkynyl group, a $C_6$-$C_{30}$ aryl group, a fluorenyl group, a $C_2$-$C_{60}$ heterocyclic group comprising at least one heteroatom selected from the group consisting of O, N, S, Si and P, a $C_3$-$C_{30}$ aliphatic ring, and a fused ring of a $C_3$-$C_{30}$ aliphatic ring and a $C_6$-$C_{30}$ aromatic ring, and adjacent groups may be bonded to each other to form an aliphatic ring. a' is an integer from 1 to 4, n4, b' and c' are each integers from 1 to 3, and when these are integers of 2 or greater, each of the plurality of $R^{71}$, each of the plurality of $R^{72}$, each of the plurality of $R^{73}$ may be the same as or different from each other.

Formula 41-1 may be represented by Formula 41-1-1 or Formula 41-1-2.

<Formula 42-1-1>

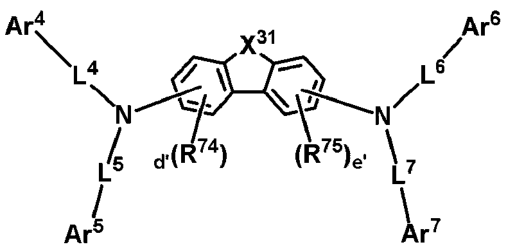

<Formula 42-1-2>

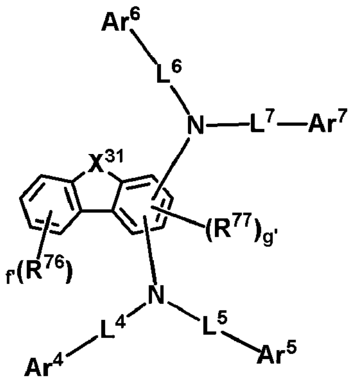

In Formula 41-1-1 and Formula 41-1-2, $Ar^4$ to $Ar^7$, $L^4$ to $L^7$ are the same as defined for Formula 41-1.

$X^{31}$ is O, S, $N(R_{21})$ or $C(R_{22})(R_{22})$.

$R^{74}$ to $R^{77}$, $R_{21}$ to $R_{23}$ are each independently selected from the group consisting of hydrogen, deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide unsubstituted or substituted with a $C_1$-$C_{20}$ alkyl group or a $C_6$-$C_{20}$ aryl group, a $C_1$-$C_{20}$ alkylthio group, a $C_1$-$C_{20}$ alkoxy group, a $C_6$-$C_{30}$ aryloxy group, a $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{60}$ heterocyclic group comprising at least one heteroatom selected from the group consisting of O, N, S, Si and P, a $C_3$-$C_{30}$ aliphatic ring, and a fused ring of a $C_3$-$C_{30}$ aliphatic ring and a $C_6$-$C_{30}$ aromatic ring, and adjacent groups may be bonded to each other to form a ring. d' and e' are each integers from 1 to 3, f' is an integer from 1 to 4, and g' is an integer from 1 to 2, and when these are integers of 2 or greater, each of the plurality of $R^{74}$, each of the plurality of $R^{75}$, each of the plurality of $R^{76}$, each of the plurality of $R^{77}$ may be the same as or different from each other.

Specifically, the compound represented by Formula 40 may be any one of compounds P1-1 to P1-49, P1-89, and P1-90 below, and the compound represented by Formula 41 may be any one of compounds P1-50 to P1-88 below, but is not limited thereto.

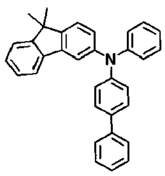

P1-1

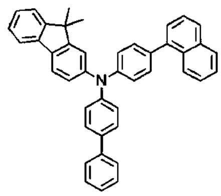

P1-2

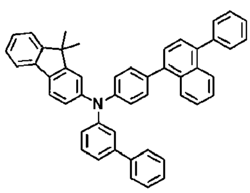

P1-3

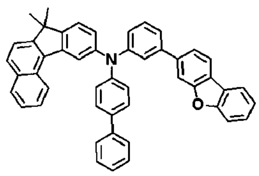

P1-4

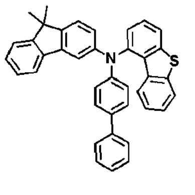

P1-5

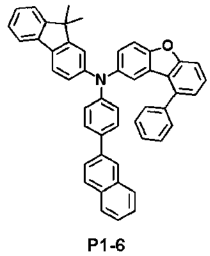

P1-6

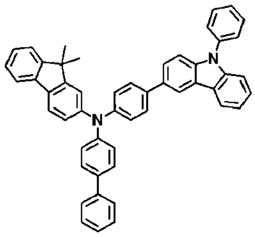

P1-7

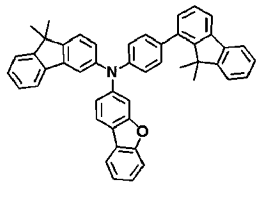

P1-8

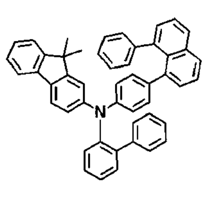
P1-9

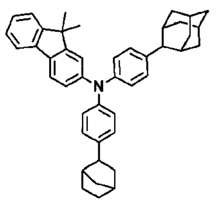
P1-10

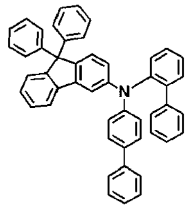
P1-11

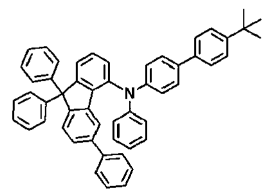
P1-12

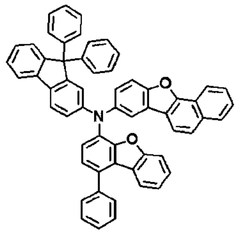
P1-13

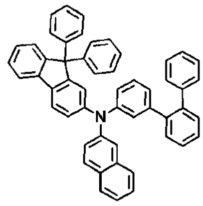
P1-14

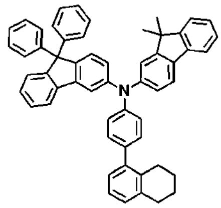
P1-15

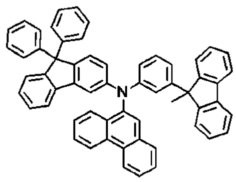
P1-16

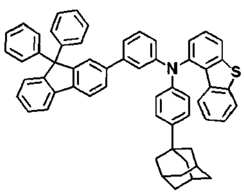
P1-17

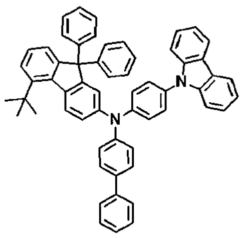
P1-18

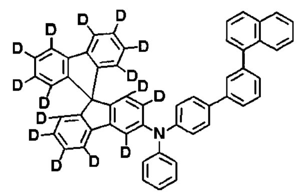
P1-19

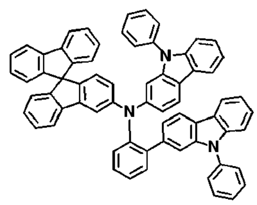
P1-20

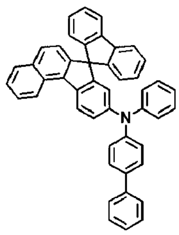
P1-21

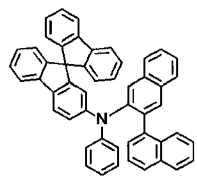
P1-22

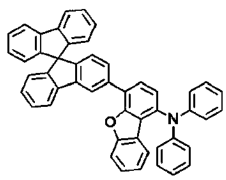
P1-23

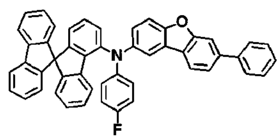
P1-24

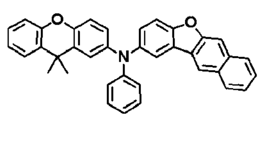
P1-25

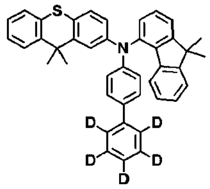
P1-26

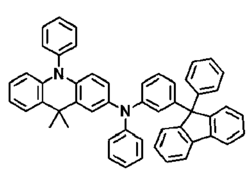
P1-27

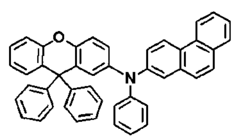
P1-28

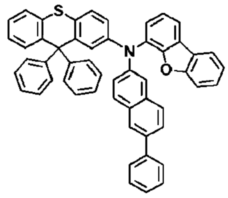
P1-29

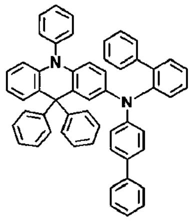
P1-30

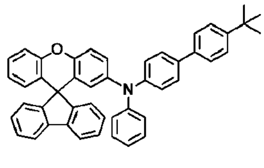
P1-31

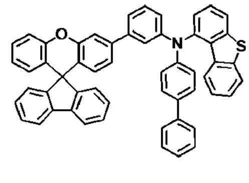
P1-32

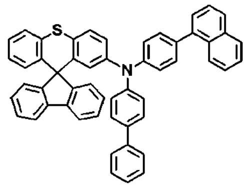

P1-33

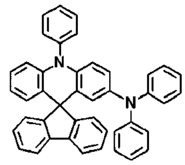

P1-34

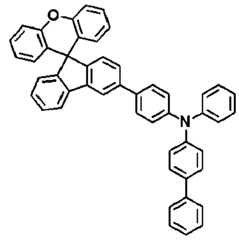

P1-35

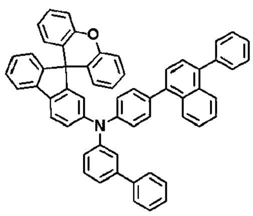

P1-36

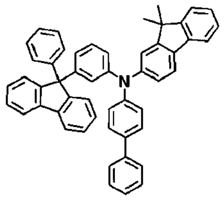

P1-37

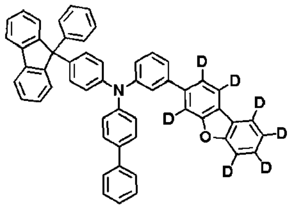

P1-38

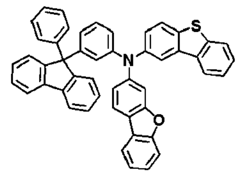

P1-39

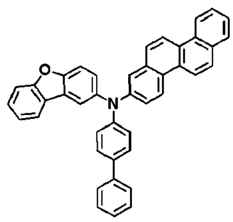

P1-40

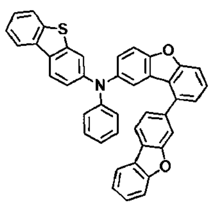

P1-41

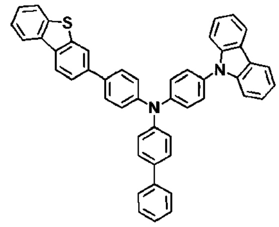

P1-42

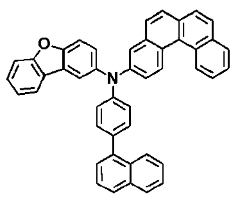

P1-43

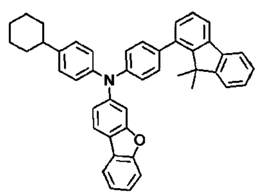

P1-44

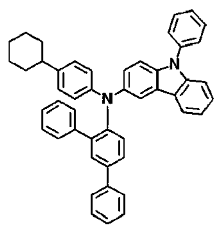

P1-45

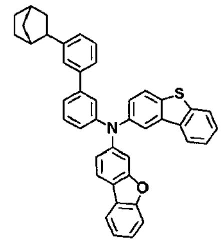

P1-46

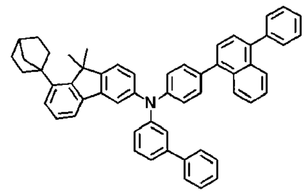

P1-47

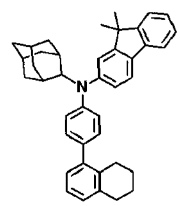

P1-48

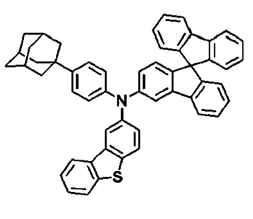

P1-49

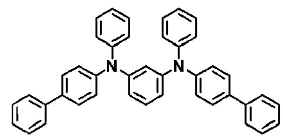

P1-50

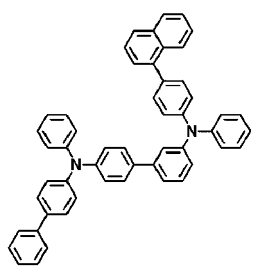

P1-51

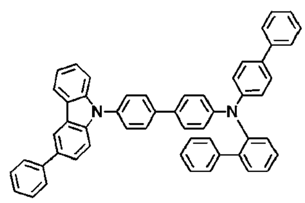

P1-52

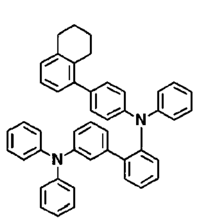

P1-53

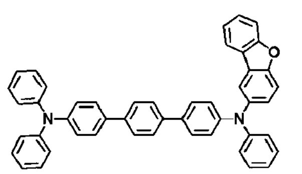

P1-54

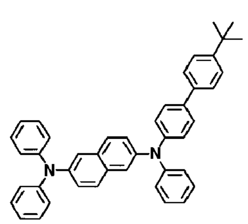

P1-55

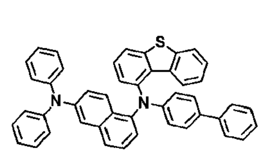

P1-56

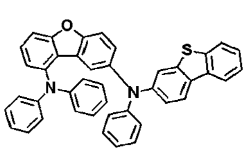
P1-57

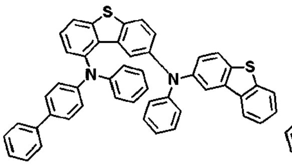
P1-58

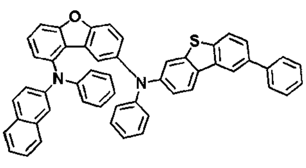
P1-59

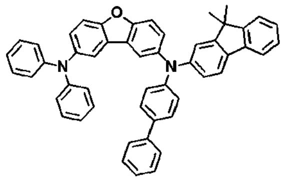
P1-60

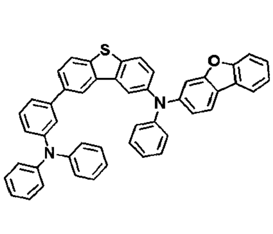
P1-61

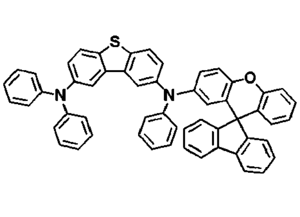
P1-62

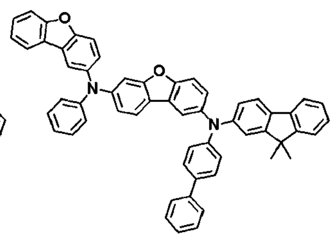
P1-63

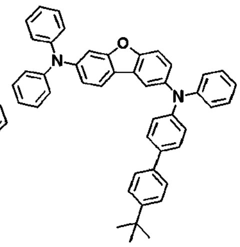
P1-64

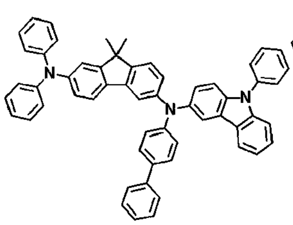
P1-65

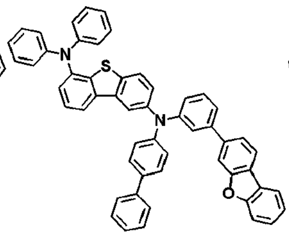
P1-66

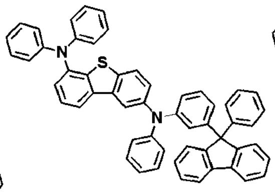
P1-67

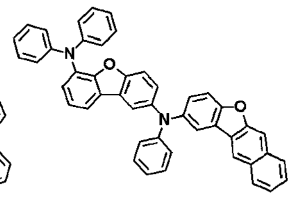
P1-68

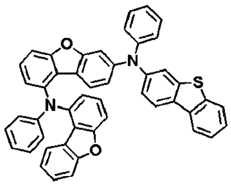
P1-69

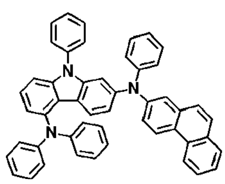
P1-70

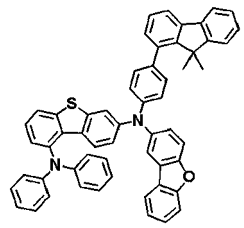
P1-71

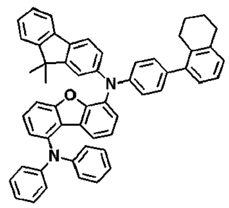
P1-72

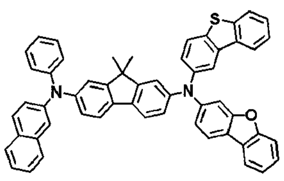
P1-73

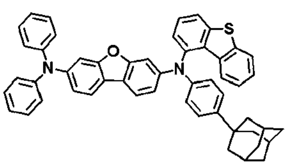
P1-74

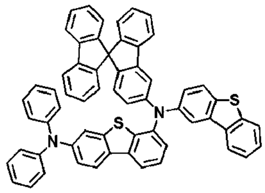
P1-75

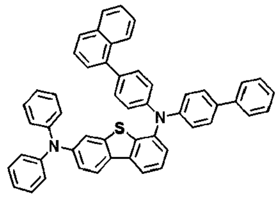
P1-76

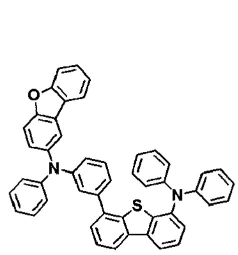
P1-77

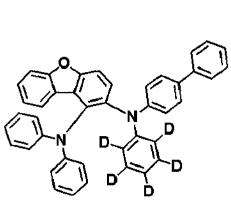
P1-78

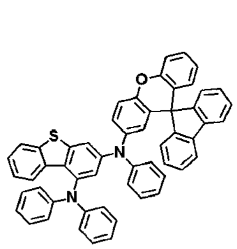
P1-79

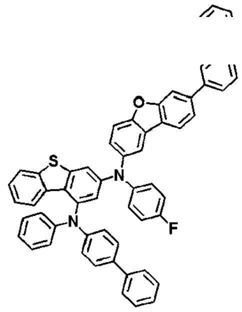
P1-80

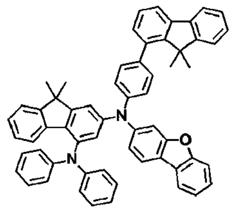

P1-81

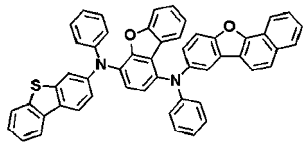

P1-82

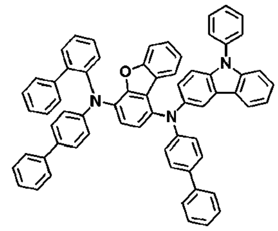

P1-83

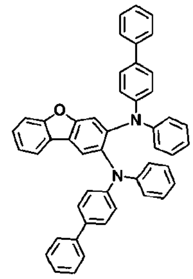

P1-84

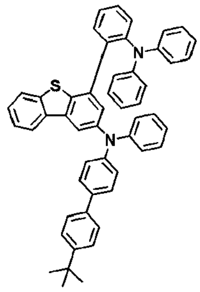

P1-85

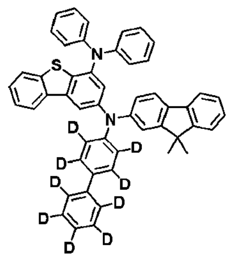

P1-86

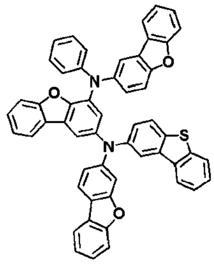

P1-87

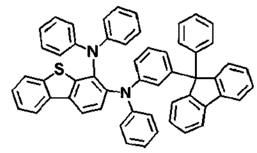

P1-88

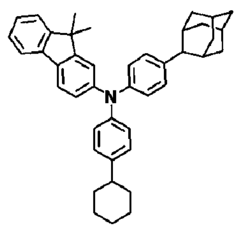

P1-89

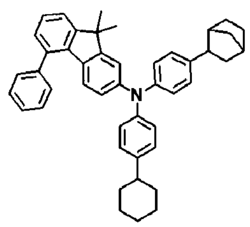

P1-90

The electron transport region 220 may comprise an electron injection layer (EIL), an electron transport layer (ETL), a hole blocking layer (HBL), and an electron transport auxiliary layer, and may include at least one electron transport layer.

The electron transport region 220 may comprise organic materials, inorganic materials, or organic-inorganic hybrid materials. The organic materials may be organic compounds having electron transport properties, and the inorganic materials may comprise molybdenum oxide, tungsten oxide, nickel oxide, lithium halides, but are not limited to these.

The electron transport region 220 may comprise organic compounds and organometallic compounds such as 1,4,5,8-Naphthalene-tetracarboxylic dianhydride (NTCDA), bathocuproine (BCP), LiF, $Alq_3$, $Gaq_3$, $Inq_3$, $Znq_2$, $Zn(BTZ)_2$, $BeBq_2$, but is not limited to these.

For example, the electron transport region 220 may comprise azine compounds, specifically compounds represented by Formula 50 or Formula 51.

<Formula 50>

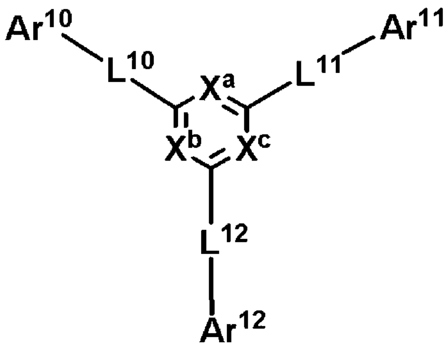

<Formula 51>

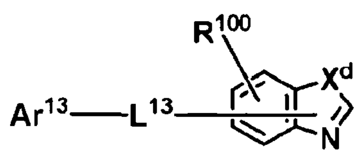

In Formulas 50 and 51, the symbols are defined as set forth below.

$X^a$ to $X^c$ are each independently N or $C(R_{51})$, and at least one of $X^a$ to $X^c$ is N. Therefore, a ring containing $X^a$ to $X^c$ may be pyridine, pyrimidine or triazine.

$X^d$ is O, S or $N(R_{52})$.

$Ar^{10}$ to $Ar^{13}$ are each independently selected from the group consisting of a $C_6$-$C_{60}$ aryl group, a fluorenyl group, $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, and a $C_1$-$C_{30}$ alkyl group.

$L^{10}$ to $L^{13}$ are each independently selected from the group consisting of a single bond, a $C_6$-$C_{60}$ arylene group, a fluorenylene group, a $C_3$-$C_{60}$ aliphatic ring group, a fused ring of a $C_3$-$C_{60}$ aliphatic ring and a $C_6$-$C_{60}$ aromatic ring, and $C_2$-$C_{60}$ heterocyclic group containing at least one heteroatom of O, N, S, Si and P.

$R^{100}$, $R_{51}$ and $R_{52}$ are each independently selected from the group consisting of hydrogen, deuterium, halogen, a $C_6$-$C_{60}$ aryl group, a fluorenyl group, $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{30}$ alkyl group, a $C_2$-$C_{30}$ alkenyl group, a $C_2$-$C_{30}$ alkynyl group, a $C_1$-$C_{30}$ alkoxyl group, and a $C_6$-$C_{30}$ aryloxy group, and adjacent groups may be bonded to each other to form a ring. In Formula 51, only one $R^{100}$ is shown; however, Formula 51 includes forms in which one to four $R^{100}$ groups are substituted.

The aryl group, the arylene group, the fluorenyl group, the fluorenylene group, the heterocyclic group, the aliphatic ring group, the fused ring, the alkyl group, the alkenyl group, the alkynyl group, the alkoxyl group, and the aryloxyl group may be each substituted with one or more substituents selected from the group consisting of deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide unsubstituted or substituted with a $C_1$-$C_{20}$ alkyl group or a $C_6$-$C_{20}$ aryl group, a $C_1$-$C_{20}$ alkylthio group, a $C_1$-$C_{20}$ alkoxy group, a $C_6$-$C_{30}$ aryloxy group, a $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{60}$ heterocyclic group comprising at least one heteroatom selected from the group consisting of O, **N,** S, Si and P, a $C_3$-$C_{30}$ aliphatic ring, and a fused ring of a $C_3$-$C_{30}$ aliphatic ring and a $C_6$-$C_{30}$ aromatic ring.

Formula 50 may be represented by any one of Formula 50-1 to Formula 50-3.

<Formula 50-1>

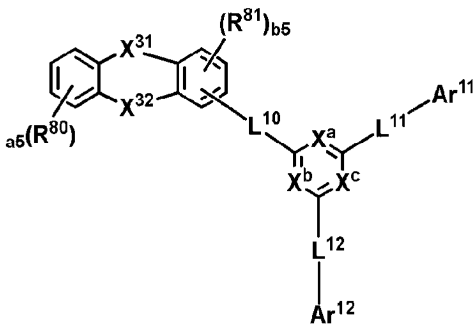

<Formula 50-2>

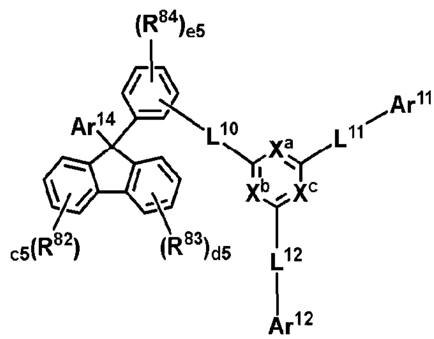

<Formula 50-3>

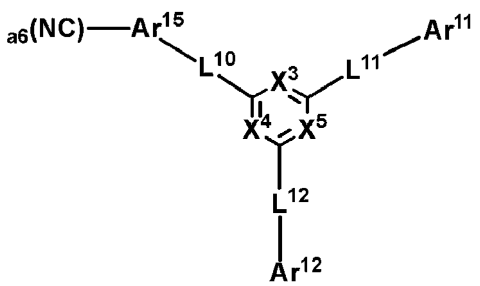

In Formula 50-1 to Formula 50-3, $X^a$ to $X^c$, $Ar^{11}$, $Ar^{12}$, $L^{10}$ to $L^{12}$ are the same as defined for Formula 50.

$X^{31}$ and $X^{32}$ are each independently a single bond, O, S, $N(R_{53})$, $C(_{54})(R_{55})$ or $Si(R_{56})(R_{57})$, and the case where both $X^{31}$ and $X^{32}$ are single bonds is excluded.

$Ar^{14}$, $R^{80}$ to $R^{84}$, $R_{53}$ to $R_{57}$ are each independently selected from the group consisting of hydrogen, deuterium,

halogen, a $C_6$-$C_{60}$ aryl group, a fluorenyl group, $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{30}$ alkyl group, a $C_2$-$C_{30}$ alkenyl group, a $C_2$-$C_{30}$ alkynyl group, a $C_1$-$C_{30}$ alkoxyl group, and a $C_6$-$C_{30}$ aryloxy group, and adjacent groups may be bonded to each other to form a ring.

a5, c5, d5 and e5 are each integers from 1 to 4, and b5 is an integer from 0 to 3.

$Ar^{15}$ is a $C_6$-$C_{60}$ aryl group, and a6 is an integer from 1 or greater.

Formula 50-1 may be reprsented by Formula 50-1-1 or Formula 50-1-2.

<Formula 50-1-1>

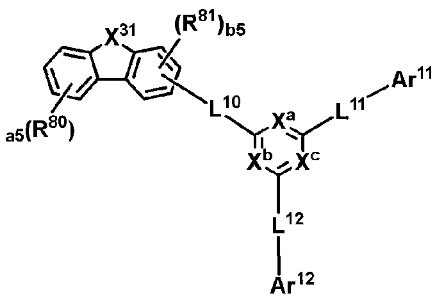

<Formula 50-1-2>

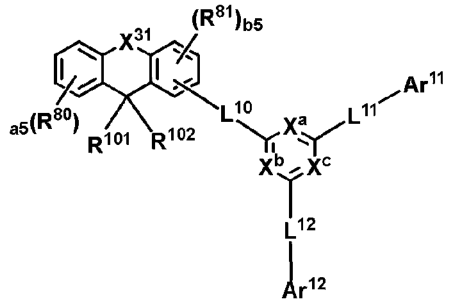

In Formula 50-1-1 and Formula 50-1-2, $X^a$ to $X^c$, $Ar^{11}$, $Ar^{12}$, $L^{10}$ to $L^{12}$, $X^{31}$, $R^{80}$, $R^{81}$, a5, b5 are the same as defined for Formula 50-1, and $R^{101}$ and $R^{102}$ are the same as $R_{54}$ and $R_{55}$.

Specifically, the compound represented by Formula 50 may be any one of compounds P2-1 to P2-48 below, and the compound represented by Formula 51 may be any one of compounds P2-49 to P2-76 below, but is not limited thereto.

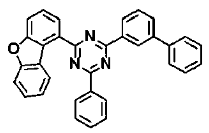

P2-1

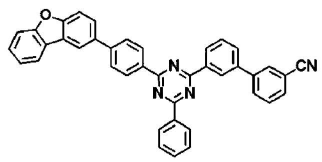

P2-2

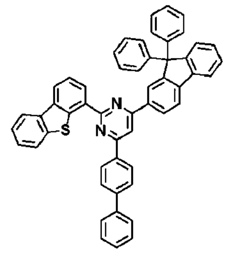

P2-3

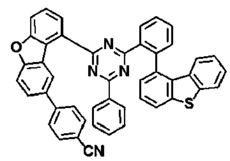

P2-4

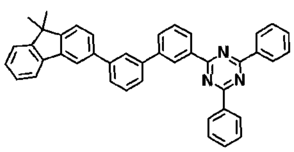

P2-5

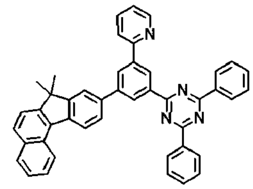

P2-6

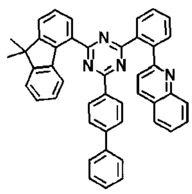

P2-7

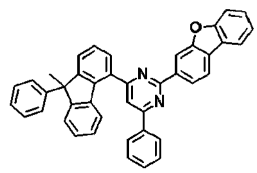

P2-8

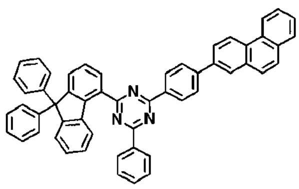

P2-9

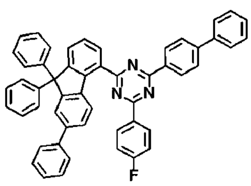

P2-10

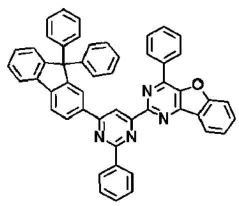

P2-11

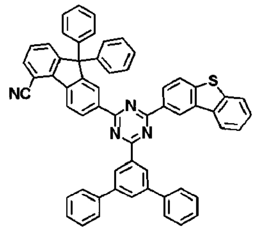

P2-12

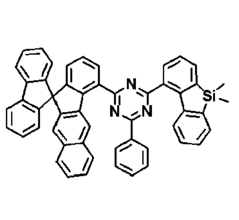

P2-13

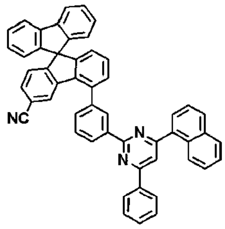

P2-14

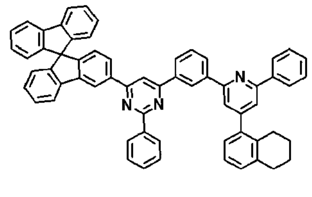

P2-15

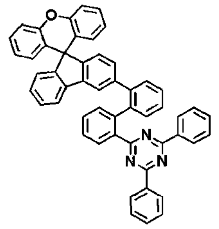

P2-16

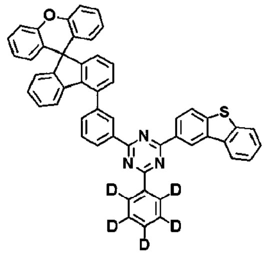

P2-17

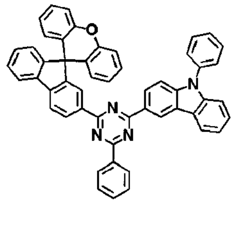

P2-18

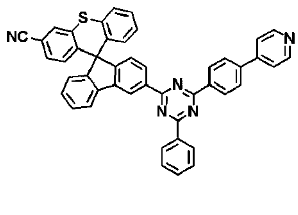

P2-19

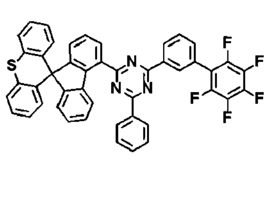

P2-20

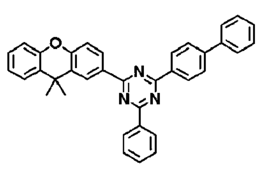

P2-21

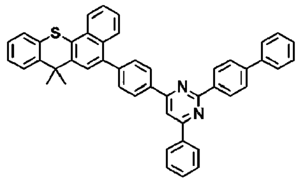

P2-22

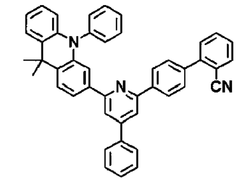

P2-23

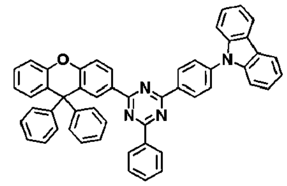

P2-24

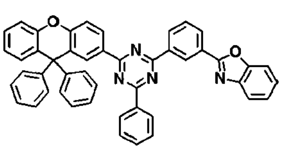

P2-25

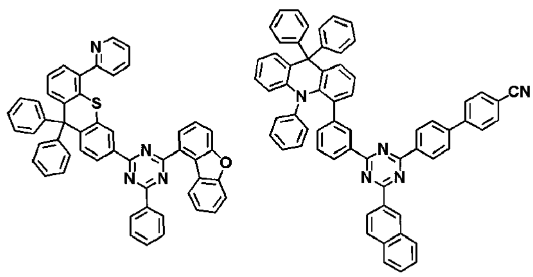

P2-26

P2-27

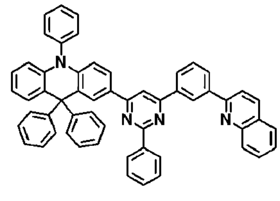

P2-28

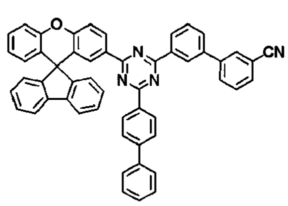

P2-29

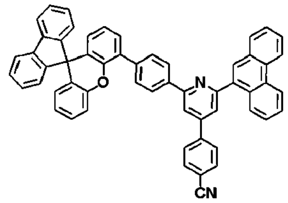

P2-30

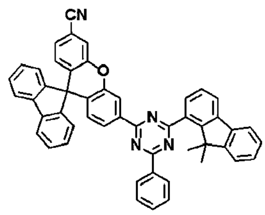

P2-31

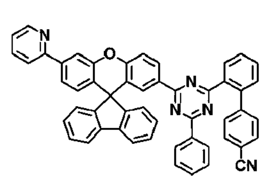

P2-32

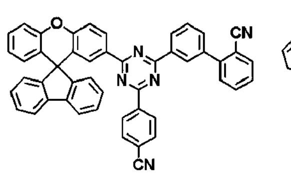

P2-33

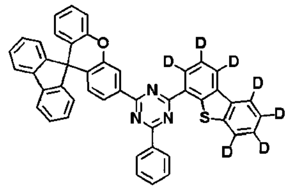

P2-34

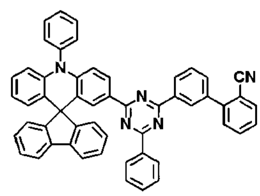

P2-35

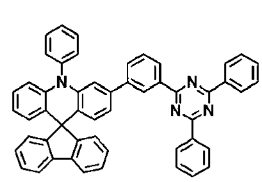

P2-36

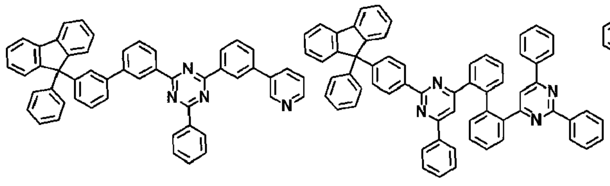

P2-37 P2-38

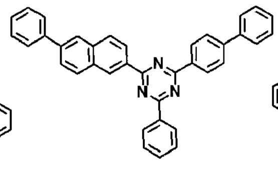

P2-39

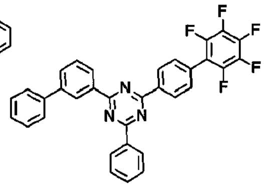

P2-40

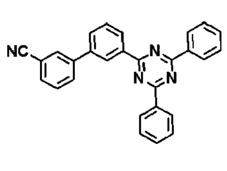

P2-41

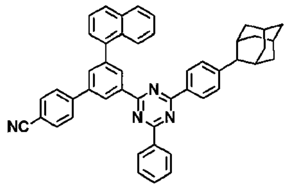

P2-42

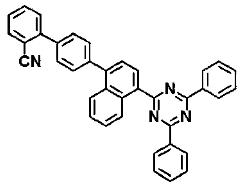

P2-43

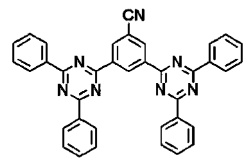

P2-44

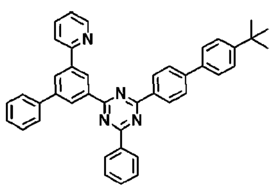

P2-45

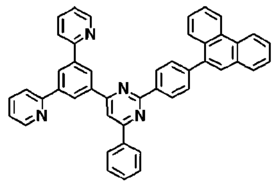

P2-46

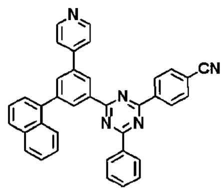

P2-47

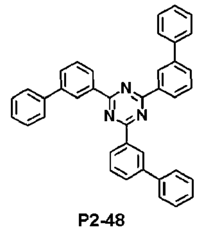

P2-48

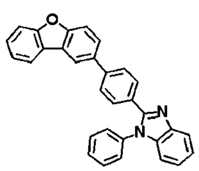

P2-49

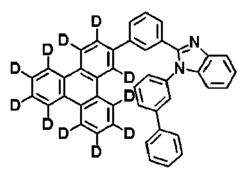

P2-50

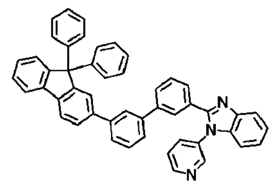

P2-51

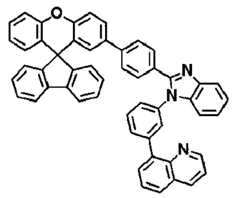

P2-52

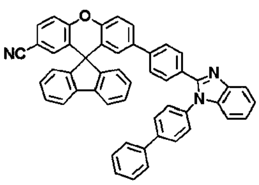

P2-53

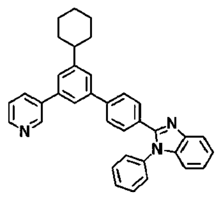

P2-54

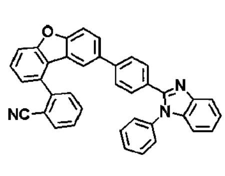

P2-55

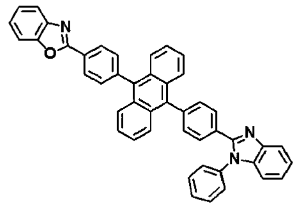

P2-56

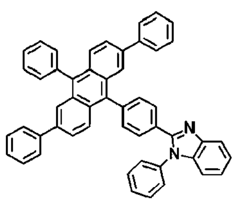

P2-57

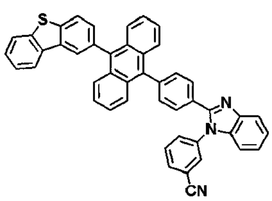

P2-58

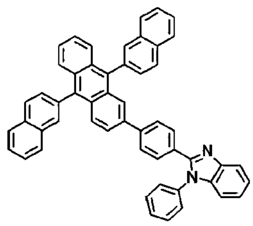

P2-59

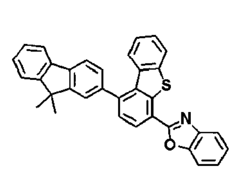

P2-60

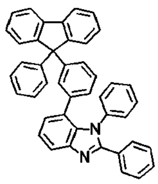

P2-61

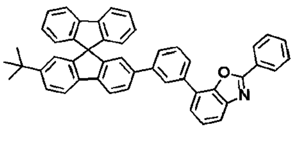

P2-62

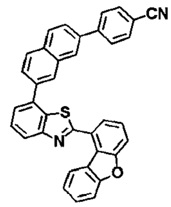

P2-63

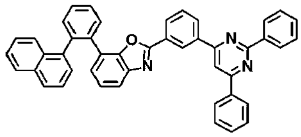

P2-64

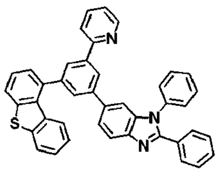

P2-65

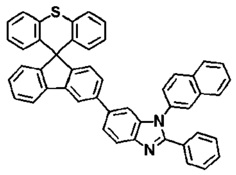

P2-66

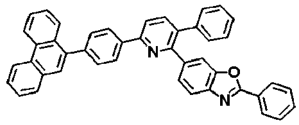

P2-67

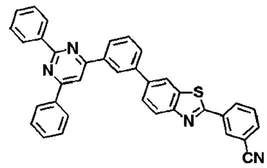

P2-68

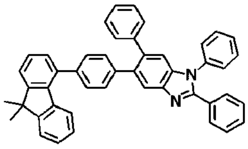

P2-69

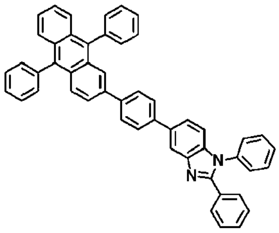

P2-70

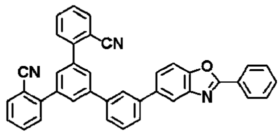

P2-71

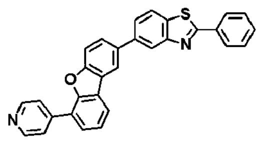

P2-72

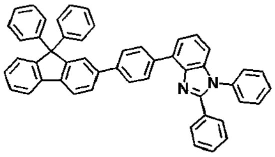

P2-73

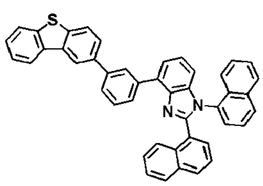

P2-74

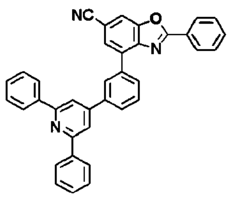

P2-75

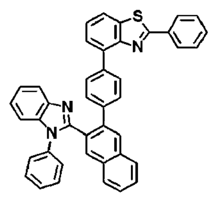

P2-76

An organic photoelectric element according to one embodiment of the present invention can be manufactured using various deposition methods. The organic photoelectric element can be fabricated by deposition techniques such as PVD (Physical Vapor Deposition) or CVD (Chemical Vapor Deposition). For example, a metal, conductive metal oxide, or their alloys can be deposited on a substrate to form the first electrode 110, and then an organic layer including the active layer 130 is formed thereon, followed by deposition of a material that can be used as the second electrode 120 on the organic layer, thereby completing the device.

The active layer 130 can also be fabricated using various polymer materials by solution processes or solvent processes instead of deposition methods. Examples include spin coating, nozzle printing, inkjet printing, slot coating, dip coating, roll-to-roll processing, doctor blading, screen printing, or thermal evaporation methods. These methods allow the active layer to be manufactured with fewer layers.

The organic photoelectric element according to one embodiment of the present invention can be applied to organic solar cells, image sensors, photodetectors, optical sensors, and organic light-emitting devices. In particular, the organic photoelectric element of the present invention can be applied to image sensors.

The image sensor comprising the organic photoelectric element may be an image sensor that detects light of 450 nm or more, preferably light with a wavelength of 450 to 650 nm. Therefore, it can selectively detect light in the green wavelength region and may replace the color filter of the green pixel.

Another embodiment of the present invention may comprise an electronic device comprising the above-described image sensor of the present invention. In this case, the electronic device may be a current or future wired or wireless communication terminal, and may include all electronic devices such as mobile communication terminals like mobile phones, navigation systems, gaming devices, various TVs, various computers, digital cameras, electronic endoscopes, and the like.

Hereinafter, the present invention will be described in detail with reference to examples. The following examples are provided for illustrative purposes only and are not intended to limit the scope of the present invention.

[Synthesis example]

The compound (final products) represented by Formula 1 of the present invention may be prepared by a method such as Reaction Scheme 1, but is not limited thereto.

<Reaction Scheme 1>

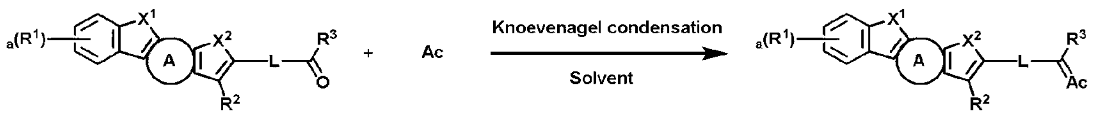


**Sub A** **Sub B** **Final product**

Synthesis example of Sub A

Sub A in Reaction Scheme 1 can be synthesized via the reaction pathway shown in Reaction Scheme 2, but is not limited thereto.

<Reaction Scheme 2>

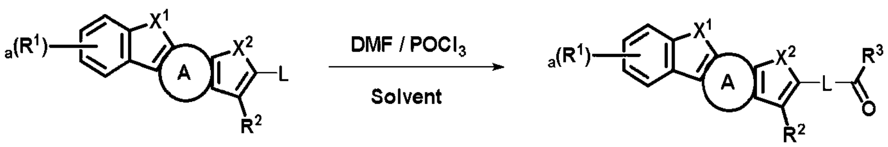


**Sub A**

1. Synthesis example of Sub A-12

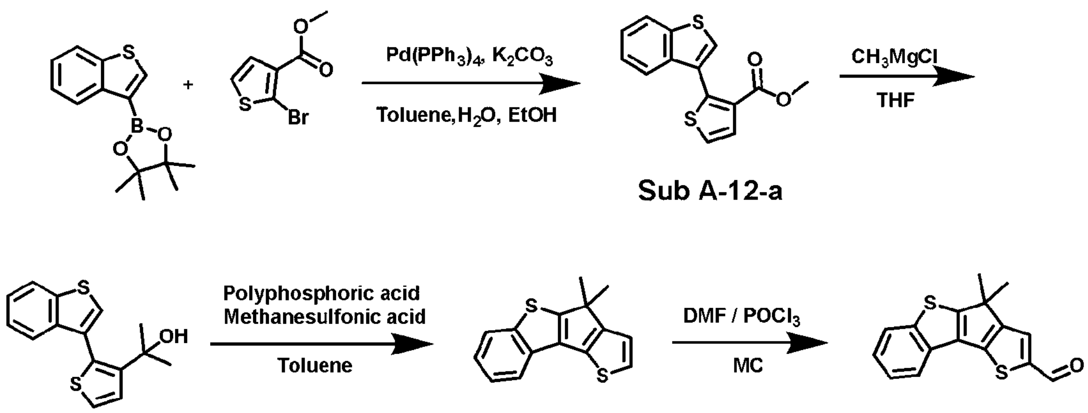


**Sub A-12-a**

**Sub A-12-b** **Sub A-12-c** **Sub A-12**

(1) Synthesis of Sub A-12-a

Methyl 2-bromothiophene-3-carboxylate (42.49 g, 192.19 mmol), $K_2CO_3$ (79.69 g, 576.57 mmol), $Pd(PPh_3)_4$ (6.66 g, 5.77 mmol), Toluene (480 mL), $H_2O$ (192mL) and EtOH (48mL) were added to 2-(benzo[b]thiophen-3-yl)-4,4,5,5-tetramethyl-1,3,2-dioxaborolane (50.0 g, 192.19 mmol), and the reaction was carried out. Upon completion of the reaction, the reaction product was extracted with methyl chloride, and the organic layer was dried over $MgSO_4$ and concentrated.

The concentrate was then separated by silica gel column and recrystallized to obtain the product (40.60 g, yield: 77%).

(2) Synthesis of Sub A-12-b

Sub A-12-a (40 g, 145.80 mmol) was dissolved in THF (729 mL) and stirred. 3 M methylmagnesium chloride solution (194.40 mL) was slowly added dropwise at 5 °C, and the mixture was stirred for 1 hour. After completion of the reaction, the reaction mixture was poured into ice water and extracted with methyl chloride. The organic layer was dried over $MgSO_4$ and concentrated. The concentrate was separated by silica gel column and recrystallized to obtain the product (36.01 g, yield: 90%).

(3) Synthesis of Sub A-12-c

Sub A-12-b (35 g, 127.55 mmol) in toluene (425 mL) was slowly added dropwise to a suspension of polyphosphoric acid (144.84 g, 1020.41 mmol) and methanesulfonic acid (66.26 mL, 1020.41 mmol), and the mixture was stirred for 24 hours. After completion of the reaction, the reaction mixture was poured into ice water and neutralized with 10% aqueous NaOH solution, and then extracted with methyl chloride. The organic layer was dried over $MgSO_4$ and concentrated. The concentrate was separated by silica gel column and recrystallized to obtain the product (20.27 g, yield: 62%).

(4) Synthesis of Sub A-12

Phosphoryl chloride (9.48 mL, 101.41 mmol) was added dropwise to DMF (30.33 mL, 390.05 mmol) at -15 °C, and the mixture was stirred at room temperature for 2 hours. Sub A-12-c (20 g, 78.01 mmol) was dissolved in methyl chloride (780 mL), and the resulting solution was slowly added dropwise to the above mixture, followed by stirring at room temperature for 24 hours. After completion of the reaction, the solvent was removed under reduced pressure at low temperature, and 10% aqueous NaOH solution was added until the pH reached 14. The mixture was then stirred at room temperature for 2 hours and extracted with methyl chloride. The organic layer was dried over $MgSO_4$ and concentrated. The concentrate was separated by silica gel column and recrystallized to obtain the product (21.08 g, yield: 95%).

2. Synthesis example of Sub A-17

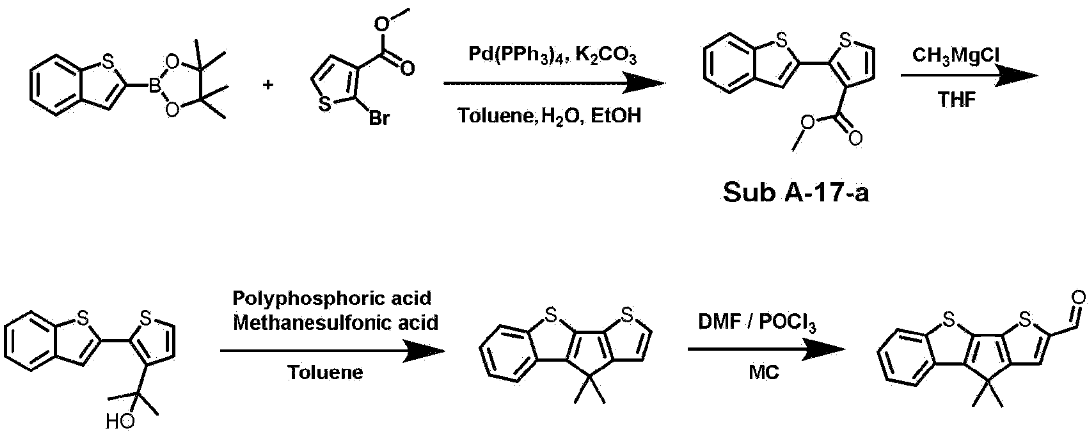


Sub A-17-a

Sub A-17-b Sub A-17-c Sub A-17

(1) Synthesis of Sub A-17-a

Methyl 2-bromothiophene-3-carboxylate (42.49 g, 192.19 mmol), $K_2CO_3$ (79.69 g, 576.57 mmol), $Pd(PPh_3)_4$ (6.66 g, 5.77 mmol), Toluene (480 mL), $H_2O$ (192mL) and EtOH (48mL) were added to 2-(benzo[b]thiophen-2-yl)-4,4,5,5-tetramethyl-1,3,2-dioxaborolane (50.0 g, 192.19 mmol), and the synthesis was carried out in the same manner as in synthesis example of Sub A-12-a to obtain the product (41.65 g, yield: 79%).

(2) Synthesis of Sub A-17-b

Sub A-17-a (40 g, 145.80 mmol) was dissolved in THF (729 mL) and stirred, and the synthesis was carried out in the same manner as in synthesis example of Sub A-12-b to obtain the product (36.81 g, yield: 92%).

(3) Synthesis of Sub A-17-c

Sub A-17-b (35 g, 127.55 mmol) in toluene (425 mL) was slowly added dropwise to a suspension of polyphosphoric acid (144.84 g, 1020.41 mmol) and methanesulfonic acid (66.26 mL, 1020.41 mmol), and the synthesis was carried out in the same manner as in synthesis example of Sub A-12-c to obtain the product (20.93 g, yield: 64%).

(4) Synthesis of Sub A-17

Phosphoryl chloride (9.48 mL, 101.41 mmol) was added dropwise to DMF (30.33 mL, 390.05 mmol) at -15 °C, and the mixture was stirred at room temperature for 2 hours. Sub A-17-c (20 g, 78.01 mmol) was dissolved in methyl chloride (780 mL), and the synthesis was carried out in the same manner as in synthesis example of Sub A-12 to obtain the product (20.85 g, yield: 94%).

3. Synthesis example of Sub A-38

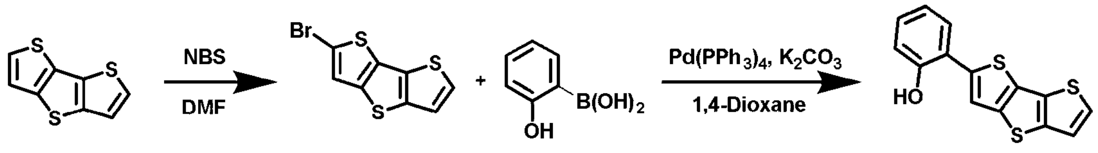


Sub A-38-a Sub A-38-b

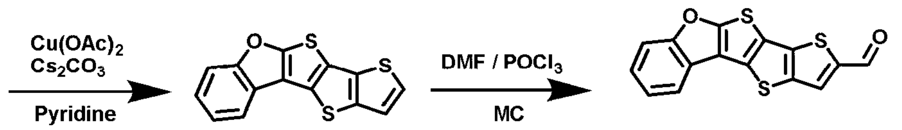


Sub A-38-c Sub A-38

(1) Synthesis of Sub A-38-a

Dithieno[3,2-b:2',3'-d]thiophene (100 g, 509.42 mmol) was dissolved in DMF, and N-bromosuccinimide (86.14 g, 483.95 mmol) was added dropwise at -10 °C. The mixture was then stirred at room temperature for 6 hours. After completion of the reaction, water was added, and the mixture was extracted with methyl chloride. The organic layer was dried over $MgSO_4$ and concentrated. The resulting concentrate was recrystallized from toluene to obtain the product(109.35 g, yield: 78%).

(2) Synthesis of Sub A-38-b

1,4-Dioxane (726 mL) and $H_2O$ (242 mL) were added to a mixture of Sub A-38-a (50 g, 181.69 mmol), 2-Hydroxyphenylboronic acid (37.59 g, 272.53 mmol), $Pd(PPh_3)_4$ (10.50 g, 9.08 mmol) and $K_2CO_3$ (50.22 g, 363.37 mmol) and stirred at 90 °C. Upon completion of the reaction, the reaction product was extracted with methyl chloride, and the organic layer was dried over $MgSO_4$ and concentrated. The concentrate was then separated by silica gel column and recrystallized to obtain the product (43.49 g, yield: 83%).

(3) Synthesis of Sub A-38-c

Sub A-38-b (30.0 g, 104.02 mmol), $Cu(OAc)_2$ (56.68 g, 312.07 mmol), and $Cs_2CO_3$ (33.89 g, 104.02 mmol) were placed in a round-bottom flask, pyridine was added, and the mixture was stirred at 110 °C. Upon completion of the reaction,

the reaction product was extracted with methyl chloride, and the organic layer was dried over $MgSO_4$ and concentrated. The concentrate was then separated by silica gel column and recrystallized to obtain the product (18.77 g, yield: 63%).

(4) Synthesis of Sub A-38

Phosphoryl chloride (7.64 mL, 81.71 mmol) was added dropwise to DMF (24.44 mL, 314.27 mmol) at -15 °C, and the mixture was stirred at room temperature for 2 hours. Sub A-38-c (18 g, 62.85 mmol) was dissolved in methyl chloride (628 mL), and the synthesis was carried out in the same manner as in synthesis example of Sub A-12 to obtain the product (18.18 g, yield: 92%).

4. Synthesis example of Sub A-58

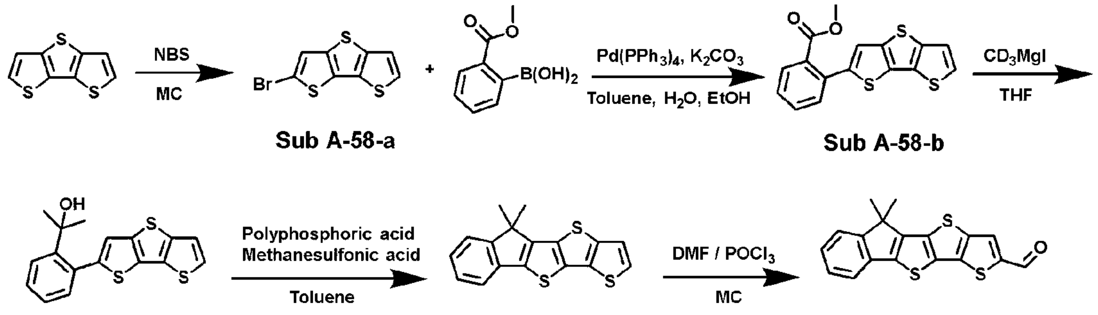


Sub A-58-a

Sub A-58-b

Sub A-58-c

Sub A-58-d

Sub A-58

(1) Synthesis of Sub A-58-a

Dithieno[3,2-b:2',3'-d]thiophene (100 g, 509.42 mmol) was dissolved in DMF, and N-bromosuccinimide (86.14 g, 483.95 mmol) was added dropwise at -10 °C. The mixture was then stirred at room temperature for 6 hours. After completion of the reaction, water was added, and the mixture was extracted with methyl chloride. The organic layer was dried over $MgSO_4$ and concentrated. The resulting concentrate was recrystallized from toluene to obtain the product(109.35 g, yield: 78%).

(2) Synthesis of Sub A-58-b

Toluene (454 mL), $H_2O$ (181mL) and EtOH (45mL) were added to a mixture of Sub A-58-a (50.0 g, 181.69 mmol), (2-(methoxycarbonyl)phenyl)boronic acid (32.70 g, 181.69 mmol), $K_2CO_3$ (75.33 g, 545.06 mmol) and $Pd(PPh_3)_4$ (6.30 g, 5.45 mmol), and the reaction was carried out. Upon completion of the reaction, the reaction product was extracted with methyl chloride, and the organic layer was dried over $MgSO_4$ and concentrated. The concentrate was then separated by silica gel column and recrystallized to obtain the product (51.03 g, yield: 85%).

(3) Synthesis of Sub A-58-c

Sub A-58-b (50 g, 151.32 mmol) was dissolved in THF (756 mL) and stirred. 3 M methylmagnesium chloride solution (201.76 mL) was added dropwise at 5 °C, and the mixture was stirred for 1 hour. After completion of the reaction, the reaction mixture was poured into ice water and extracted with methyl chloride. The organic layer was dried over $MgSO_4$ and concentrated. The concentrate was then separated by silica gel column and recrystallized to obtain the product (46.51 g, yield: 93%).

(4) Synthesis of Sub A-58-d

Sub A-58-c (46 g, 139.19 mmol) in toluene (463 mL) was slowly added dropwise to a suspension of polyphosphoric acid (158.05 g, 1113.53 mmol) and methanesulfonic acid (72.31 mL, 1113.53 mmol), and the synthesis was carried out in the same manner as in synthesis example of Sub A-12-c to obtain the product (25.23 g, yield: 58%).

(5) Synthesis of Sub A-58

Phosphoryl chloride (9.72 mL, 104.01 mmol) was added dropwise to DMF (31.11 mL, 400.05 mmol) at -15 °C, and the mixture was stirred at room temperature for 2 hours. Sub A-58-d (25.0 g, 80.01 mmol) was dissolved in methyl chloride (800 mL), and the synthesis was carried out in the same manner as in synthesis example of Sub A-12 to obtain the product (23.90 g, yield: 95%).

5. Synthesis example of Sub A-72

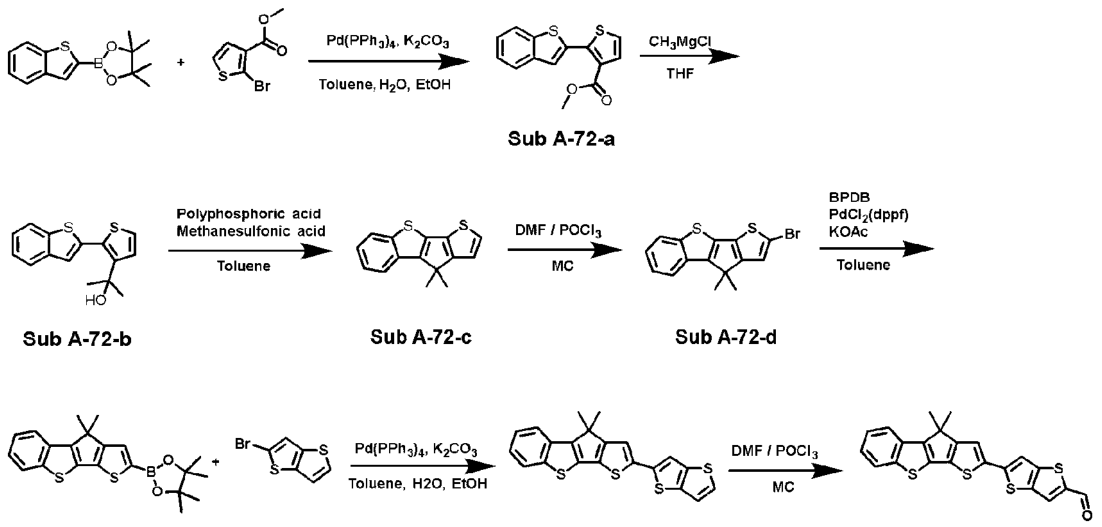


Sub A-72-a

Sub A-72-b

Sub A-72-c

Sub A-72-d

Sub A-72-e

Sub A-72-f

Sub A-72

(1) Synthesis of Sub A-72-a

Methyl 2-bromothiophene-3-carboxylate (42.49 g, 192.19 mmol), $K_2CO_3$ (79.69 g, 576.57 mmol), $Pd(PPh_3)_4$ (6.66 g, 5.77 mmol), Toluene (480 mL), $H_2O$ (192mL) and EtOH (48mL) were added to 2-(benzo[b]thiophen-2-yl)-4,4,5,5-tetramethyl-1,3,2-dioxaborolane (50.0 g, 192.19 mmol), and the synthesis was carried out in the same manner as in synthesis example of Sub A-12-a to obtain the product (41.65 g, yield: 79%).

(2) Synthesis of Sub A-72-b

Sub A-72-a (40 g, 145.80 mmol) was dissolved in THF (729 mL) and stirred, and then the synthesis was carried out in the same manner as in synthesis example of Sub A-12-b to obtain the product (36.81 g, yield: 92%).

(3) Synthesis of Sub A-72-c

Sub A-72-b (35 g, 127.55 mmol) in toluene (425 mL) was slowly added dropwise to a suspension of polyphosphoric acid (144.84 g, 1020.41 mmol) and methanesulfonic acid (66.26 mL, 1020.41 mmol), and the synthesis was carried out in the same manner as in synthesis example of Sub A-12-c to obtain the product (20.93 g, yield: 64%).

(4) Synthesis of Sub A-72-d

Sub A-72-c (30 g, 117.01 mmol) was dissolve in DMF, and N-bromosuccinimide (20.83 g, 117.01 mmol) was added dropwise at -10 °C. The mixture was then stirred at room temperature for 6 hours. After completion of the reaction, water was added, and the mixture was extracted with methyl chloride. The organic layer was dried over $MgSO_4$ and concentrated. The resulting concentrate was recrystallized from toluene to obtain the product (33.35 g, yield: 85%).

(5) Synthesis of Sub A-72-e

A mixture of Sub A-72-d (33.0 g, 98.43 mmol), Bis(pinacolato)diboron (29.99 g, 118.11 mmol), KOAc (28.98 g,

295.28 mmol), Pd(dppf)$Cl_2$ (4.02 g, 4.92 mmol) and Toluene (250 mL) was stirred at 100 °C. Upon completion of the reaction, the reaction product was extracted with methyl chloride, and the organic layer was dried over $MgSO_4$ and concentrated. The concentrate was then separated by silica gel column and recrystallized to obtain the product (30.48 g, yield: 81%).

(6) Synthesis of Sub A-72-f

$K_2CO_3$ (32.53 g, 235.39 mmol), $Pd(PPh_3)_4$ (2.72 g, 2.35 mmol), Toluene (196 mL), $H_2O$ (78 mL) and EtOH (19 mL) were added to a mixture of Sub A-72-e (30.0 g, 78.46 mmol) and 2-bromothieno[3,2-b]thiophene (17.19 g, 78.46 mmol), and stirred at 100 °C. Upon completion of the reaction, the reaction product was extracted with methyl chloride, and the organic layer was dried over $MgSO_4$ and concentrated. The concentrate was then separated by silica gel column and recrystallized to obtain the product (26.63 g, yield: 86%).

(7) Synthesis of Sub A-72

Phosphoryl chloride (8.01 mL, 85.66 mmol) was added dropwise to DMF (25.62 mL, 329.46 mmol) at -15 °C, and the mixture was stirred at room temperature for 2 hours. Sub A-72-f (26.0 g, 65.89 mmol) was dissolved in methyl chloride (658 mL), and the synthesis was carried out in the same manner as in synthesis example of Sub A-12 to obtain the product (25.90 g, yield: 93%).

The compound belonging to Sub A may comprise compounds as described below, but is not limited thereto. Table 1 shows the FD-MS (Field Desorption-Mass Spectrometry) values of the compounds.

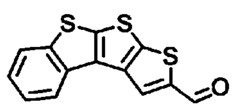

**Sub A-1**

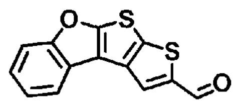

**Sub A-2**

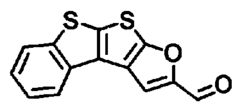

**Sub A-3**

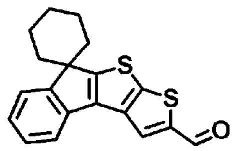

**Sub A-4**

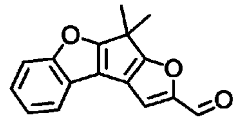

**Sub A-5**

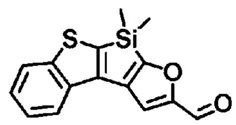

**Sub A-6**

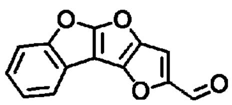

**Sub A-7**

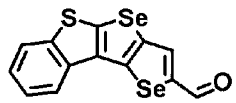

**Sub A-8**

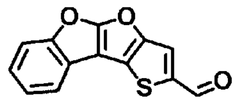

**Sub A-9**

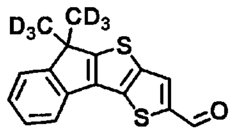

**Sub A-10**

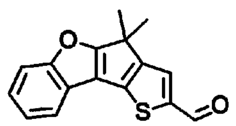

**Sub A-11**

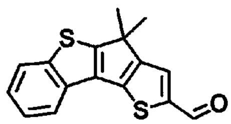

**Sub A-12**

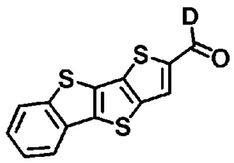

Sub A-13

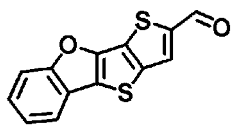

Sub A-14

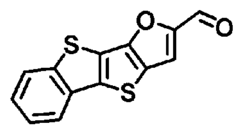

Sub A-15

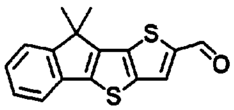

Sub A-16

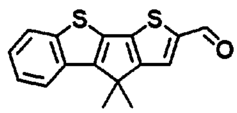

Sub A-17

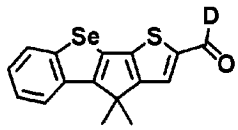

Sub A-18

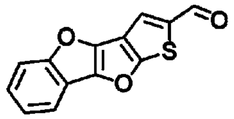

Sub A-19

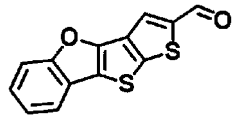

Sub A-20

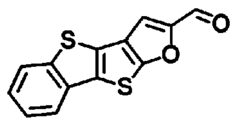

Sub A-21

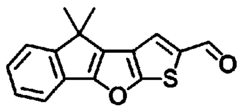

Sub A-22

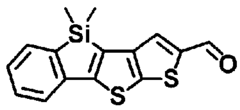

Sub A-23

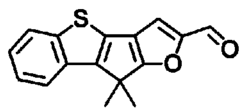

Sub A-24

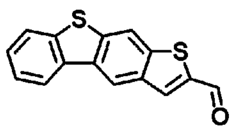

Sub A-25

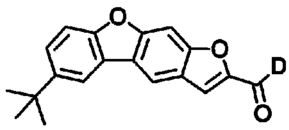

Sub A-26

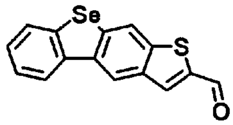

Sub A-27

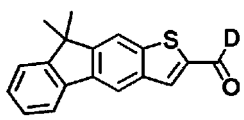

Sub A-28

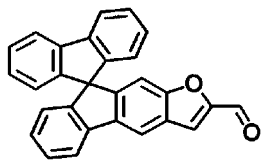

Sub A-29

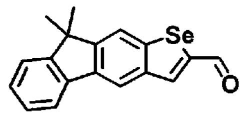

Sub A-30

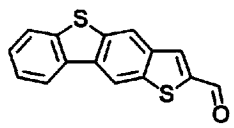

Sub A-31

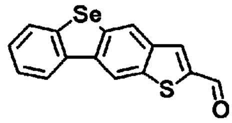

Sub A-32

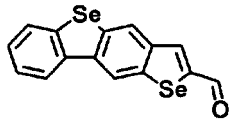

Sub A-33

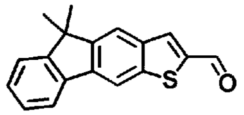

Sub A-34

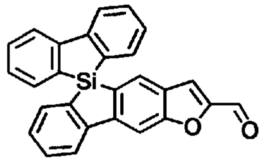

Sub A-35

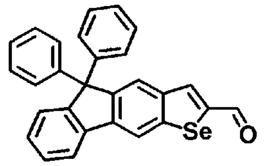

Sub A-36

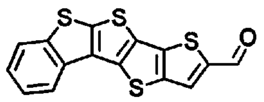

Sub A-37

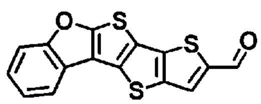

Sub A-38

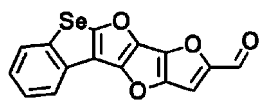

Sub A-39

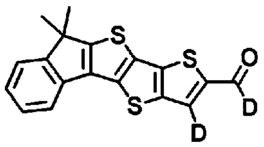

Sub A-40

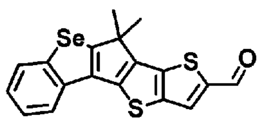

Sub A-41

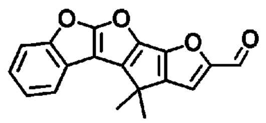

Sub A-42

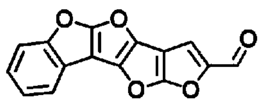

Sub A-43

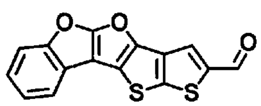

Sub A-44

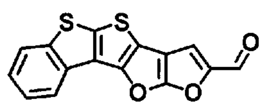

Sub A-45

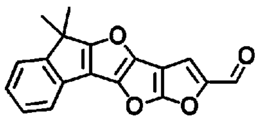

Sub A-46

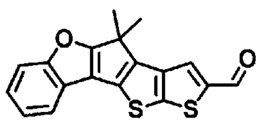

Sub A-47

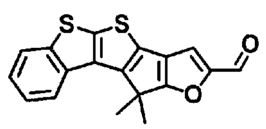

Sub A-48

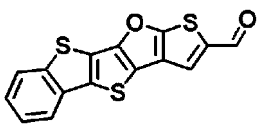

Sub A-49

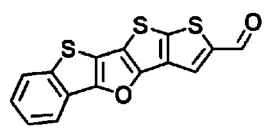

Sub A-50

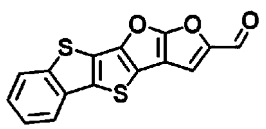

Sub A-51

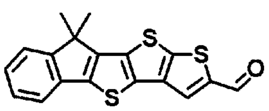

Sub A-52

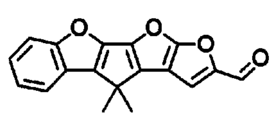

Sub A-53

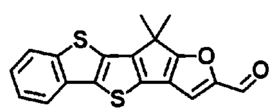

Sub A-54

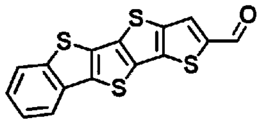

Sub A-55

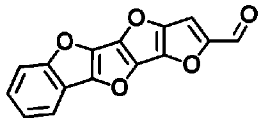

Sub A-56

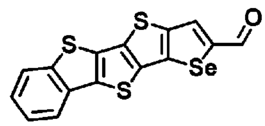

Sub A-57

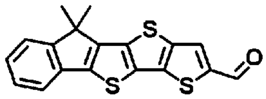

Sub A-58

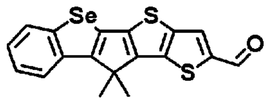

Sub A-59

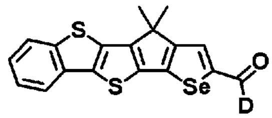

Sub A-60

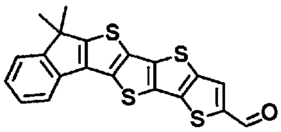

Sub A-61

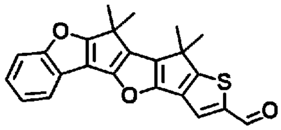

Sub A-62

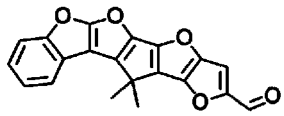

Sub A-63

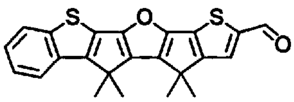

Sub A-64

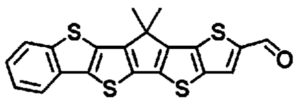

Sub A-65

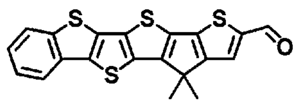

Sub A-66

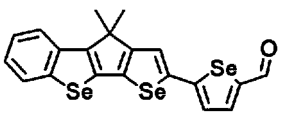

Sub A-67

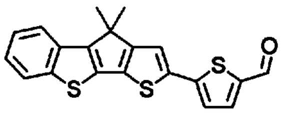

Sub A-68

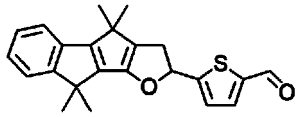

Sub A-69

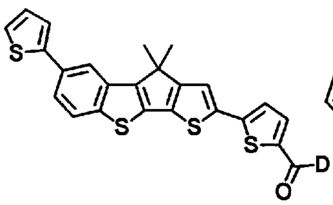

Sub A-70

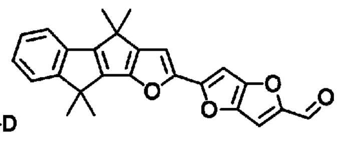

Sub A-71

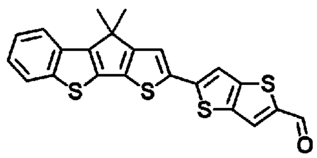

Sub A-72

[Table 1]

| Compound | FD-MS | Compound | FD-MS |
|---|---|---|---|
| Sub A-1 | m/z=273.96($C_{13}H_6OS_3$=274.37) | Sub A-2 | m/z=257.98($C_{13}H_6O_2S_2$=258.31) |
| Sub A-3 | m/z=257.98($C_{13}H_6O_2S_2$=258.31) | Sub A-4 | m/z=324.06($C_{19}H_{16}OS_2$=324.46) |
| Sub A-5 | m/z=252.08($C_{16}H_{12}O_3$=252.27) | Sub A-6 | m/z=284.03($C_{15}H_{12}O_2SSi$=284.40) |
| Sub A-7 | m/z=226.03($C_{13}H_6O_4$=226.19) | Sub A-8 | m/z=369.85($C_{13}H_6OSSe_2$=368.19) |
| Sub A-9 | m/z=242.00($C_{13}H_6O_3S$=242.25) | Sub A-10 | m/z=290.07($C_{16}H_6D_6OS_2$=290.43) |
| Sub A-11 | m/z=268.06($C_{16}H_{12}O_2S$=268.33) | Sub A-12 | m/z=284.03($C_{16}H_{12}OS_2$=284.39) |

(continued)

| Compound | FD-MS | Compound | FD-MS |
|---|---|---|---|
| Sub A-13 | m/z=274.96($C_{13}H_5DOS_3$=275.38) | Sub A-14 | m/z=257.98($C_{13}H_6O_2S_2$=258.31) |
| Sub A-15 | m/z=257.98($C_{13}H_6O_2S_2$=258.31) | Sub A-16 | m/z=284.03($C_{16}H_{12}OS_2$=284.39) |
| Sub A-17 | m/z=284.03($C_{16}H_{12}OS_2$=284.39) | Sub A-18 | m/z=332.98($C_{16}H_{11}DOSSe$=332.31) |
| Sub A-19 | m/z=242.00($C_{13}H_6O_3S$=242.25) | Sub A-20 | m/z=257.98($C_{13}H_6O_2S_2$=258.31) |
| Sub A-21 | m/z=257.98($C_{13}H_6O_2S_2$=258.31) | Sub A-22 | m/z=268.06($C_{16}H_{12}O_2S$=268.33) |
| Sub A-23 | m/z=300.01($C_{15}H_{12}O_S2Si$=300.46) | Sub A-24 | m/z=268.06($C_{16}H_{12}O_2S$=268.33) |
| Sub A-25 | m/z=268.00($C_{15}H_8OS_2$=268.35) | Sub A-26 | m/z=293.12($C_{19}H_{15}DO_3$=293.34) |
| Sub A-27 | m/z=315.95($C_{15}H_8OSSe$=315.26) | Sub A-28 | m/z=279.08($C_{18}H_{13}DOS$=279.38) |
| Sub A-29 | m/z=384.12($C_{28}H_{16}O_2$=384.43) | Sub A-30 | m/z=326.02($C_{18}H_{14}OSe$=325.28) |
| Sub A-31 | m/z=268.00($C_{15}H_8OS_2$=268.35) | Sub A-32 | m/z=315.95($C_{15}H_8OSSe$=315.26) |
| Sub A-33 | m/z=363.89($C_{15}H_8OSe_2$=362.17) | Sub A-34 | m/z=278.08($C_{18}H_{14}OS$=278.37) |
| Sub A-35 | m/z=400.09($C_{27}H_{16}O_2Si$=400.51) | Sub A-36 | m/z=450.05($C_{28}H_{18}OSe$=449.42) |
| Sub A-37 | m/z=329.93($C_{15}H_6OS_4$=330.45) | Sub A-38 | m/z=313.95($C_{15}H_6O_2S_3$=314.39) |
| Sub A-39 | m/z=329.94($C_{15}H_6O_4Se$=329.18) | Sub A-40 | m/z=342.02($C_{18}H_{10}D_2OS_3$=342.49) |
| Sub A-41 | m/z=387.95($C_{18}H_{12}OS_2Se$=387.38) | Sub A-42 | m/z=292.07($C_{18}H_{12}O_4$=292.29) |
| Sub A-43 | m/z=266.02($C_{15}H_6O_5$=266.21) | Sub A-44 | m/z=297.98($C_{15}H_6O_3S_2$=298.33) |
| Sub A-45 | m/z=297.98($C_{15}H_6O_3S_2$=298.33) | Sub A-46 | m/z=292.07($C_{18}H_{12}O_4$=292.29) |
| Sub A-47 | m/z=324.03($C_{18}H_{12}O_2S_2$=324.41) | Sub A-48 | m/z=324.03($C_{18}H_{12}O_2S_2$=324.41) |
| Sub A-49 | m/z=313.95($C_{15}H_6O_2S_3$=314.39) | Sub A-50 | m/z=313.95($C_{15}H_6O_2S_3$=314.39) |
| Sub A-51 | m/z=297.98($C_{15}H_6O_3S_2$=298.33) | Sub A-52 | m/z=340.01($C_{18}H_{12}OS_3$=340.47) |
| Sub A-53 | m/z=292.07($C_{18}H_{12}O_4$=292.29) | Sub A-54 | m/z=324.03($C_{18}H_{12}O_2S_2$=324.41) |
| Sub A-55 | m/z=329.93($C_{15}H_6OS_4$=330.45) | Sub A-56 | m/z=266.02($C_{15}H_6O_5$=266.21) |
| Sub A-57 | m/z=377.87($C_{15}H_6OS_3Se$=377.36) | Sub A-58 | m/z=340.01($C_{18}H_{12}OS_3$=340.47) |
| Sub A-59 | m/z=387.95($C_{18}H_{12}OS_2Se$=387.38) | Sub A-60 | m/z=388.96($C_{18}H_{11}DOS_2Se$=388.39) |
| Sub A-61 | m/z=395.98($C_{20}H_{12}OS_4$=396.56) | Sub A-62 | m/z=374.10($C_{23}H_{18}O_3S$=374.45) |
| Sub A-63 | m/z=332.07($C_{20}H_{12}O_5$=332.31) | Sub A-64 | m/z=390.07($C_{23}H_{18}O_2S_2$=390.51) |
| Sub A-65 | m/z=395.98($C_{20}H_{12}OS_4$=396.56) | Sub A-66 | m/z=395.98($C_{20}H_{12}OS_4$=396.56) |
| Sub A-67 | m/z=509.85($C_{20}H_{14}OSe_3$=507.24) | Sub A-68 | m/z=366.02($C_{20}H_{12}OS_3$=366.51) |
| Sub A-69 | m/z=362.13($C_{23}H_{22}O_2S$=362.49) | Sub A-70 | m/z=449.01($C_{24}H_{15}DOS_4$=449.64) |
| Sub A-71 | m/z=384.14($C_{25}H_{20}O_4$=384.43) | Sub A-72 | m/z=421.99($C_{22}H_{14}OS_4$=422.59) |

Synthesis example of Sub B

1. Synthesis example of Sub B-1

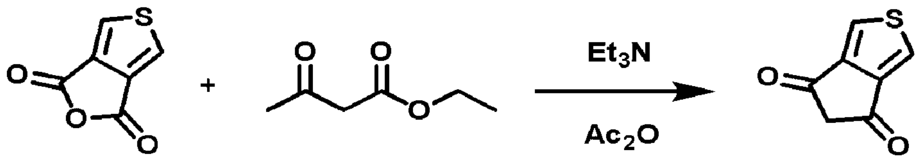


Sub B-1

Triethylamine (26.2 g, 259.5 mmol) and ethyl acetoacetate (18.5 g, 142.7 mmol) were added to a mixture of 1H,3H-thieno[3,4-c]furan-1,3-dione (20 g, 129.8 mmol) and acetic anhydride (132.4 g, 1297.5 mmol), and the mixture was stirred at 65 °C for 24 hours. After completion of the reaction, the reaction mixture was poured into ice water and extracted with methyl chloride, then concentrated. HCl was added, and the mixture was stirred at 70 °C for 1 hour, cooled to room temperature, and extracted with methyl chloride. The organic layer was dried over $MgSO_4$ and concentrated. The resulting concentrate was separated by silica gel column and recrystallized to obtain the product (11.8 g, yield: 60%).

2. Synthesis example of Sub B-14

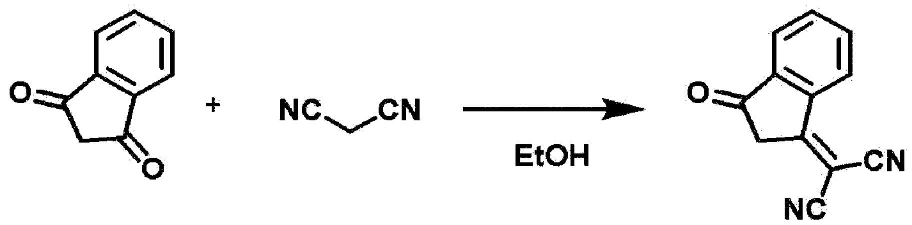


Sub B-14

1H-indene-1,3(2H)-dione (25 g, 171.1 mmol) and malononitrile (33.9 g, 513.2 mmol) were dissolved in ethanol (430 mL) and stirred for 30 minutes, followed by the addition of sodium acetate (18.2 g, 222.4 mmol), and the mixture was stirred for 1 hour. After completion of the reaction, the reaction mixture was slowly poured into water, and HCl was added until the pH reached 1-2. The resulting precipitate was filtered, washed with water, and was separated by silica gel column and recrystallized to obtain the product (11.6 g, yield: 35%).

3. Synthesis example of Sub B-18

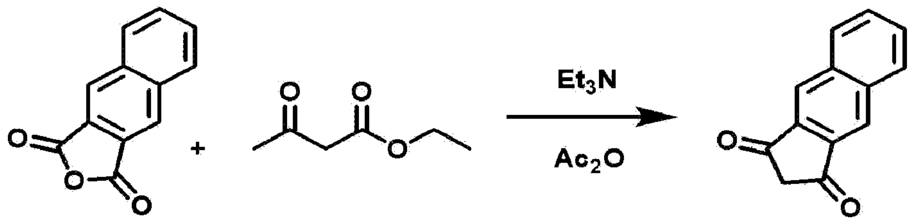


Sub B-18

Naphtho[2,3-c]furan-1,3-dione (20 g, 100.9 mmol) was used in place of 1H,3H-thieno[3,4-c]furan-1,3-dione, and the synthesis was carried out in the same manner as in synthesis example of Sub B-1 to obtain the product (12.7 g, yield: 64%).

4. Synthesis example of Sub B-34

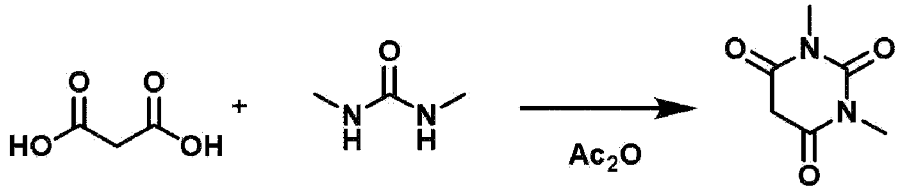


**Sub B-34**

Malonic acid (15 g, 144.1 mmol) and 1,3-dimethylurea (12.7 g, 144.1 mmol) were dissolved in acetic acid (290 mL), and acetic anhydride (29.4 g, 288.3 mmol) was added. The mixture was stirred at 70 °C for 20 hours. After completion of the reaction, the solvent was removed, and the residue was extracted with water and ethyl acetate. The concentrated compound was recrystallized at low temperature to obtain the product (16.2 g, yield: 72%).

The compound belonging to Sub B may comprise compounds as described below, but is not limited thereto. Table 2 shows the FD-MS values of the compounds.

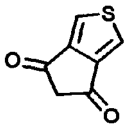

**Sub B-1**

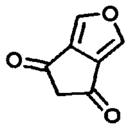

**Sub B-2**

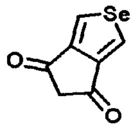

**Sub B-3**

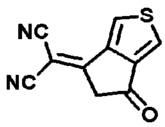

**Sub B-4**

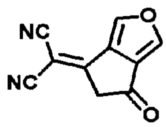

**Sub B-5**

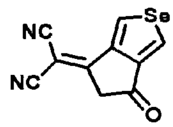

**Sub B-6**

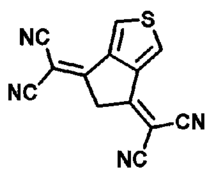

**Sub B-7**

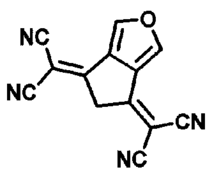

**Sub B-8**

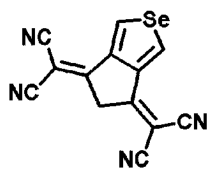

**Sub B-9**

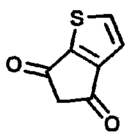

**Sub B-10**

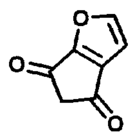

**Sub B-11**

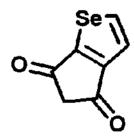

**Sub B-12**

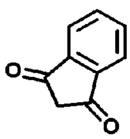

**Sub B-13**

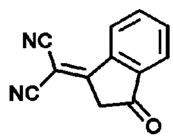

**Sub B-14**

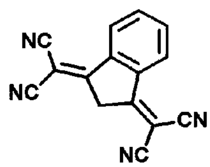

**Sub B-15**

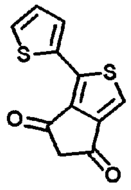

Sub B-16

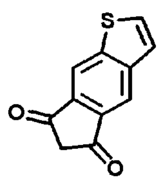

Sub B-17

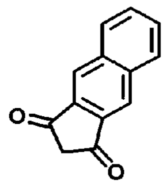

Sub B-18

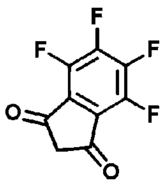

Sub B-19

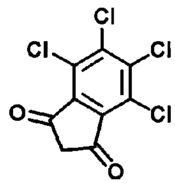

Sub B-20

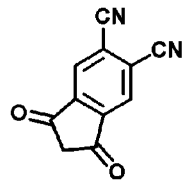

Sub B-21

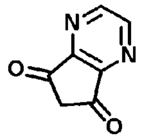

Sub B-22

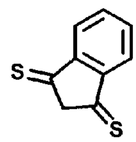

Sub B-23

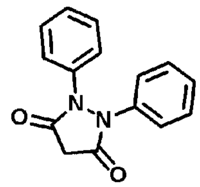

Sub B-24

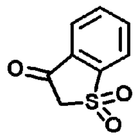

Sub B-25

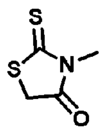

Sub B-26

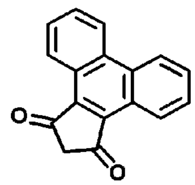

Sub B-27

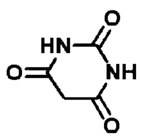

Sub B-28

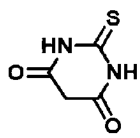

Sub B-29

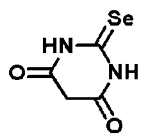

Sub B-30

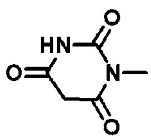

Sub B-31

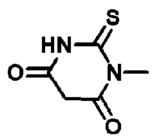

Sub B-32

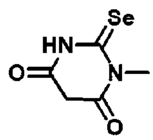

Sub B-33

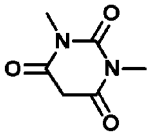

Sub B-34

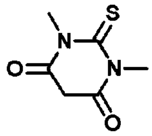

Sub B-35

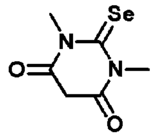

Sub B-36

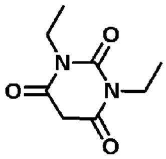

Sub B-37

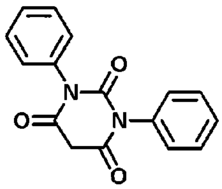

Sub B-38

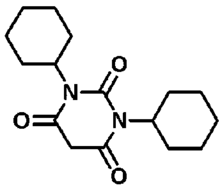

Sub B-39

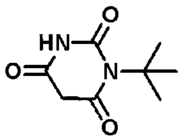

Sub B-40

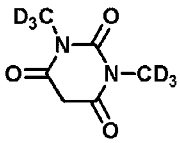

Sub B-41

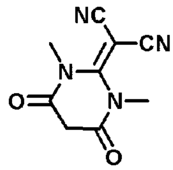

Sub B-42

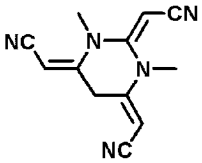

Sub B-43

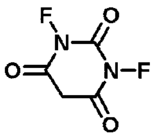

Sub B-44

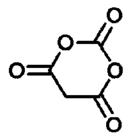

Sub B-45

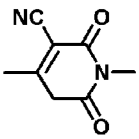

Sub B-46

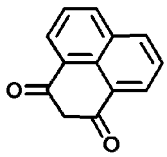

Sub B-47

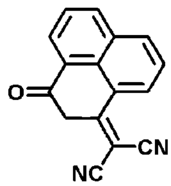

Sub B-48

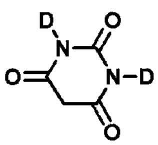

Sub B-49

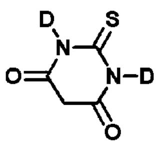

Sub B-50

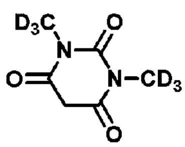

Sub B-51

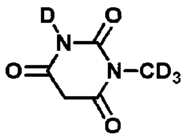

Sub B-52

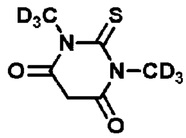

Sub B-53

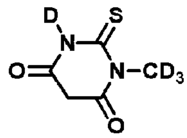

Sub B-54

[Table 2]

| Compound | FD-MS | Compound | FD-MS |
|---|---|---|---|
| Sub B-1 | m/z=151.99($C_7H_4O_2S$=152.17) | Sub B-2 | m/z=136.02($C_7H_4O_3$=136.11) |
| Sub B-3 | m/z=199.94($C_7H_4O_2Se$=199.08) | Sub B-4 | m/z=200.00($C_{10}H_4N_2OS$=200.22) |
| Sub B-5 | m/z=184.03($C_{10}H_4N_2O_2$=184.15) | Sub B-6 | m/z=247.95($C_{10}H_4N_2OSe$=247.13) |
| Sub B-7 | m/z=248.02($C_{13}H_4N_4S$=248.26) | Sub B-8 | m/z=232.04($C_{13}H_4N_4O$=232.2) |
| Sub B-9 | m/z=295.96($C_{13}H_4N_4Se$=295.17) | Sub B-10 | m/z=151.99($C_7H_4O_2S$=152.17) |
| Sub B-11 | m/z=136.02($C_7H_4O_3$=136.11) | Sub B-12 | m/z=199.94($C_7H_4O_2Se$=199.08) |
| Sub B-13 | m/z=146.04($C_9H_6O_2$=146.15) | Sub B-14 | m/z=194.05($C_{12}H_6N_2O$=194.19) |
| Sub B-15 | m/z=242.06($C_{15}H_6N_4$=242.24) | Sub B-16 | m/z=233.98($C_{11}H_6O_2S_2$=234.29) |
| Sub B-17 | m/z=202.01($C_{11}H_6O_2S$=202.23) | Sub B-18 | m/z=196.05($C_{13}H_8O_2$=196.21) |
| Sub B-19 | m/z=218.00($C_9H_2F_4O_2$=218.11) | Sub B-20 | m/z=281.88($C_9H_2Cl_4O_2$=283.91) |
| Sub B-21 | m/z=196.03($C_{11}H_4N_2O_2$=196.17) | Sub B-22 | m/z=148.03($C_7H_4N_2O_2$=148.12) |
| Sub B-23 | m/z=177.99($C_9H_6S_2$=178.27) | Sub B-24 | m/z=252.09($C_{15}H_{12}N_2O_2$=252.27) |
| Sub B-25 | m/z=182.00($C_8H_6O_3S$=182.19) | Sub B-26 | m/z=146.98($C_4H_5NOS_2$=147.21) |
| Sub B-27 | m/z=246.07($C_{17}H_{10}O_2$=246.27) | Sub B-28 | m/z=128.02($C_4H_4N_2O_3$=128.09) |
| Sub B-29 | m/z=144.00($C_4H_4N_2O_2S$=144.15) | Sub B-30 | m/z=191.94($C_4H_4N_2O_2Se$=191.06) |
| Sub B-31 | m/z=142.04($C_5H_6N_2O_3$=142.11) | Sub B-32 | m/z=158.01($C_5H_6N_2O_2S$=158.18) |
| Sub B-33 | m/z=205.96($C_5H_6N_2O_2Se$=205.09) | Sub B-34 | m/z=156.05($C_6H_8N_2O_3$=156.14) |
| Sub B-35 | m/z=172.03($C_6H_8N_2O_2S$=172.2) | Sub B-36 | m/z=219.98($C_6H_8N_2O_2Se$=219.11) |
| Sub B-37 | m/z=184.08($C_8H_{12}N_2O_3$=184.2) | Sub B-38 | m/z=280.08($C_{16}H_{12}N_2O_3$=280.28) |
| Sub B-39 | m/z=292.18($C_{16}H_{24}N_2O_3$=292.38) | Sub B-40 | m/z=184.08($C_8H_{12}N_2O_3$=184.2) |
| Sub B-41 | m/z=162.09($C_6H_2D_6N_2O_3$=162.18) | Sub B-42 | m/z=204.06($C_9H_8N_4O_2$=204.19) |
| Sub B-43 | m/z=225.10($C_{12}H_{11}N_5$=225.26) | Sub B-44 | m/z=164.00($C_4H_2F_2N_2O_3$=164.07) |
| Sub B-45 | m/z=129.99($C_4H_2O_5$=130.06) | Sub B-46 | m/z=164.06($C_8H_8N_2O_2$=164.16) |
| Sub B-47 | m/z=196.05($C_{13}H_8O_2$=196.21) | Sub B-48 | m/z=244.06($C_{16}H_8N_2O$=244.25) |
| Sub B-49 | m/z= 130.03($C_4H_2D_2N_2O_3$=130.10) | Sub B-50 | m/z=146.01($C_4H_2D_2N_2O_2S$=146.16) |
| Sub B-51 | m/z= 162.09($C_6H_2D_6N_2O_3$=162.18) | Sub B-52 | m/z=146.06($C_5H_2D_4N_2O_3$=146.14) |
| Sub B-53 | m/z= 178.07($C_6H_2D_6N_2O_2S$=178.24) | Sub B-54 | m/z=162.04($C_5H_2D_4N_2O_2S$=162.20) |

Synthesis example of final product

1. Synthesis example of P12

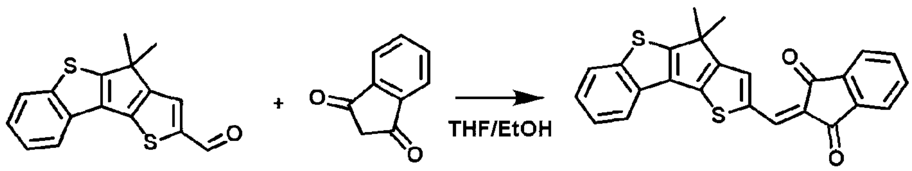


Sub A-12 Sub B-13 P12

Sub B-13 (6.17 g, 42.20 mmol), THF (117 mL) and EtOH (58 mL) were added to Sub A-12 (10 g, 35.16 mmol), and the mixture was reacted at 80 °C for 10 hours. Upon completion of the reaction, the solvent was removed, and the resulting product was recrystallized to obtain the product (13.78 g, yield: 95%).

2. Synthesis example of P17

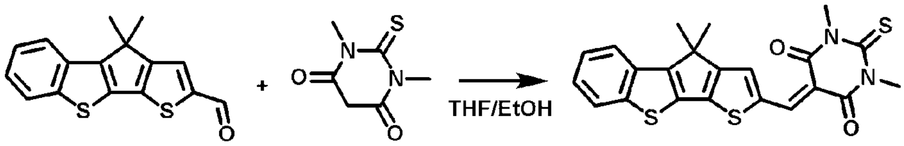


Sub A-17 Sub B-35 P17

Sub B-35 (7.27 g, 42.20 mmol), THF (117 mL) and EtOH (58 mL) were added to Sub A-17 (10 g, 35.16 mmol), and the reaction was carried out in the same manner as in synthesis example of P12 to obtain the product (13.73 g, yield: 89%).

3. Synthesis example of P38

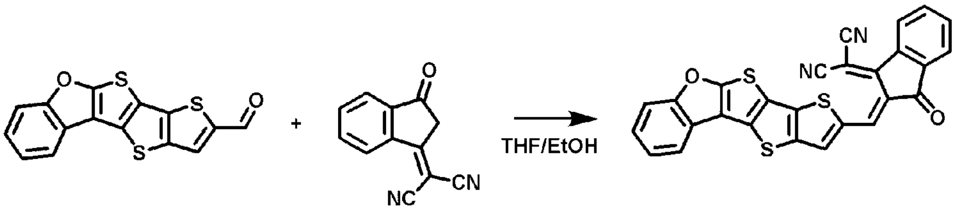


Sub A-38 Sub B-14 P38

Sub B-14 (7.41 g, 38.17 mmol), THF (106 mL) and EtOH (53 mL) were added to Sub A-38 (10 g, 31.81 mmol), and the reaction was carried out in the same manner as in synthesis example of P12 to obtain the product (13.42 g, yield: 86%).

4. Synthesis example of P58

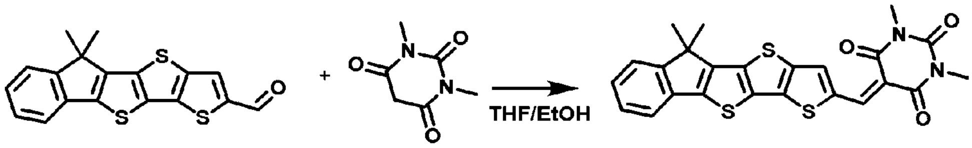


Sub A-58 Sub B-34 P58

Sub B-34 (5.50 g, 35.25 mmol), THF (98 mL) and EtOH (48 mL) were added to Sub A-58 (10 g, 29.37 mmol), and the reaction was carried out in the same manner as in synthesis example of P12 to obtain the product (13.21 g, yield: 94%).

5. Synthesis example of P72

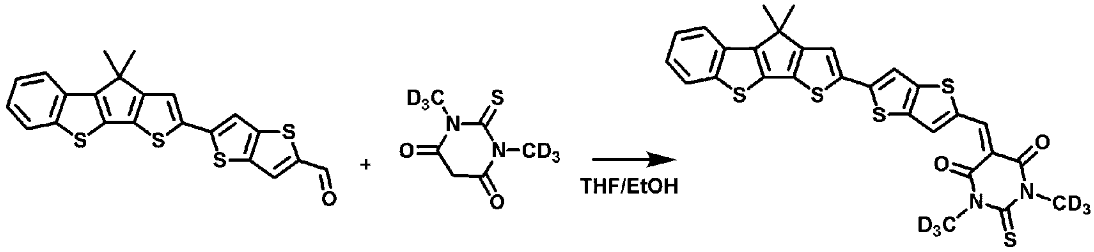


**Sub A-72** **Sub B-53** **P72**

Sub B-53 (5.06 g, 28.40 mmol), THF (80 mL) and EtOH (40 mL) were added to Sub A-72 (10 g, 23.66 mmol), and the reaction was carried out in the same manner as in synthesis example of P12 to obtain the product (12.41 g, yield: 90%).

The FD-MS values of compounds P1 to P75, which were prepared according to the above synthesis examples, are shown in Table 3.

[Table 3]

| Compound | FD-MS | Compound | FD-MS |
|---|---|---|---|
| P1 | m/z=391.96($C_{20}H_8O_3S_3$=392.46) | P2 | m/z=412.00($C_{19}H_{12}N_2O_3S_3$=412.50) |
| P3 | m/z=444.04($C_{22}H_{12}N_4O_3S_2$=444.48) | P4 | m/z=458.05($C_{26}H_{18}O_2S_3$=458.61) |
| P5 | m/z=430.12($C_{29}H_{18}O_4$=430.46) | P6 | m/z=438.05($C_{21}H_{18}N_2O_3S_2Si$=438.59) |
| P7 | m/z=404.07($C_{26}H_{12}O_5$=404.38) | P8 | m/z=479.86($C_{17}H_8N_2O_3SSe_2$=478.26) |
| P9 | m/z=386.08($C_{19}H_6D_6N_2O_5S$=386.41) | P10 | m/z=474.07($C_{27}H_{10}D_6O_2S_3$=474.64) |
| P11 | m/z=496.11($C_{33}H_{20}O_3S$=496.58) | P12 | m/z=412.06($C_{25}H_{16}O_2S_2$=412.52) |
| P13 | m/z=402.99($C_{22}H_9DO_2S_3$=403.51) | P14 | m/z=396.02($C_{19}H_{12}N_2O_4S_2$=396.43) |
| P15 | m/z=465.07($C_{25}H_{15}N_5OS_2$=465.55) | P16 | m/z=438.05($C_{22}H_{18}N_2O_2S_3$=438.58) |
| P17 | m/z=438.05($C_{22}H_{18}N_2O_2S_3$=438.58) | P18 | m/z=522.94($C_{25}H_{13}DO_2S_3Se$=522.54) |
| P19 | m/z=388.00($C_{17}H_6F_2N_2O_5S$=388.30) | P20 | m/z=404.02($C_{22}H_{12}O_4S_2$=404.45) |
| P21 | m/z=385.98($C_{20}H_6N_2O_3S_2$=386.40) | P22 | m/z=422.08($C_{22}H_{18}N_2O_3S_2$=422.52) |
| P23 | m/z=515.98($C_{26}H_{16}O_2S_4Si$=516.74) | P24 | m/z=498.13($C_{33}H_{22}O_3S$=498.60) |
| P25 | m/z=406.04($C_{21}H_{14}N_2O_3S_2$=406.47) | P26 | m/z=421.14($C_{28}H_{19}DO_4$=421.47) |
| P27 | m/z=449.93($C_{22}H_{10}O_2S_2Se$=449.41) | P28 | m/z=443.09($C_{27}H_{15}DF_2O2S$=443.49) |
| P29 | m/z=560.15($C_{40}H_{20}N_2O_2$=560.61) | P30 | m/z=504.06($C_{31}H_{20}O_2Se$=503.47) |
| P31 | m/z=378.05($C_{20}H_{14}N_2O_2S_2$=378.46) | P32 | m/z=493.97($C_{25}H_{10}N_4OSSe$=493.41) |
| P33 | m/z=545.88($C_{25}H_{10}N_2OSSe_2$=544.37) | P34 | m/z=402.10($C_{23}H_{18}N_2O_3S$=402.47) |
| P35 | m/z=566.11($C_{37}H_{18}N_2O_3Si$=566.65) | P36 | m/z=632.05($C_{38}H_{20}N_2OSSe$=631.62) |
| P37 | m/z=496.00($C_{23}H_{1\ 6}N_2O_3S_4$=496.63) | P38 | m/z=489.99($C_{27}H_{10}N_2O_2S_3$=490.57) |
| P39 | m/z=445.97($C_{23}H_{10}O_5Se$=445.30) | P40 | m/z=486.10($C_{24}H_{10}D_8N_2O_3S_3$=486.65) |
| P41 | m/z=534.00($C_{26}H_{18}N_2O_2S_2Se$=533.53) | P42 | m/z=422.15($C_{28}H_{14}D_4O_4$=422.47) |
| P43 | m/z=420.04($C_{21}H_{12}N_2O_6S$=420.40) | P44 | m/z=409.96($C_{19}H_6O_7S_2$=410.37) |
| P45 | m/z=522.02($C_{30}H_{10}N_4O_2S_2$=522.56) | P46 | m/z=446.09($C_{24}H_{18}N_2O_5S$=446.48) |

(continued)

| Compound | FD-MS | Compound | FD-MS |
|---|---|---|---|
| P47 | m/z=434.08($C_{23}H_{18}N_2O_3S_2$=434.53) | P48 | m/z=502.04($C_{29}H_{14}N_2O_3S_2$=502.56) |
| P49 | m/z=447.94($C_{22}H_8O_3S_4$=448.54) | P50 | m/z=452.00($C_{21}H_{12}N_2O_4S_3$=452.52) |
| P51 | m/z=457.96($C_{24}H_{10}O_2S_4$=458.58) | P52 | m/z=486.05($C_{26}H_{18}N_2O_2S_3$=486.62) |
| P53 | m/z=526.15($C_{33}H_{22}N_2O_5$=526.55) | P54 | m/z=470.04($C_{27}H_{15}FO_3S_2$=470.53) |
| P55 | m/z=593.80($C_{24}H_6Cl_4O_2S_4$=596.35) | P56 | m/z=432.10($C_{23}H_{16}N_2O_7$=432.39) |
| P57 | m/z=556.91($C_{27}H_{11}NO_2S_3Se$=556.54) | P58 | m/z=478.05($C_{24}H_{18}N_2O_3S_3$=478.60) |
| P59 | m/z=516.92($C_{22}H_{15}NOS_4Se$=516.58) | P60 | m/z=588.94($C_{27}H_{11}DF_4O_2S_2Se$=588.48) |
| P61 | m/z=525.99($C_{27}H_{14}N_2O_2S_4$=526.66) | P62 | m/z=484.11($C_{27}H_{20}N_2O_5S$=484.53) |
| P63 | m/z=466.05($C_{27}H_{14}O_6S$=466.46) | P64 | m/z=518.03($C_{27}H_{18}O_5S_3$=518.62) |
| P65 | m/z=572.01($C_{32}H_{16}N_2OS_4$=572.73) | P66 | m/z=582.03($C_{29}H_{18}N_4O_2S_4$=582.73) |
| P67 | m/z=637.88($C_{29}H_{18}O_2Se_3$=635.37) | P68 | m/z=504.06($C_{26}H_{20}N_2O_3S_3$=504.64) |
| P69 | m/z=516.15($C_{29}H_{28}N2O_3S_2$=516.67) | P70 | m/z=577.04($C_{33}H_{19}DO_2S_4$=577.77) |
| P71 | m/z=528.22($C_{31}H_{20}D_6N_2O_6$=528.59) | P72 | m/z=582.05($C_{28}H_{14}D_6N_2O_2S_5$=582.82) |
| P73 | m/z=538.08($C_{27}H_{14}D_6N_2O_2S_4$=538.75 ) | P74 | m/z=483.21($C_{27}H_{13}D_{11}N_2O_4S$=483.63 ) |
| P75 | m/z=580.09($C_{32}H_{24}N_2O_3S_3$=580.74) | | |

Although the synthesis examples of the present invention represented by Formula 1 have been described above, they are all based on Suzuki cross-coupling reactions, Miyaura boration reactions, $PPh_3$-mediated reductive cyclization reactions (J. Org. Chem. 2005, 70, 5014), Buchwald-Hartwig cross-coupling reactions, and the like. Therefore, even if substituents other than those specified in the detailed synthesis examples are attached as defined in Formula 1, those skilled in the art can easily understand that the aforementioned reactions will proceed.

Meanwhile, the quantum efficiency of an organic photoelectric element is influenced by the oscillator strength (f).

The oscillator strength is a dimensionless value representing the degree of interaction with light, and it indicates the absorption and emission intensity of the corresponding material. In the present invention, the oscillator strength was calculated based on the transition dipole moment (TDM) from the ground state to the first singlet excited state ($S_1$) according to the Franck-Condon principle. The calculation formula is as follows.

$$f = \frac{8\pi^2 \tilde{\nu} m_e c}{3he^2} |\mu|^2$$

(f: Oscillator strength, $\tilde{V}$: $S_1$ state wave number, $m_e$: Electron mass, c: Speed of light, h: Planck's constant, e: Charge of electron, $|\mu|^2$: Transition dipole moment strength)

In the present invention, the Jaguar module, a quantum mechanics calculation module of Materials Science (Maestro Materials Science 4.9.128, Release 2023-1) from Schrodinger, was used, and molecular structure optimization was performed using Density Functional Theory (DFT) of the module. The B3LYP method (Becke, 3-parameter, Lee-Yang-Parr), which is known to give results similar to experimental values for organic compounds, was used as the DFT method, and the 6-31G(d) Pople basis set was employed. Based on the optimized molecular structure, the oscillator strength of the first singlet excited state according to the Franck-Condon principle was calculated using TD-B3LYP, which applies time-dependent density functional theory (TD-DFT).

From the above equation, it can be seen that the oscillator strength is proportional to the TDM strength. Therefore, a larger oscillator strength corresponds to a greater TDM strength, which represents a higher probability and intensity of electronic transition. This implies a larger molar absorptivity at the corresponding wavelength in the UV/VIS spectrum, meaning that the compound absorbs more light. The greater the light absorption, the higher the potential quantum efficiency of the compound.

Fabrication of organic photoelectric element and measurement of external quantum efficiency

[Test Example 1] Organic photoelectric element

After forming a 150 nm thick ITO layer on a glass substrate, a 150 nm thick active layer was formed on the ITO layer by co-depositing the compound P12 of the present invention and $C_{60}$ (fullerene) at a volume ratio of 1.1:1, and a 5 nm thick ITO layer was vacuum-deposited on the active layer, thereby fabricating an organic photoelectric element.

[Test Example 2] to [Test Example 10]

An organic photoelectric element was fabricated in the same manner as in Test Example 1, except that a compound of the present invention listed in Table 4 was used as the active layer material instead of the compound P12 of the present invention.

[Comparative example 1] to [Comparative example 3]

An organic photoelectric element was fabricated in the same manner as in Test Example 1, except that one of Comparative Compounds A to C was used as the active layer material instead of the compound P12 of the present invention."

<Comparative Compound A>

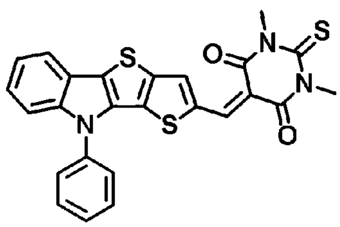

<Comparative Compound B>

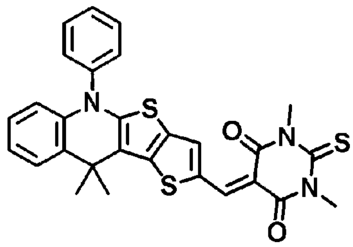

<Comparative Compound C>

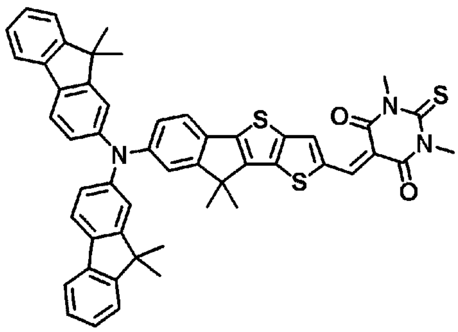

The external quantum efficiency (EQE) of the organic photoelectric elements fabricated in the above Test Examples and Comparative Examples was measured using IPCE measurement (Mc Science). The measurement results are shown in Table 4. The measurement apparatus allows comparison of the performance of the new material with comparative compounds under the same conditions, without being affected by daily variations of parameters. Therefore, the experimental values obtained using this measurement apparatus have statistical significance.

[Table 4]

| | Compound | EQE (room temperature, %) |
|---|---|---|
| comp.Ex 1 | Comp. compd A | 34% |
| comp.Ex 2 | Comp. compd B | 52% |
| comp.Ex 3 | Comp. compd C | 31% |
| Test Ex. 1 | Com. P12 | 63% |

(continued)

| | Compound | EQE (room temperature, %) |
|---|---|---|
| Test Ex. 2 | Com. P13 | 66% |
| Test Ex. 3 | Com. P16 | 68% |
| Test Ex. 4 | Com. P17 | 70% |
| Test Ex. 5 | Com. P40 | 64% |
| Test Ex. 6 | Com. P58 | 71% |
| Test Ex. 7 | Com. P60 | 72% |
| Test Ex. 8 | Com. P67 | 67% |
| Test Ex. 9 | Com. P68 | 71% |
| Test Ex. 10 | Com. P72 | 72% |

As can be seen from the results in Table 4, the EQE of the organic photoelectric elements using the material of the present invention as the active layer is significantly improved compared to devices using the comparative compounds. Comparative Compound A has a structure containing an indole ring, and Comparative Compound B has a structure containing a dihydroquinoline ring, which differ from the compounds of the present invention that include an indenyl, benzofuran, benzothiophene, or benzoselenophene structure. Comparative Compound C differs from the compounds of the present invention in that an amino group is substituted on the core, whereas $R^1$ in the compounds of the present invention cannot be an amino group.

Table 5 shows the excitation energy and oscillator strength (f) of Compound P16 and Comparative Compounds A to C, measured using molecular simulation (Schrodinger Maestro Materials Science 4.9.128, Release 2023-1).

[Table 5]

| material name | P16 | Comp. compd A | Comp. compd B | Comp. compd C |
|---|---|---|---|---|
| Excitation Energy | 462 | 448 | 495 | 446 |
| oscillator strength | 1.348 | 1.155 | 1.199 | 0.950 |
| molecular weight | 438.58 | 487.61 | 529.69 | 838.12 |

As can be seen from Table 5, the excitation energy of the present invention allows absorption of light in the green region (450-650 nm), whereas Comparative Compounds A and C have excitation energies below 450 nm, which are inadequate for absorbing light in the green region. Regarding the oscillator strength, Compound P16 of the present invention exhibits the highest value. Compared to Comparative Compound B, which has the highest oscillator strength among Comparative Compounds A to C, the oscillator strength of Compound P16 of the present invention is approximately 12% higher.

A higher oscillator strength leads to a higher probability and intensity of electronic transitions, as well as potentially higher molar absorptivity. This implies that, for an active layer of the same thickness in an organic photoelectric element, a higher oscillator strength would result in improved quantum efficiency. As shown in Table 4, the quantum efficiency appears to be influenced by the oscillator strength.

In particular, comparing Comparative Compound C and Compound P16 of the present invention, the substitution of an amino group on the core decreases both the excitation energy (nm) and oscillator strength, resulting in a significant difference in EQE, as observed in Table 4.

Therefore, when the compound of the present invention is used as the p-type semiconductor material in an organic photoelectric element, the high oscillator strength can significantly contribute to increasing the overall quantum efficiency of the element.

As can be seen from Tables 4 and 5 and Figures 1 and 2, even for compounds with similar structures, it can be observed that when a compound satisfying all the combined factors, such as the type and substitution position of substituents, as in the compounds of the present invention, is used as a material for an organic photoelectric element, it exhibits significantly superior performance compared to the comparative compounds. Therefore, it can be understood that, in addition to the comparative compounds, the compounds of the present invention would exhibit remarkable effects when used as materials for organic photoelectric elements, compared to other compounds having structures similar to those of the present invention.

The above description is merely illustrative of the present invention, and it will be apparent to those skilled in the art to which the present invention pertains that various modifications can be made without departing from the essential characteristics of the present invention. Accordingly, the embodiments disclosed in the present specification are not intended to limit the present invention but to explain it, and the spirit and scope of the present invention are not limited by these embodiments. The scope of protection of the present invention should be construed based on the claims set forth below, and all technologies within a scope equivalent thereto should be interpreted as being included within the scope of rights of the present invention.

## Claims

1. A compound of Formula 1:

<Formula 1>

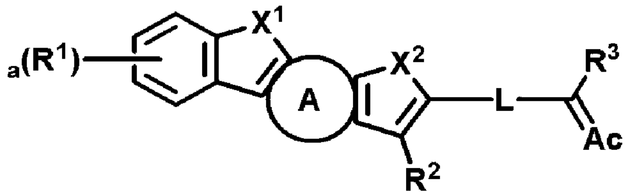

in Formula 1,

A ring is selected from the group consisting of the following Formula A-1 to A-4,

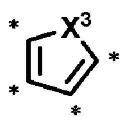

A-1

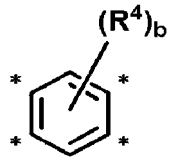

A-2

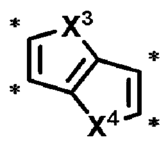

A-3

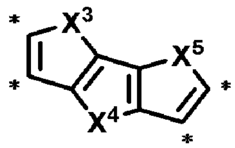

A-4

$X^1$ to $X^5$ are each independently O, S, Se, Te, $C(R^a)(R^b)$ or $Si(R^c)(R^d)$,
$R^1$ to $R^4$, $R^a$ to $R^d$ are each independently selected from the group consisting of hydrogen, deuterium, halogen, a $C_6$-$C_{60}$ aryl group, a fluorenyl group, a $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{30}$ alkyl group, a $C_2$-$C_{30}$ alkenyl group, a $C_2$-$C_{30}$ alkynyl group, a $C_1$-$C_{30}$ alkoxyl group, a $C_1$-$C_{30}$ alkylthio group, and a $C_6$-$C_{30}$ aryloxy group, and adjacent $R^1$ groups may be bonded to each other to form a ring, $R^a$ and $R^b$ may be bonded to each other to form a ring, $R^c$ and $R^d$ may be bonded to each other to form a ring,
a is an integer from 0 to 4, b is an integer from 0 to 2,
L is selected from the group consisting of a single bond, a $C_6$-$C_{60}$ arylene group, a fluorenylene group, a $C_2$-$C_{60}$ heterocyclic group containing at least one heteroatom of O, N, S, Si and P, a $C_3$-$C_{60}$ aliphatic ring group, a fused ring of a $C_3$-$C_{60}$ aliphatic ring and a $C_6$-$C_{60}$ aromatic ring, a $C_1$-$C_{30}$ alkylene group, a $C_2$-$C_{30}$ alkenylene group, a $C_2$-$C_{30}$ alkynylene group, and a $C_1$-$C_{30}$ akoxylene group,
Ac is selected from the group consisting of a $C_2$-$C_{60}$ heterocyclic group containing at least one heteroatom of O, N, S, Si and P, a $C_3$-$C_{60}$ aliphatic ring group, and a fused ring of a $C_3$-$C_{60}$ aliphatic ring and a $C_6$-$C_{60}$ aromatic ring, and Ac comprises one or more functional groups selected from the group consisting of C=O, C=S, C=Se, CN and $CF_3$,
L and $R^3$ may be bonded to each other to form a ring, $R^3$ and Ac may be bonded to each other to form a ring, and the aryl group, the arylene group, the fluorenyl group, the fluorenylene group, the heterocyclic group, the aliphatic ring group, the fused ring, the alkyl group, the alkylene group, the alkenyl group, the alkenylene group, the alkynyl group, the alkynylene group, the alkoxy group, the alkoxylene group, the arylthio group, the aryloxyl group, and the ring formed by adjacent groups may be each substituted with one or more substituents selected from the group consisting of deuterium, halogen, a siloxane group, a cyano group, a nitro group, a silane group

unsubstituted or substituted with a $C_1$-$C_{20}$ alkyl group or a $C_6$-$C_{20}$ aryl group, a phosphine oxide substituted or unsubstituted with a $C_1$-$C_{20}$ alkyl group or a $C_6$-$C_{20}$ aryl group, $C_1$-$C_{20}$ alkylthio group, $C_1$-$C_{20}$ alkoxy group, $C_6$-$C_{30}$ aryloxy group, $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{60}$ heterocyclic group comprising at least one heteroatom selected from the group consisting of O, N, S, Si and P, a $C_3$-$C_{30}$ aliphatic ring group, a fused ring of a $C_3$-$C_{30}$ aliphatic ring and a $C_6$-$C_{30}$ aromatic ring, =O, =C(CN)$_2$, and =S.

**2.** The compound of claim 1, wherein Formula 1 is represented by one of Formula 2 to Formula 15:

<Formula 2>

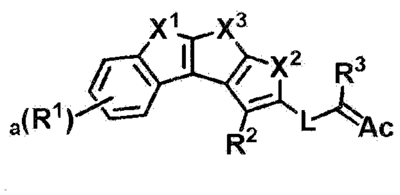

<Formula 3>

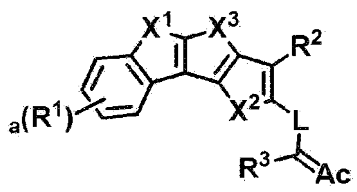

<Formula 4>

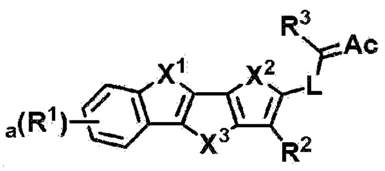

<Formula 5>

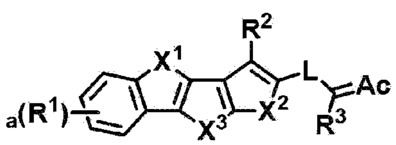

<Formula 6>

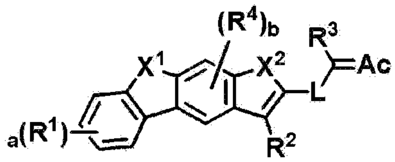

<Formula 7>

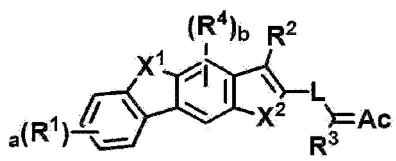

<Formula 8>

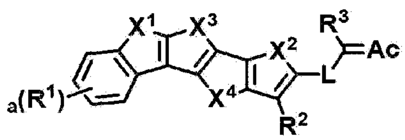

<Formula 9>

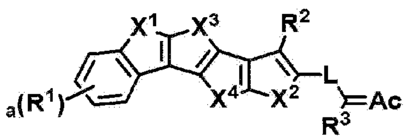

<Formula 10>

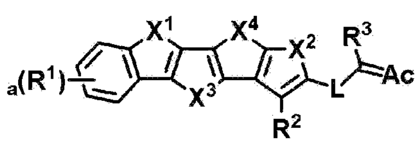

<Formula 11>

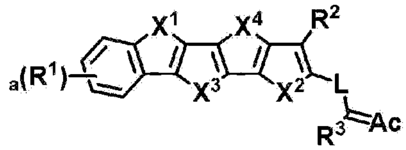

<Formula 12>

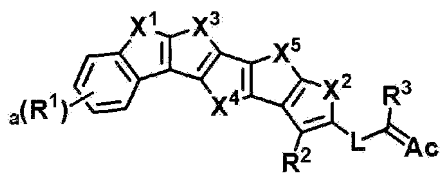

<Formula 13>

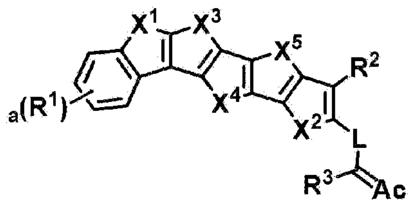

<Formula 14>

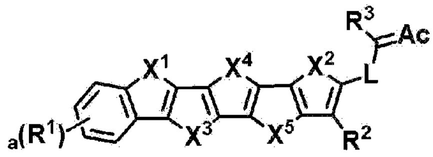

<Formula 15>

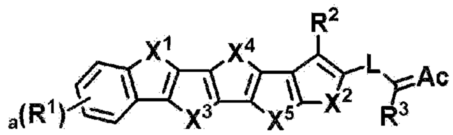

in Formula 2 to Formula 15, $X^1$ to $X^5$, $R^1$ to $R^3$, L, Ac, a are the same as defined in claim 1.

**3.** The compound of claim 1, wherein $R^a$ and $R^b$ are bonded to each other to form a ring, or $R^c$ and $R^d$ are be bonded to each other to form a ring.

**4.** The compound of claim 1, wherein Formula Ac is represented by one of Formula 16 to Formula 18:

<Formula 16>

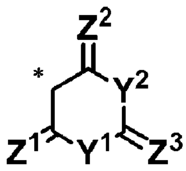

<Formula 17>

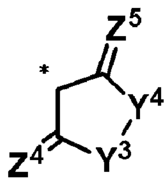

<Formula 18>

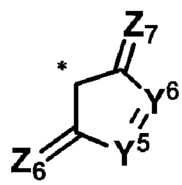

in Formula 16 to Formula 18,

$Z^1$ to $Z^5$, $Z_6$ and $Z_7$ are each independently O, S, Se, Te or $C(R^e)(R^f)$,
$Y^1$ to $Y^4$ are each independently O, S, Se, Te, $C(R^g)(R^h)$ or $N(R^i)$,
$Y^5$ and $Y^6$ are each independently N or $C(R^j)$,
$R^e$ to $R^i$ are each independently selected from the group consisting of hydrogen, deuterium, halogen, a cyano group, a $C_1$-$C_{20}$ alkyl group, and a $C_2$-$C_{20}$ alkenyl group,
$R^j$ is selected from the group consisting of hydrogen, deuterium, halogen, a siloxane group, a cyano group, a nitro group, a silane group unsubstituted or substituted with a $C_1$-$C_{20}$ alkyl group or a $C_6$-$C_{20}$ aryl group, a phosphine oxide substituted or unsubstituted with a $C_1$-$C_{20}$ alkyl group or a $C_6$-$C_{20}$ aryl group, $C_1$-$C_{20}$ alkylthio group, $C_1$-$C_{20}$ alkoxy group, $C_6$-$C_{30}$ aryloxy group, $C_6$-$C_{30}$ arylthio group, a $C_1$-$C_{20}$ alkyl group, a $C_2$-$C_{20}$ alkenyl group, a $C_2$-$C_{20}$ alkynyl group, a $C_6$-$C_{30}$ aryl group, a fluorenyl group, a $C_2$-$C_{60}$ heterocyclic group comprising at least one heteroatom selected from the group consisting of O, N, S, Si and P, and a $C_3$-$C_{30}$ aliphatic ring group, a fused ring of a $C_3$-$C_{30}$ aliphatic ring and a $C_6$-$C_{30}$ aromatic ring, =O, $=C(CN)_2$, and =S, and adjacent groups may be bonded to each other to form a ring.

**5.** The compound of claim 1, wherein the compound represented by Formula 1 is one of the following compounds:

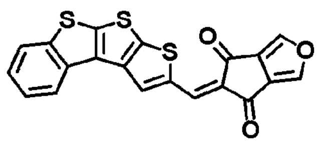

**P1**

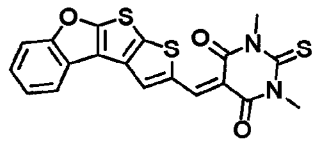

**P2**

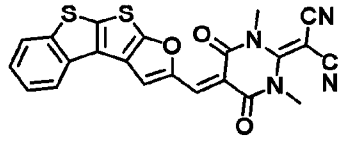

**P3**

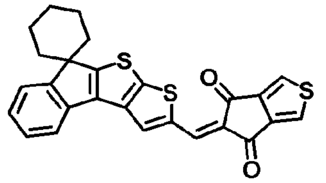

**P4**

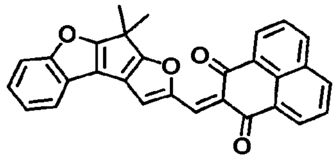

**P5**

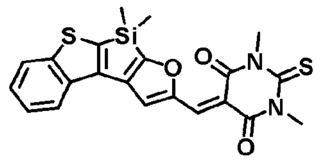

**P6**

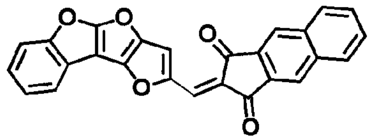

**P7**

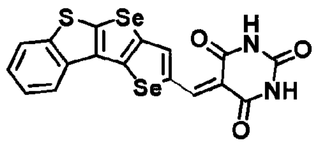

**P8**

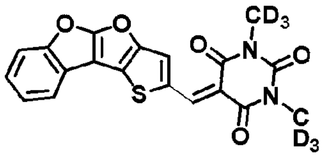

**P9**

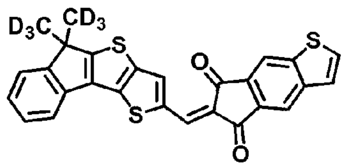

**P10**

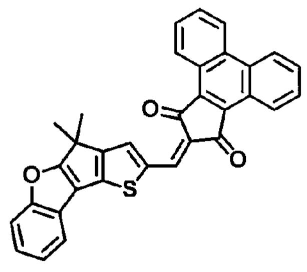

**P11**

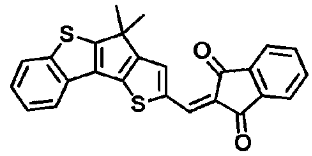

**P12**

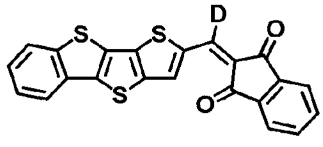

**P13**

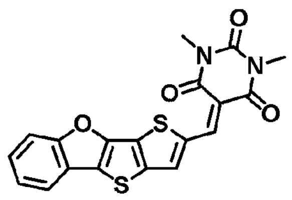

**P14**

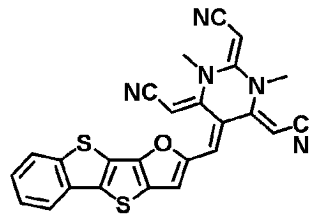

**P15**

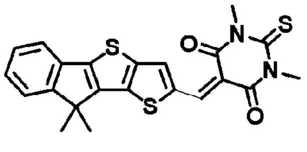

**P16**

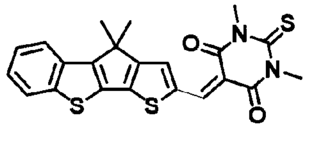

**P17**

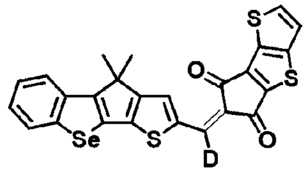

**P18**

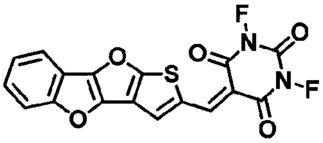

P19

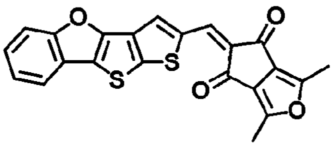

P20

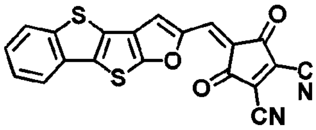

P21

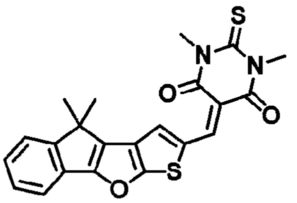

P22

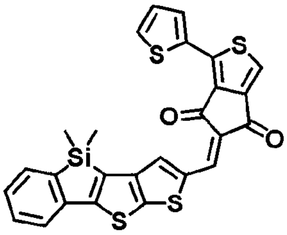

P23

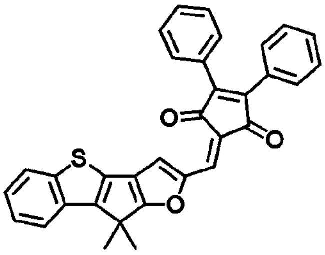

P24

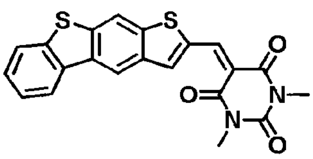

P25

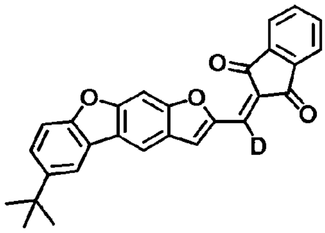

P26

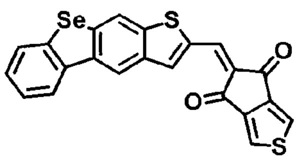

P27

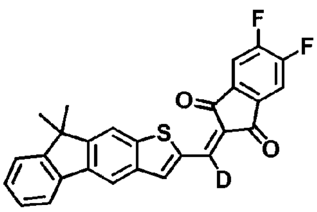

P28

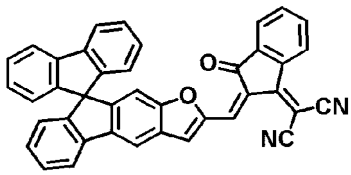

P29

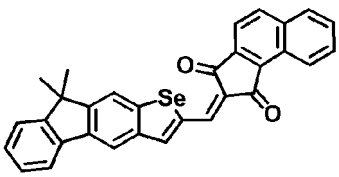

P30

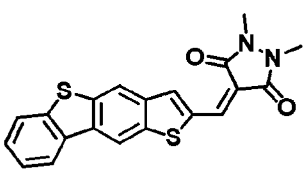

P31

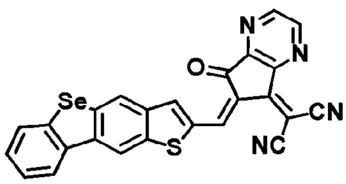

P32

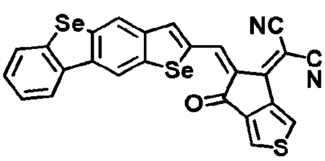

P33

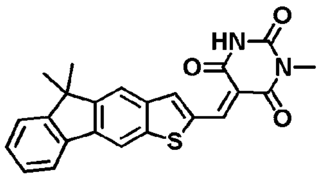

P34

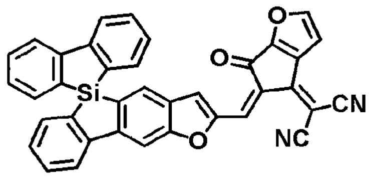

P35

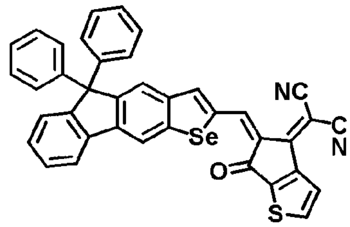

P36

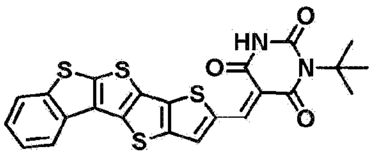

P37

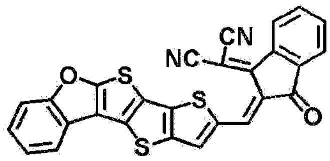

P38

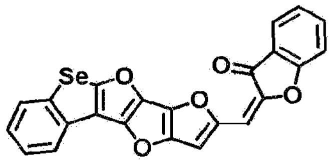

P39

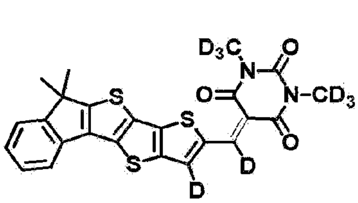

P40

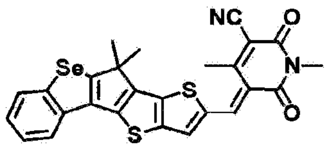

P41

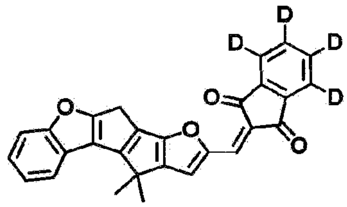

P42

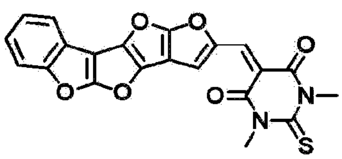

P43

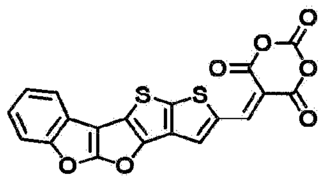

P44

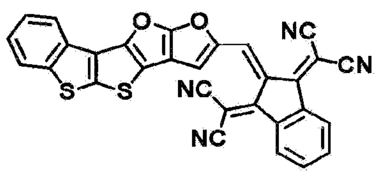

P45

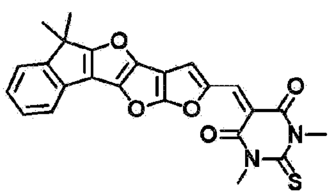

P46

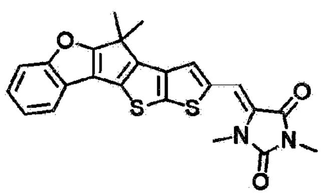

P47

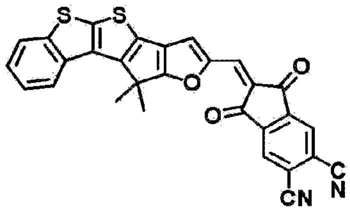

P48

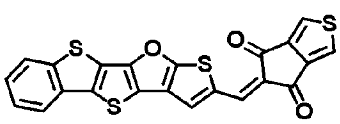

P49

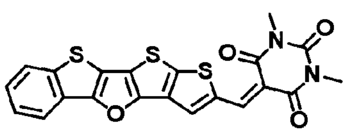

P50

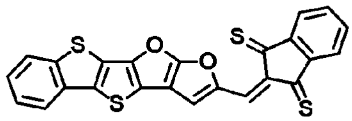

P51

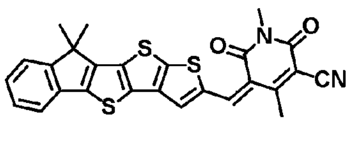

P52

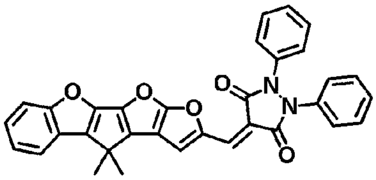

P53

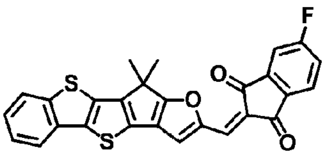

P54

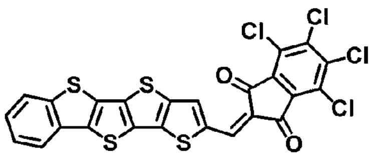

P55

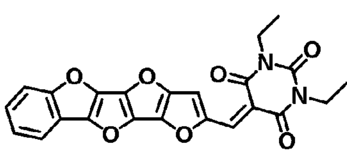

P56

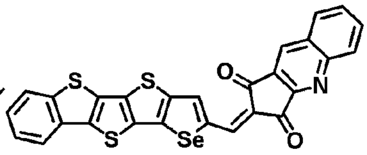

P57

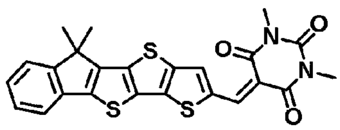

P58

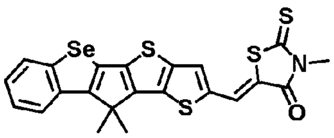

P59

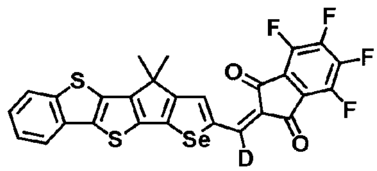

P60

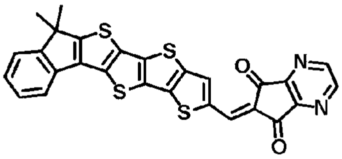

P61

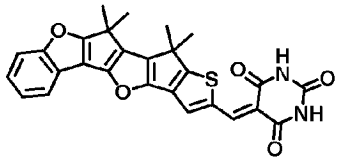

P62

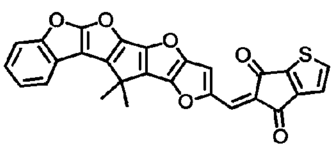

P63

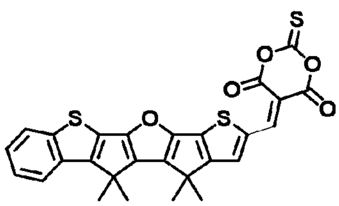

P64

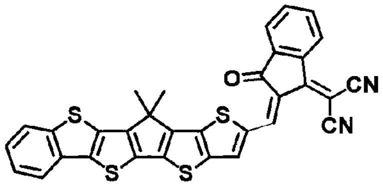

P65

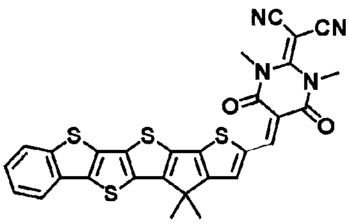

P66

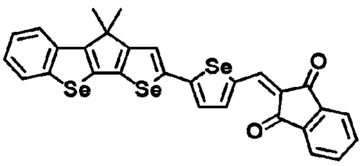

P67

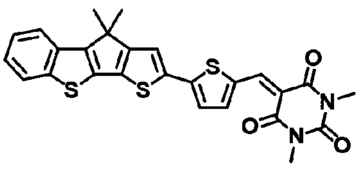

P68

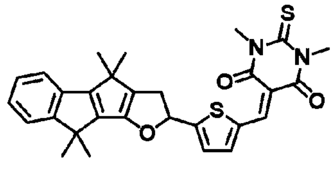

P69

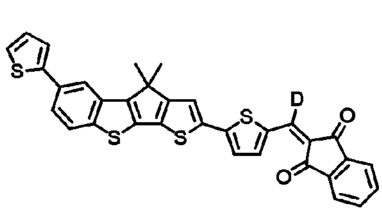

P70

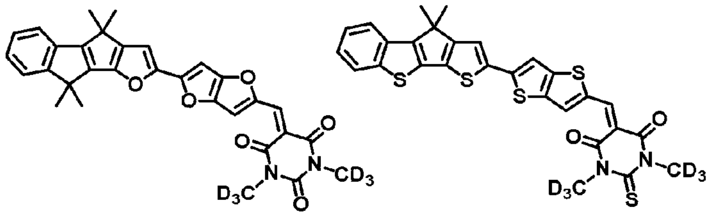

P71 P72

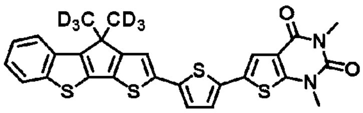

P73

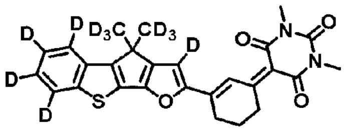

P74

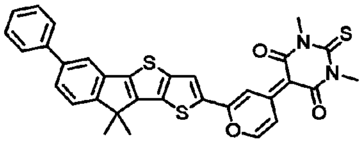

P75 .

6. The compound of claim 1, wherein the compound has a maximum absorption wavelength at 450-650 nm in a thin film state.

7. An organic photoelectric element comprising a first electrode, a second electrode, and an active layer between the first electrode and the second electrode, wherein the active layer comprises the compound of claim 1.

8. The organic photoelectric element of claim 7, wherein the active layer comprises a p-type semiconductor compound and a n-type semiconductor compound, and the p-type semiconductor compound is the compound of claim 1.

9. An image sensor comprising the organic photoelectric element of claim 7.

10. An electronic apparatus comprising the image sensor of claim 9.

FIG. 1

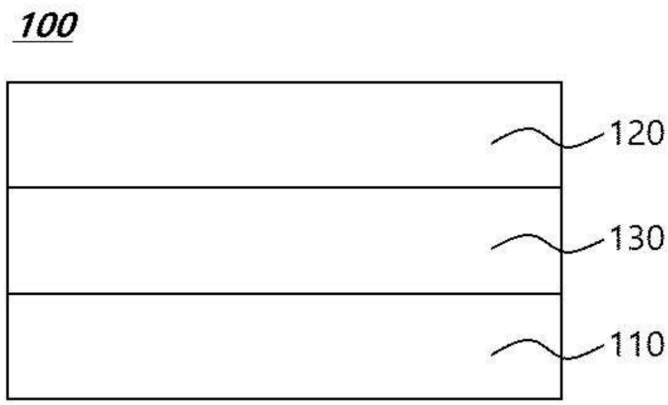


FIG. 2

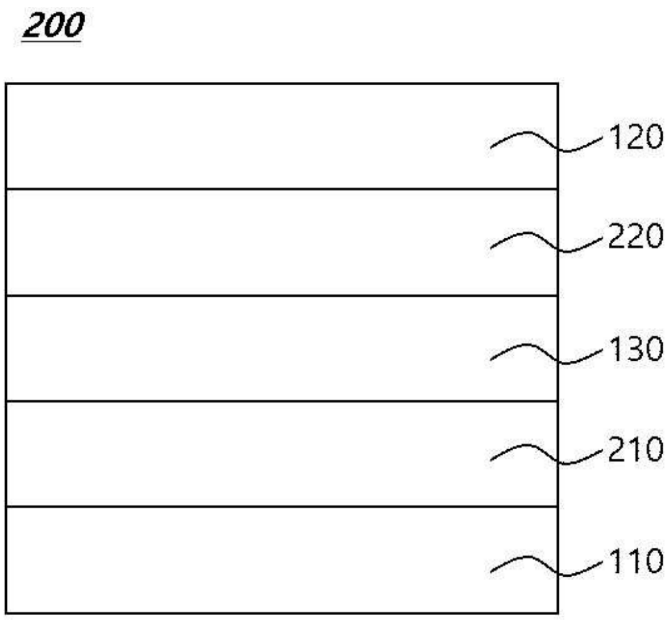

**INTERNATIONAL SEARCH REPORT**

International application No.
**PCT/KR2024/003448**

**A. CLASSIFICATION OF SUBJECT MATTER**

**C07D 495/14**(2006.01)i; **C07D 495/04**(2006.01)i; **C07D 493/04**(2006.01)i; **C07F 7/08**(2006.01)i; **C07D 493/14**(2006.01)i; **C07D 517/14**(2006.01)i; **C07D 517/04**(2006.01)i; **C07D 333/78**(2006.01)i; **C07D 307/94**(2006.01)i; **C07D 331/00**(2006.01)i

According to International Patent Classification (IPC) or to both national classification and IPC

**B. FIELDS SEARCHED**

Minimum documentation searched (classification system followed by classification symbols)

C07D 495/14(2006.01); C07D 495/04(2006.01); C07D 495/12(2006.01); C07D 517/12(2006.01); C07F 7/08(2006.01); H01L 51/42(2006.01); H01L 51/46(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 유기 광전소자(organic photovoltaic device), 450nm, 650nm, 최대 흡수 파장 (maximum absorption wavelength), 녹색 파장(green wavelength)

**C. DOCUMENTS CONSIDERED TO BE RELEVANT**

| Category* | Citation of document, with indication, where appropriate, of the relevant passages | Relevant to claim No. |
|---|---|---|
| X | LI, X. et al. Synthesis and Photovoltaic Properties of a Series of Narrow Bandgap Organic Semiconductor Acceptors with Their Absorption Edge Reaching 900 nm. Chem. Mater. 2017, vol. 29, pp. 10130-10138. See abstract; and figure 1. | 1,2,4,6-10 |
| X | KR 10-2021-0000583 A (SAMSUNG ELECTRONICS CO., LTD.) 05 January 2021 (2021-01-05) See claims 1, 7, 9, 12, 13 and 18; and paragraph [0258]. | 1-10 |
| X | ALI, A. et al. TD-DFT benchmark for UV-visible spectra of fused-ring electron acceptors using global and range-separated hybrids. Phys. Chem. Chem. Phys. 2020, vol. 22, pp. 7864-7874. See abstract; and figures 2 and 3. | 1,2,4,6-10 |
| X | JP 2021-190551 A (FUJIFILM CORP.) 13 December 2021 (2021-12-13) See claim 1; and paragraphs [0055] and [0061]. | 1,2,4,6-10 |
| A | US 2022-0135587 A1 (UBIQUITOUS ENERGY, INC.) 05 May 2022 (2022-05-05) See entire document. | 1-10 |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 June 2024** | **21 June 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2024/003448**

| Patent document cited in search report | Publication date (day/month/year) | Patent family member(s) | Publication date (day/month/year) |
|---|---|---|---|
| KR 10-2021-0000583 A | 05 January 2021 | EP 3757108 A1 | 30 December 2020 |
| | | US 11401289 B2 | 02 August 2022 |
| | | US 2020-0407384 A1 | 31 December 2020 |
| JP 2021-190551 A | 13 December 2021 | JP 7366841 B2 | 23 October 2023 |
| US 2022-0135587 A1 | 05 May 2022 | AU 2022-211038 A1 | 07 September 2023 |
| | | CN 116711029 A | 05 September 2023 |
| | | CN 117062821 A | 14 November 2023 |
| | | EP 4241318 A1 | 13 September 2023 |
| | | EP 4282012 A1 | 29 November 2023 |
| | | JP 2023-549734 A | 29 November 2023 |
| | | JP 2024-516759 A | 17 April 2024 |
| | | KR 10-2023-0117123 A | 07 August 2023 |
| | | KR 10-2023-0148164 A | 24 October 2023 |
| | | US 2022-0140266 A1 | 05 May 2022 |
| | | US 2022-0149298 A1 | 12 May 2022 |
| | | US 2022-0242881 A1 | 04 August 2022 |
| | | WO 2022-098917 A1 | 12 May 2022 |
| | | WO 2022-159769 A1 | 28 July 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Org. Chem.*, 2005, vol. 70, 5014